(19)

Europäisches Patentamt

European Patent Office

Office européen des brevets

(11)     **EP 3 961 219 A2**

(12)                   **EUROPEAN PATENT APPLICATION**

(43) Date of publication:
**02.03.2022   Bulletin 2022/09**

(21) Application number: **21193874.1**

(22) Date of filing: **30.08.2021**

(51) International Patent Classification (IPC):
**G01N 33/68** (2006.01)

(52) Cooperative Patent Classification (CPC):
**G01N 33/6893;** G01N 2800/10; G01N 2800/60

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:   **31.08.2020   US 202063072917 P**

(71) Applicant: **The Chinese University Of Hong Kong New Territories (HK)**

(72) Inventors:
• **FUNG, Erik Yee Mun George**
  **Hong Kong (HK)**

• **LI, Qi**
  **Zhengzhou, Henan Province (CN)**
• **LUI, Leong Ting**
  **Hong Kong (HK)**
• **MA, Ronald Ching Wan**
  **Hong Kong (HK)**
• **WOO, Jean**
  **Hong Kong (HK)**

(74) Representative: **Cabinet Chaillot**
  **16/20, avenue de l'Agent Sarre**
  **B.P. 74**
  **92703 Colombes Cedex (FR)**

(54)   **USE OF GDF-15 IN THE DIAGNOSIS AND TREATMENT OF FRAILTY AND CONDITIONS ASSOCIATED WITH ALTERED PHYSIOLOGICAL RESERVE, PHYSICAL FITNESS AND EXERCISE CAPACITY**

(57)     Provided herein are biomarkers useful for determining frailty, a biomarker signature for frailty, and methods of using the biomarkers to identify, classify, and treat a subject having frailty. Provided herein are also biomarkers useful for determining, identifying, classifying, and treating conditions associated with altered physical reserve, physical fitness, and exercise capacity.

EP 3 961 219 A2

**Description**

FIELD

This application relates to the identification, stratification, and treatment of frailty and conditions associated with altered physical reserve, physical fitness, and exercise capacity.

BACKGROUND

Frailty is a complex multidomain syndrome characterized by a decline in systemic physiological reserve, reduced physical health and fitness, and an accumulation of multiple medical comorbidities ('multimorbidity'), including hypertension and diabetes mellitus. Frailty encompasses a conglomerate of signs, symptoms and clinical findings, including skeletal muscle wasting (sarcopenia), reduced muscle strength, progressive unintentional weight loss (cachexia), anorexia, systemic inflammation ('inflammaging'), neurohormonal maladaptation, immune dysfunction, and depression. Frailty is a serious problem because it is difficult to detect, yet once it appears, can quickly lead to increased morbidity in a patient. However, studies have shown that frailty is potentially reversible and may even transition between non-frail/pre-frail and frail states through time. For all of the above reasons, there is a strong need for new methods of identifying and treating frailty in patients.

SUMMARY OF THE INVENTION

Provided herein is one or more blood biomarkers that can detect, diagnose, gauge, profile, classify or stratify frailty. In some embodiments, provided is a method of using one or more circulating blood biomarkers optionally in combination with metabolites or metabolomics to detect, diagnose, gauge, profile, classify or stratify frailty. Some embodiments provide a method of monitoring frailty status over time. Another embodiment provides a methodological process using one or more blood biomarkers for profiling, classifying, diagnosing and risk stratifying conditions associated with altered physical reserve, physical fitness or exercise capacity. In some embodiments, the blood biomarker is growth differentiation factor 15 (GDF-15), also known as macrophage inhibitory cytokine 1 (MIC-1).

Another embodiment provides a methodological process for profiling, classifying, diagnosing and risk stratifying the clinical syndrome of frailty with/without cardiac dysfunction (CD) through the use of one or more biomarkers, including but not limited to GDF-15 and NT-proBNP (N-terminal prohormone of B-type (brain) natriuretic peptide), coupled with metabolic/metabolomic profiling.

Another embodiment provides a method of determining frailty severity in a subject comprising the steps of

a) measuring the levels of GDF-15 in a biological sample from the subject;
b) measuring the levels of one or more biomarkers selected from the group consisting of albumin, glutamine, and GlycA.

Some embodiments further comprise the step of measuring the levels of one or more biomarkers selected from the group consisting of phosphoglycerides, glycine, and alanine.

Some embodiments further comprise the step of determining frailty severity according to methods described herein, and treating the subject determined to have severe frailty.

Yet another embodiment provides a method of using of a biomarker in combination with metabolic or metabolomic profiling to provide a comprehensive high-dimensional picture of a subject's total health condition (e.g. internal milieu). In some embodiments, the total health conditions include one or more pathophysiological diagnoses and/or monitoring of such conditions.

Another embodiment provides the use of GDF-15 and NT-proBNP, optionally with the metabolome, to identify, define, characterize, diagnose and profile the frailty and non-frailty spectrum from robust to frail status, particularly in the subphenotyping or classification of individuals with and without CD.

Up until now there has been no available blood biomarker or imaging test that can reliably detect, diagnose, classify or risk stratify frailty. Accordingly, the biomarkers and methods provided herein solve such a problem and furthermore have several advantages over current solutions.

In some embodiments the use of circulating biomarker(s) and a broad array of metabolic measures/features (metabolomics) provides quantitation of differences between disease states and/or disorders, and serves as objective measures over time.

In some embodiments the provided methods provide objective testing, diagnosis and monitoring of frailty which are improvements over frailty assessment schemes based on point scoring along multidomain scales (e.g. Fried phenotype assessment [Fried 2001]) which are limited by subjectivity, recall bias, interobserver variability, require the subject to have a certain level of auditory, cognitive and mental competence, and pertain to domains that are under the influence of metabolic, neurohormonal and circulating factors in the bloodstream.

In some embodiments, the use of the $^1$H-nuclear magnetic resonance (NMR) Nightingale or similar platform coupled with functional biomarkers (e.g. NT-proBNP, GDF-15) provides more accurate and advanced frailty-related diagnostics, classification of health status, and health and disease management.

In some embodiments, the combination of biomarkers with metabolomics (biomarker-guided metabolomics) provides a comprehensive high-dimensional picture of the internal milieu.

## BRIEF DESCRIPTION OF THE FIGURES

The disclosure will be readily understood by the following detailed description in conjunction with the accompanying figures.

Fig. 1 shows receiver operative characteristic (ROC) curves for CD for different biomarkers.

Fig. 2A shows metabolomic biosignatures of NT-proBNP according to CD status for 250 metabolites/metabolic features and the strength of the association between NT-proBNP and the metabolites measured using the β coefficient values.

Fig. 2B shows continued metabolomic biosignatures of NT-proBNP of Fig. 2A thereof.

Fig. 2C shows continued metabolomic biosignatures of NT-proBNP of Fig. 2A thereof.

Fig. 2D shows continued metabolomic biosignatures of NT-proBNP of Fig. 2A thereof.

Fig. 2E shows continued metabolomic biosignatures of NT-proBNP of Fig. 2A thereof.

Fig. 2F shows continued metabolomic biosignatures of NT-proBNP of Fig. 2A thereof.

Fig. 2G shows continued metabolomic biosignatures of NT-proBNP of Fig. 2A thereof.

Fig. 2H shows continued metabolomic biosignatures of NT-proBNP of Fig. 2A thereof.

Fig. 2I shows continued metabolomic biosignatures of NT-proBNP of Fig. 2A thereof.

Fig. 2J shows continued metabolomic biosignatures of NT-proBNP of Fig. 2A thereof.

Fig. 3A shows metabolic profiles of paired comparisons among frailty status.

Fig. 3B shows continued metabolic profiles of Fig. 3A thereof.

Fig. 3C shows continued metabolic profiles of Fig. 3A thereof.

Fig. 3D shows continued metabolic profiles of Fig. 3A thereof.

Fig. 3E shows continued metabolic profiles of Fig. 3A thereof.

Fig. 3F shows continued metabolic profiles of Fig. 3A thereof.

Fig. 3G shows continued metabolic profiles of Fig. 3A thereof.

Fig. 3H shows continued metabolic profiles of Fig. 3A thereof.

Fig. 3I shows continued metabolic profiles of Fig. 3A thereof.

Fig. 3J shows continued metabolic profiles of Fig. 3A thereof.

Fig. 4A(i) shows a discovery set of metabolic profiles of GDF-15 shifted with frailty status.

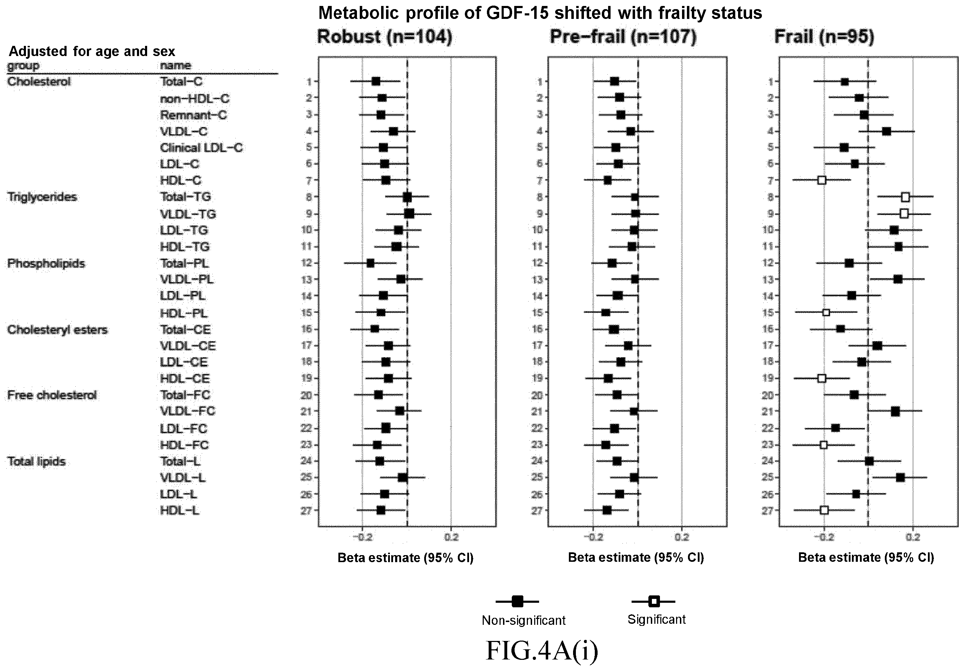

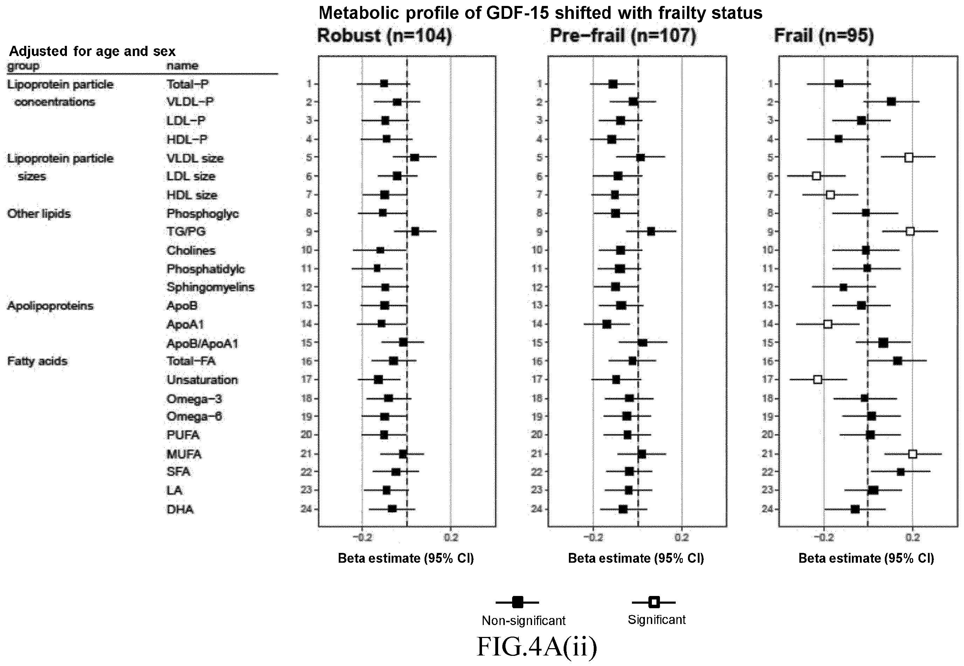

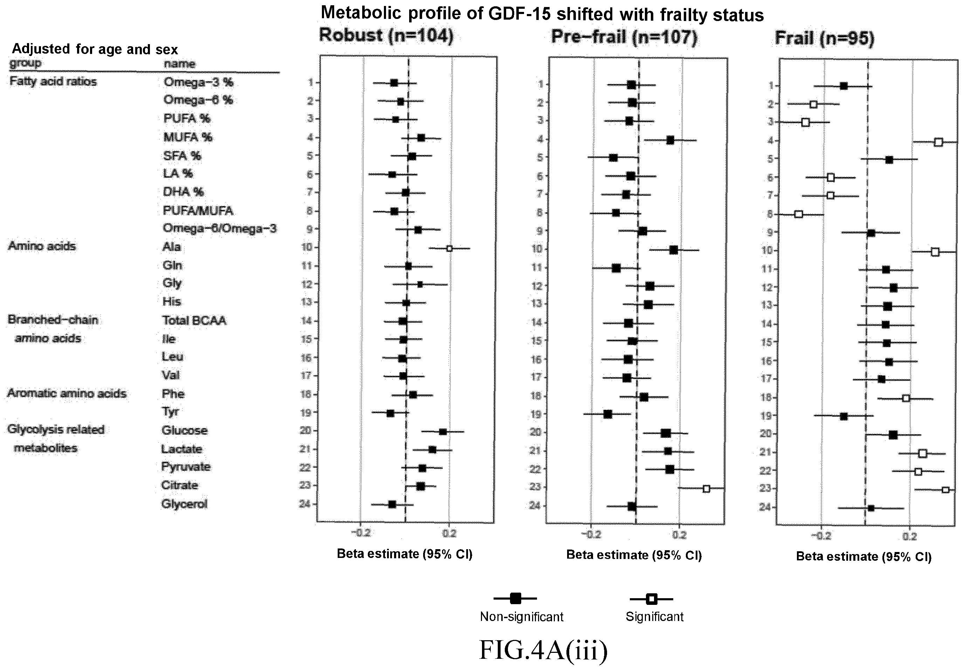

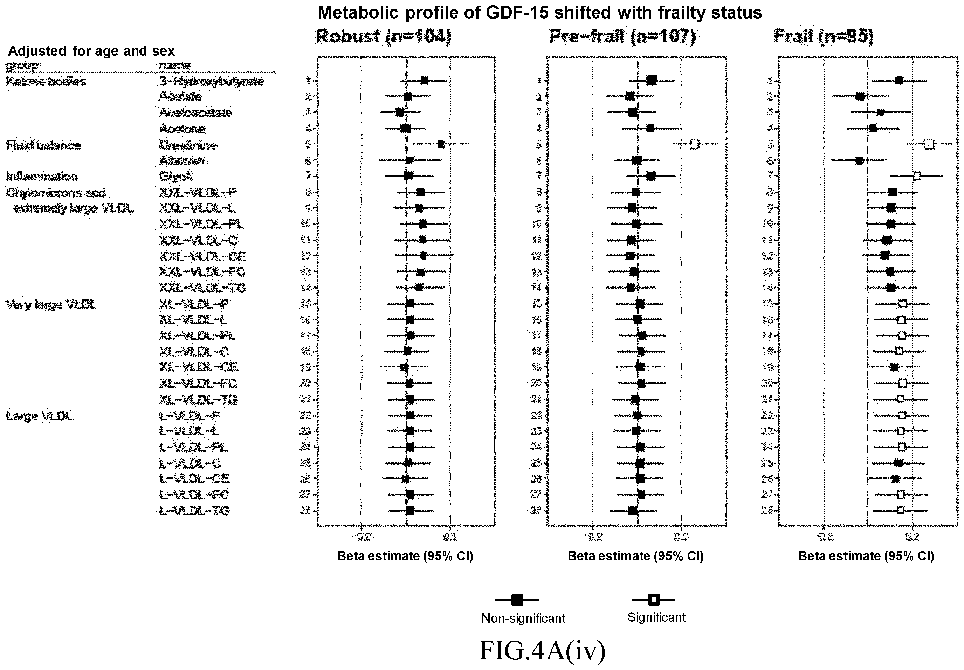

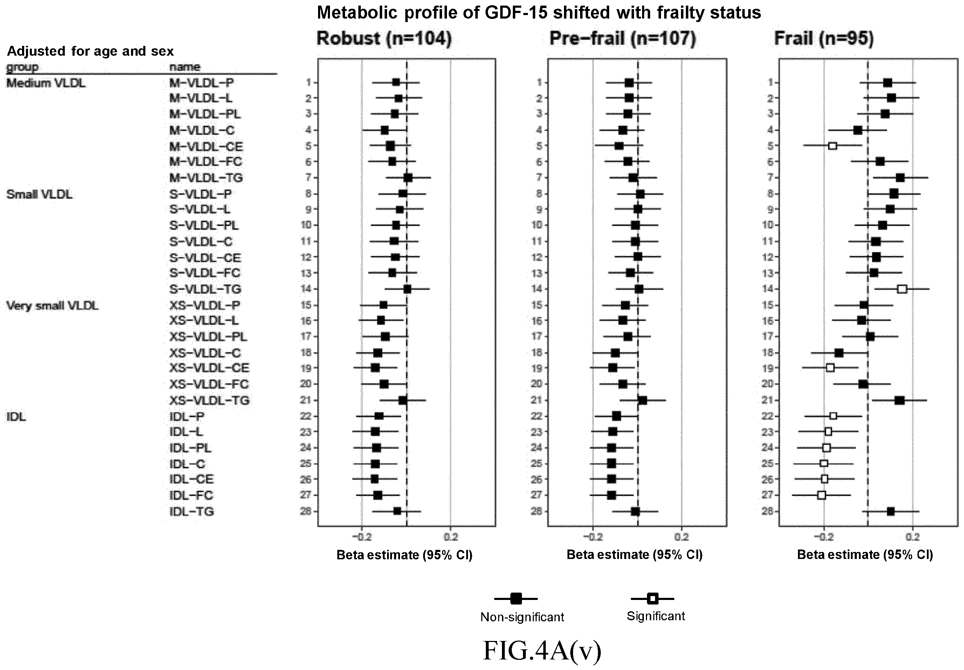

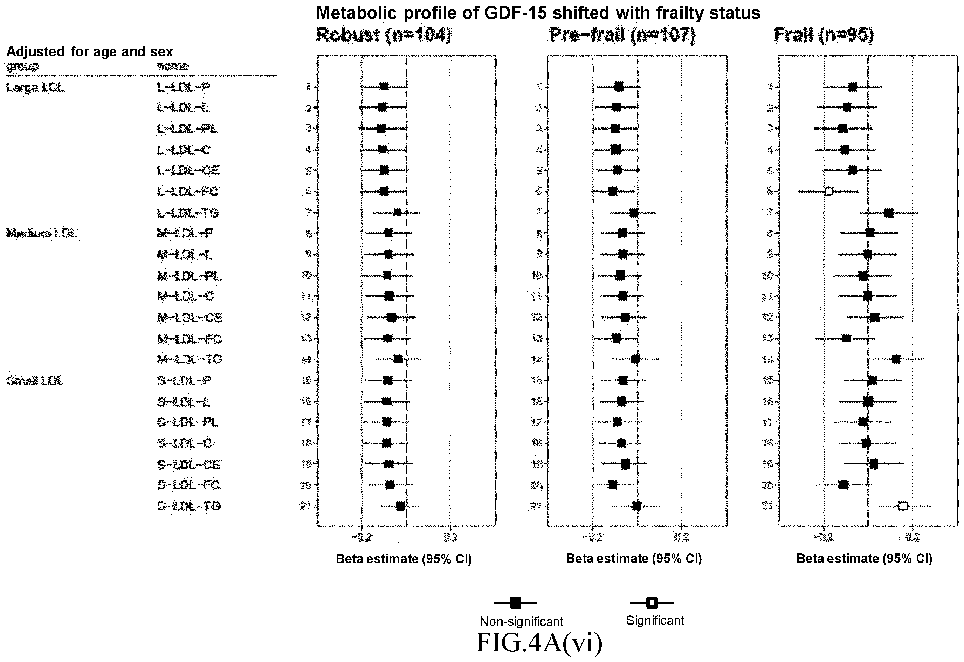

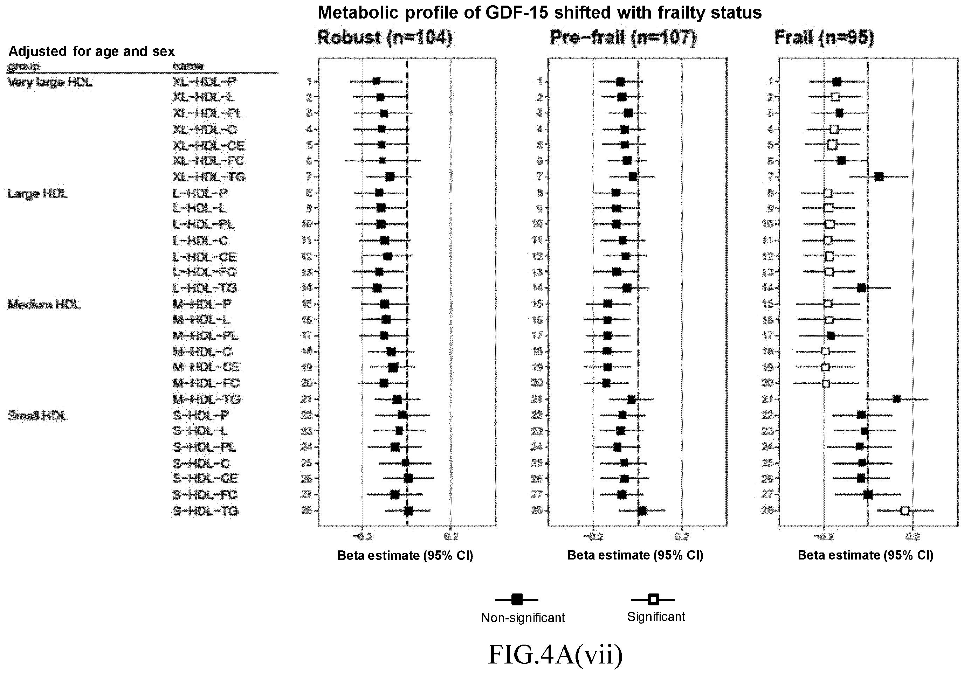

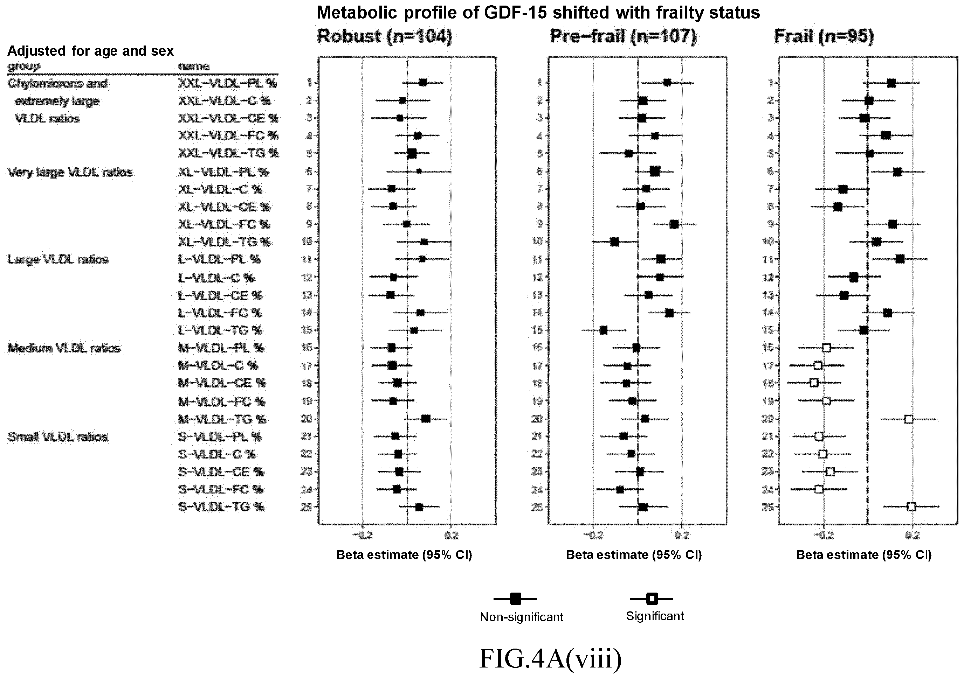

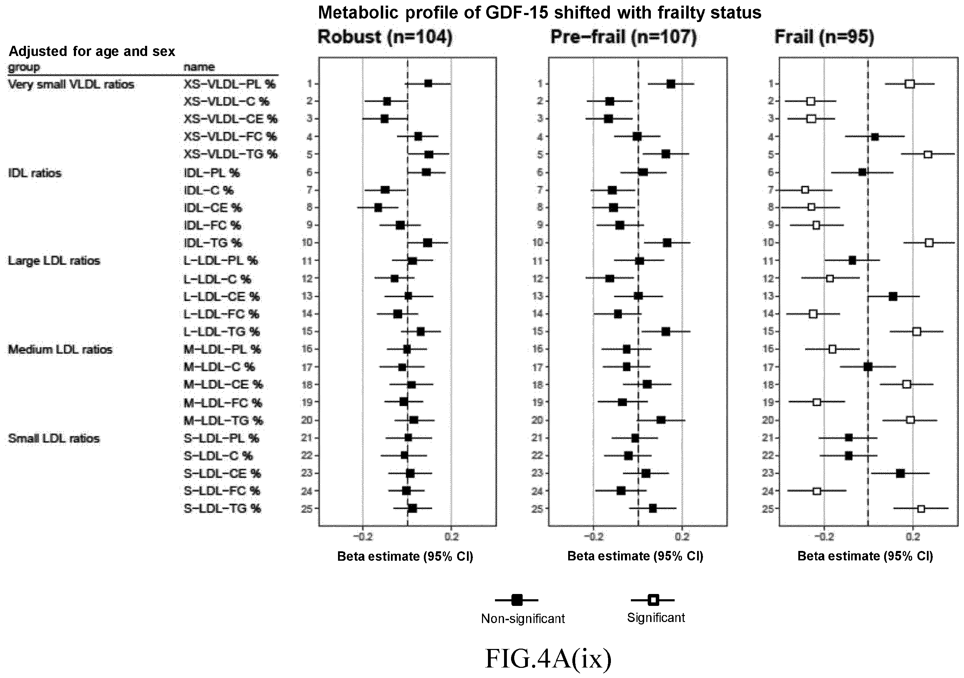

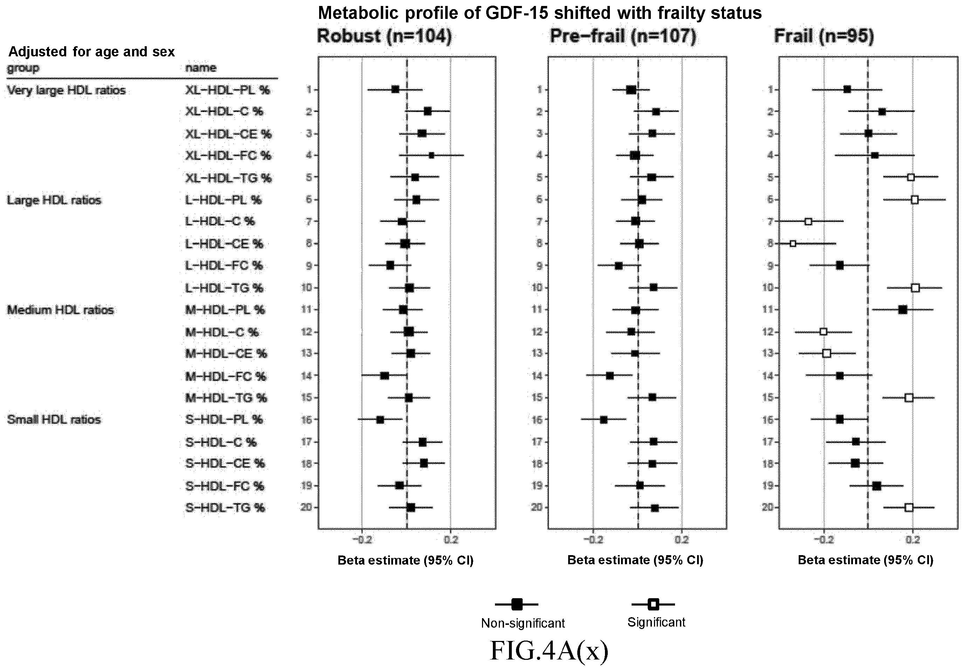

Fig. 4A(ii) shows a continued discovery set of metabolic profiles of GDF-15 of Fig. 4A(i) thereof.

Fig. 4A(iii) shows a continued discovery set of metabolic profiles of GDF-15 of Fig. 4A(i) thereof.

Fig. 4A(iv) shows a continued discovery set of metabolic profiles of GDF-15 of Fig. 4A(i) thereof.

Fig. 4A(v) shows a continued discovery set of metabolic profiles of GDF-15 of Fig. 4A(i) thereof.

Fig. 4A(vi) shows a continued discovery set of metabolic profiles of GDF-15 of Fig. 4A(i) thereof.

Fig. 4A(vii) shows a continued discovery set of metabolic profiles of GDF-15 of Fig. 4A(i) thereof.

Fig. 4A(viii) shows a continued discovery set of metabolic profiles of GDF-15 of Fig. 4A(i) thereof.

Fig. 4A(ix) shows a continued discovery set of metabolic profiles of GDF-15 of Fig. 4A(i) thereof.

Fig. 4A(x) shows a continued discovery set of metabolic profiles of GDF-15 of Fig. 4A(i) thereof.

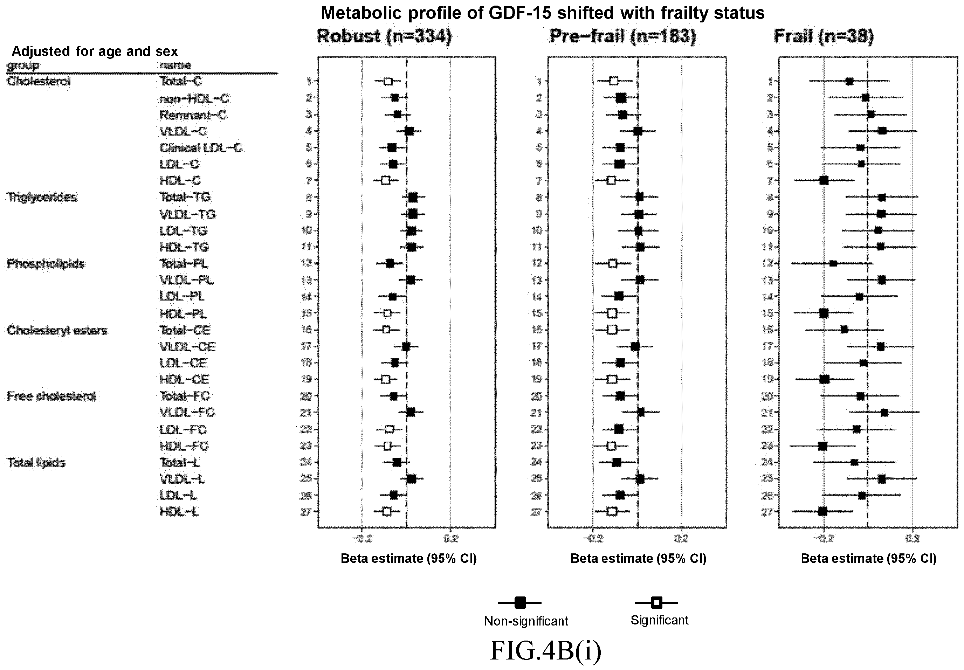

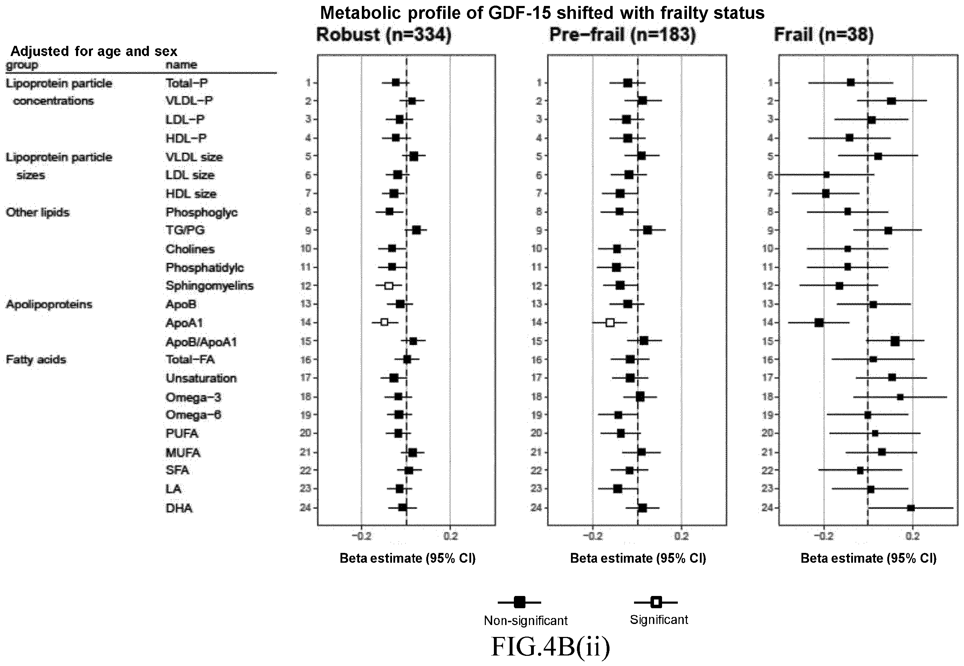

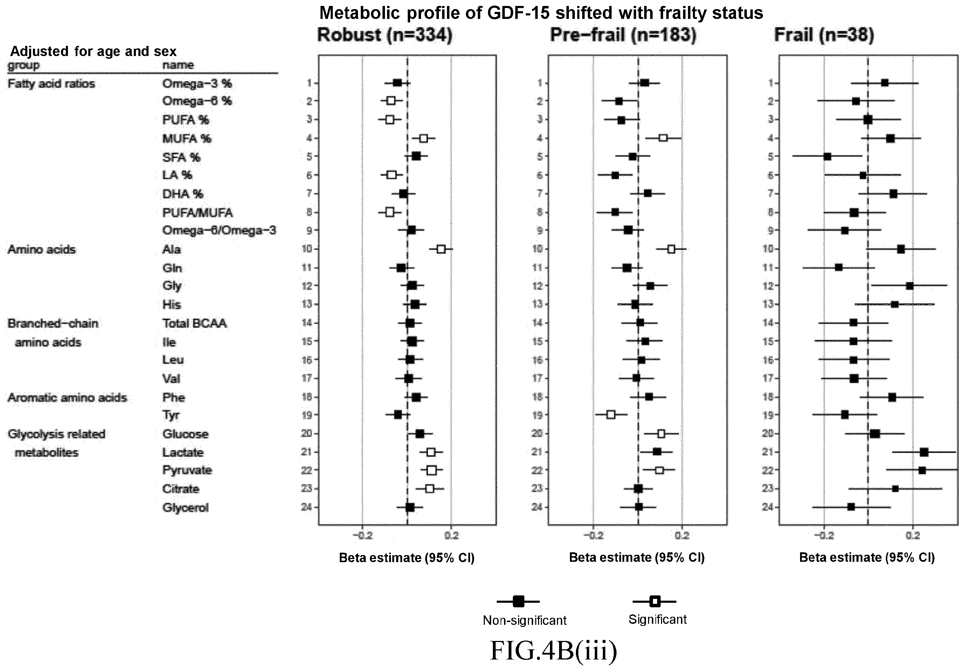

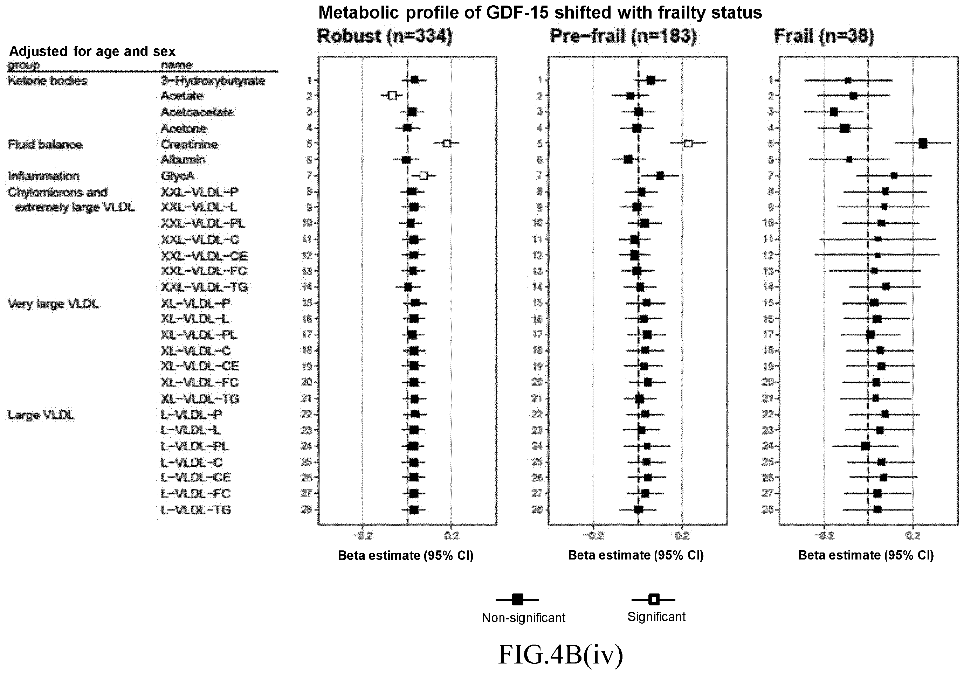

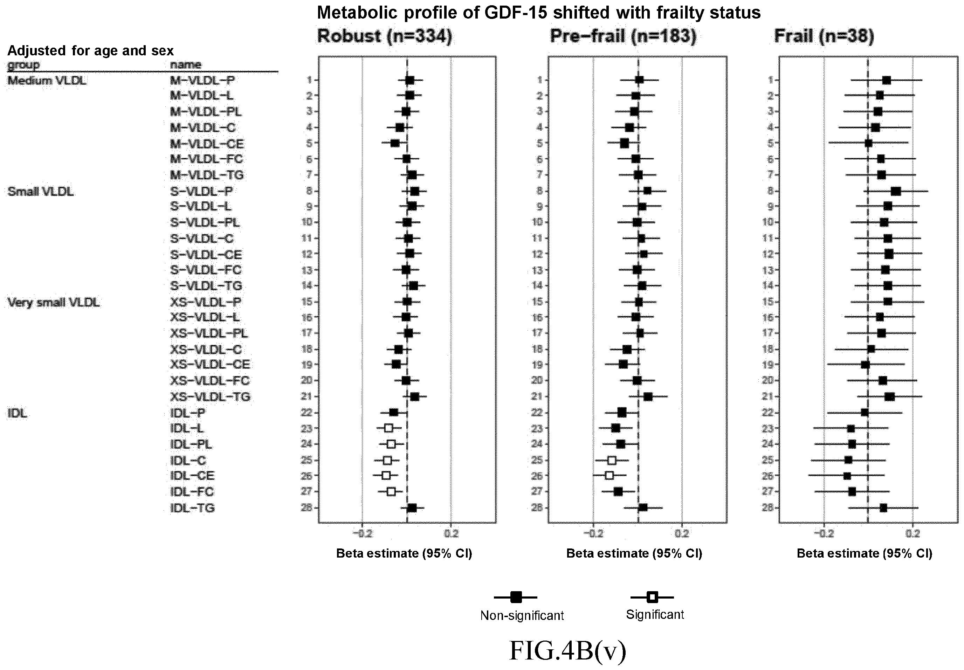

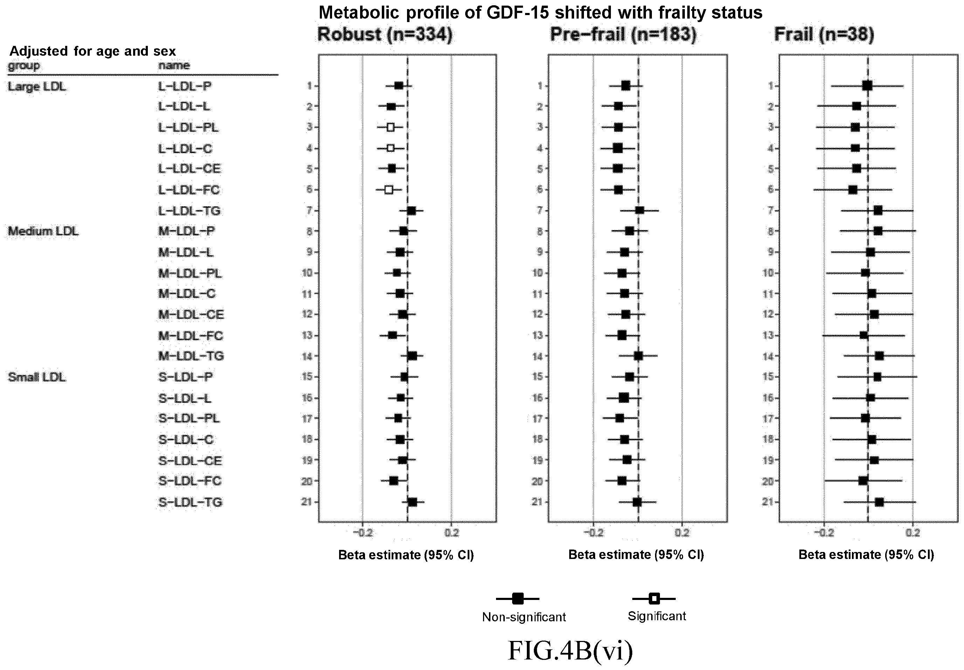

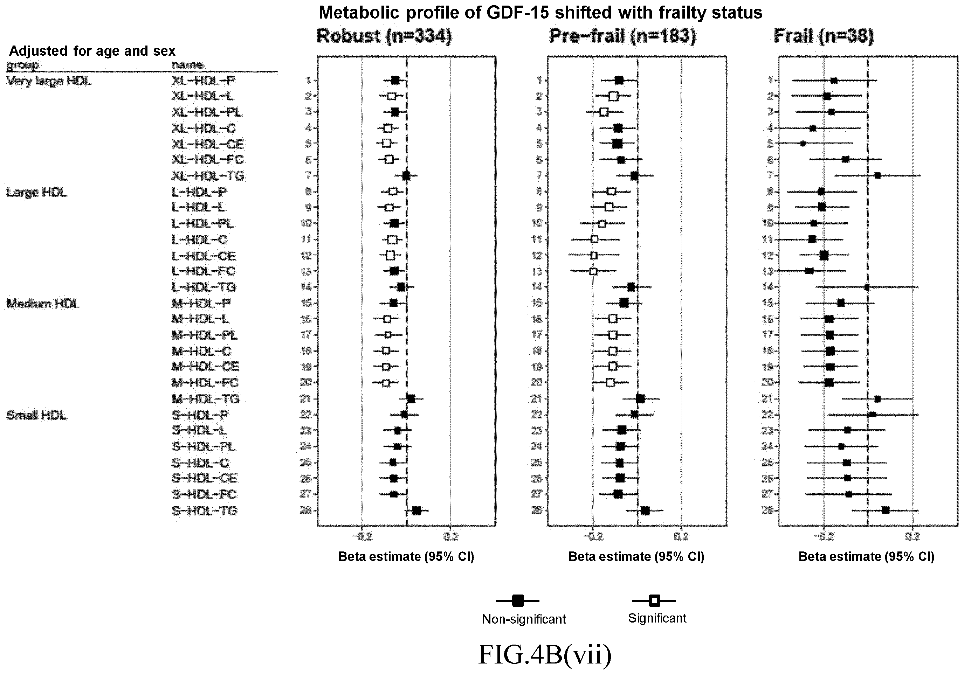

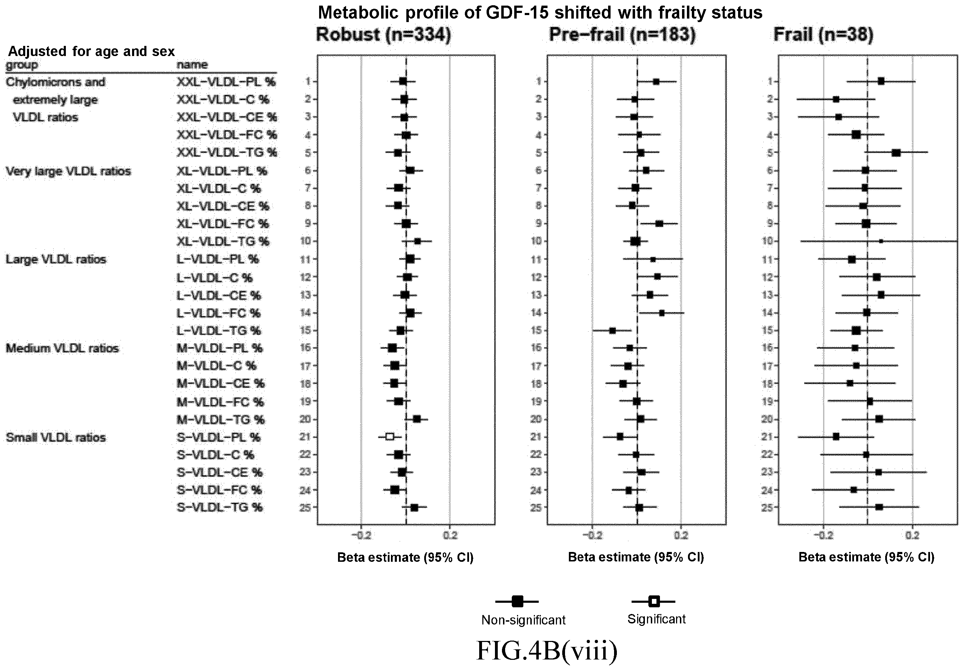

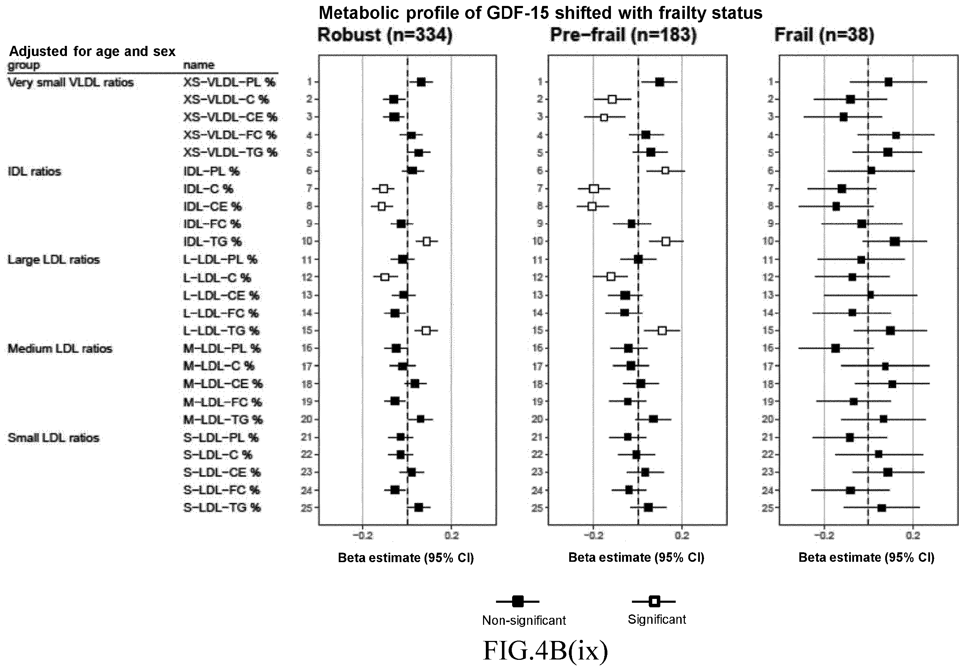

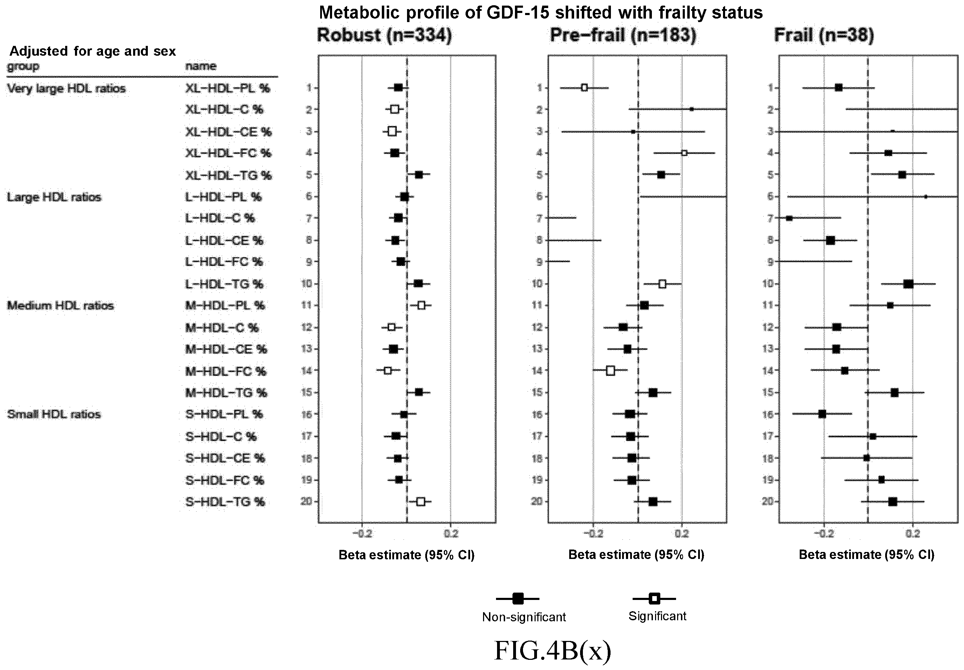

Fig. 4B(i) shows a replication/validation set of metabolic profiles of GDF-15 shifted with frailty status.

Fig. 4B(ii) shows a continued replication/validation set of metabolic profiles of GDF-15 of Fig. 4B(i) thereof.

Fig. 4B(iii) shows a continued replication/validation set of metabolic profiles of GDF-15 of Fig. 4B(i) thereof.

Fig. 4B(iv) shows a continued replication/validation set of metabolic profiles of GDF-15 of Fig. 4B(i) thereof.

Fig. 4B(v) shows a continued replication/validation set of metabolic profiles of GDF-15 of Fig. 4B(i) thereof.

Fig. 4B(vi) shows a continued replication/validation set of metabolic profiles of GDF-15 of Fig. 4B(i) thereof.

Fig. 4B(vii) shows a continued replication/validation set of metabolic profiles of GDF-15 of Fig. 4B(i) thereof.

Fig. 4B(viii) shows a continued replication/validation set of metabolic profiles of GDF-15 of Fig. 4B(i) thereof.

Fig. 4B(ix) shows a continued replication/validation set of metabolic profiles of GDF-15 of Fig. 4B(i) thereof.

Fig. 4B(x) shows a continued replication/validation set of metabolic profiles of GDF-15 of Fig. 4B(i) thereof.

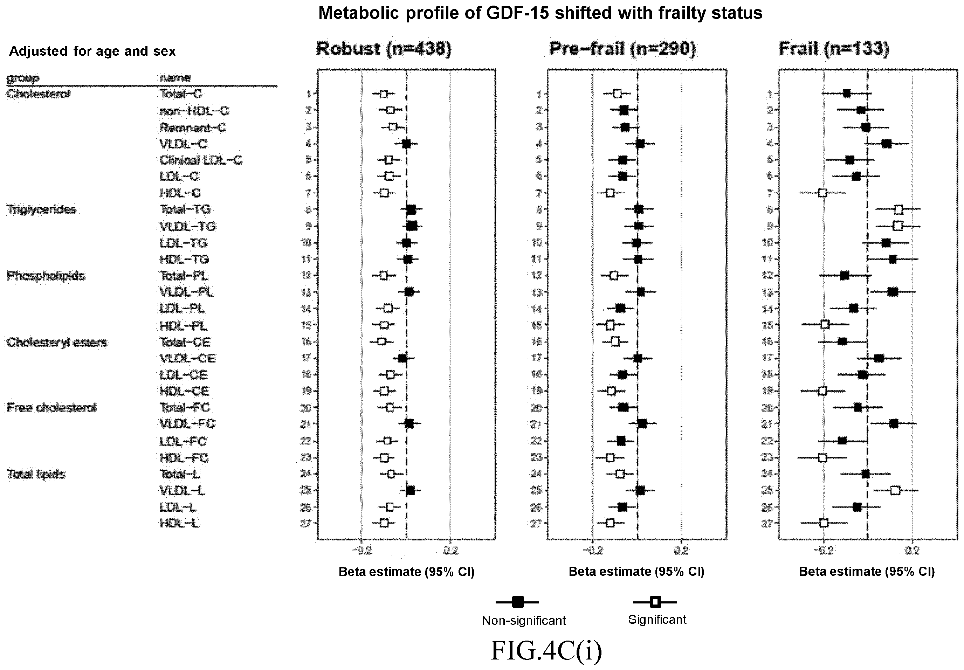

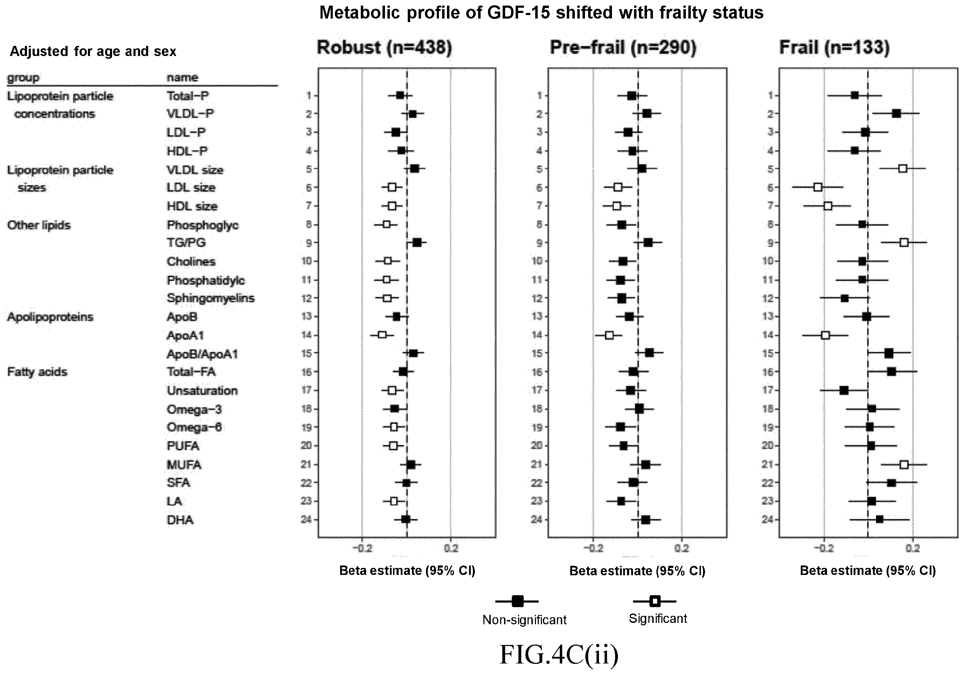

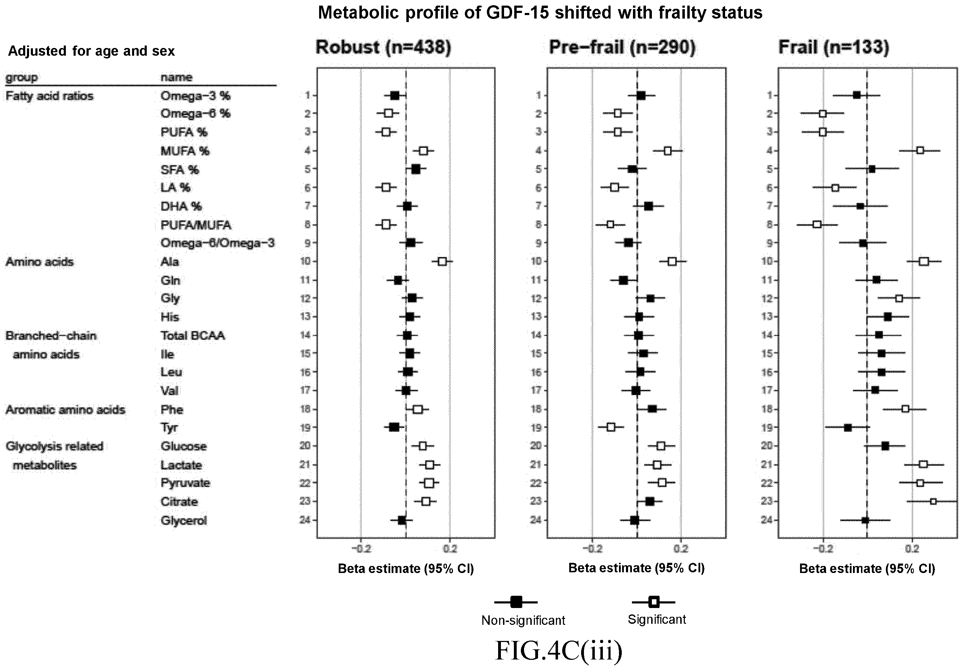

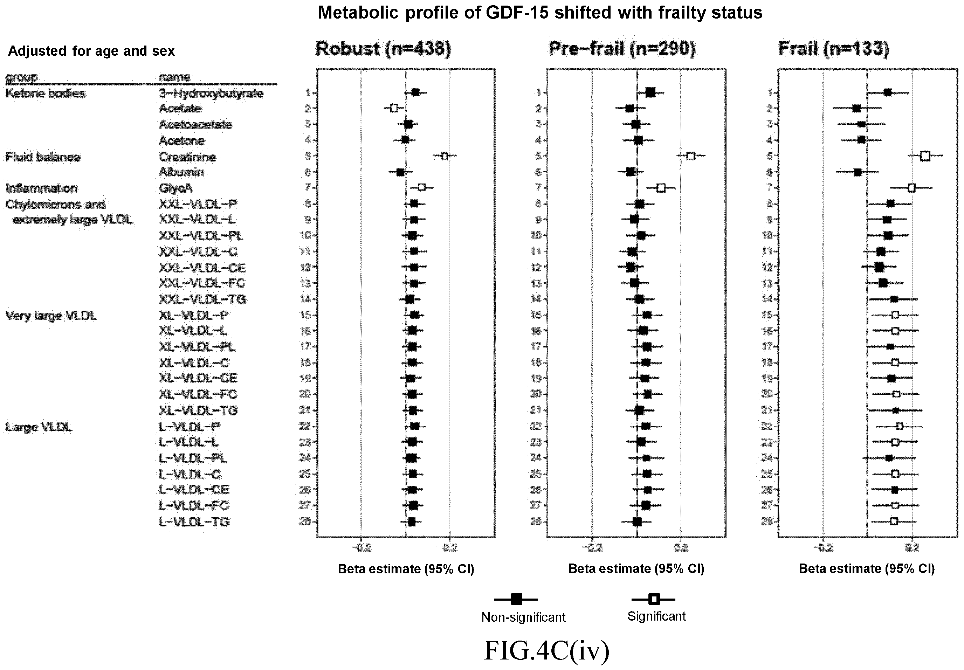

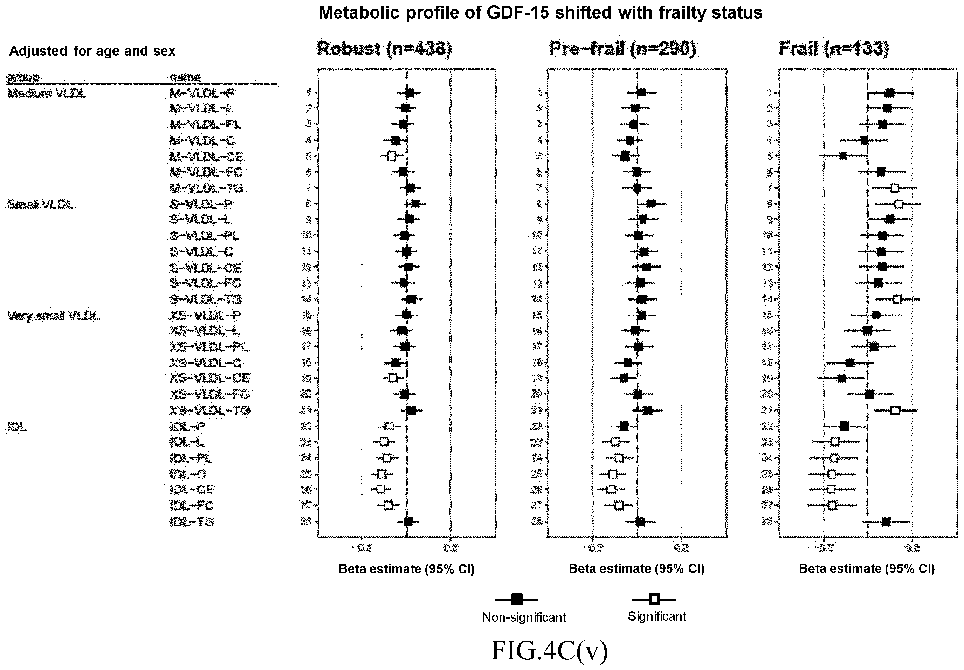

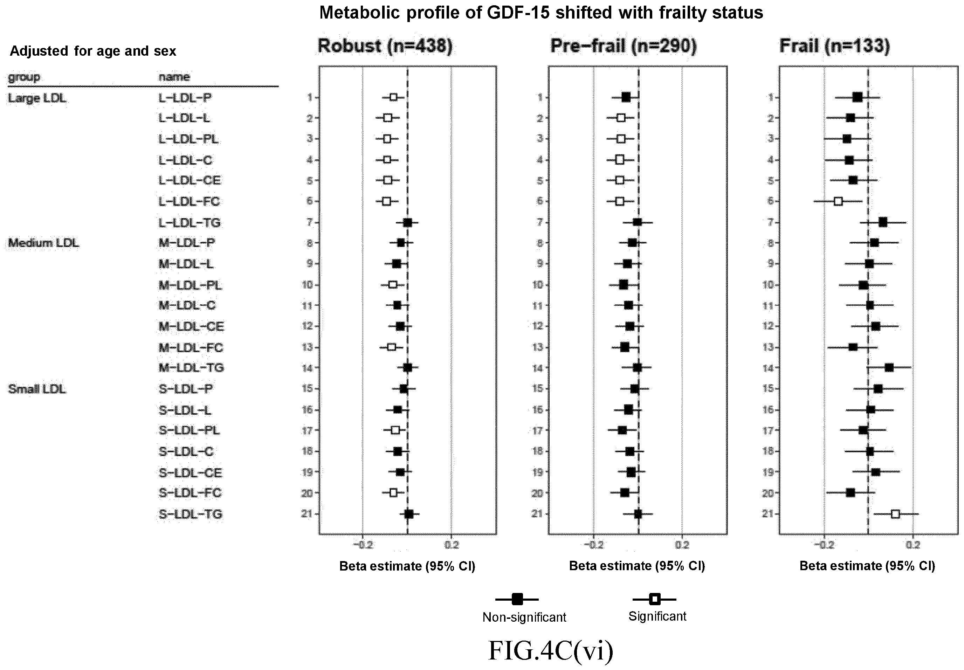

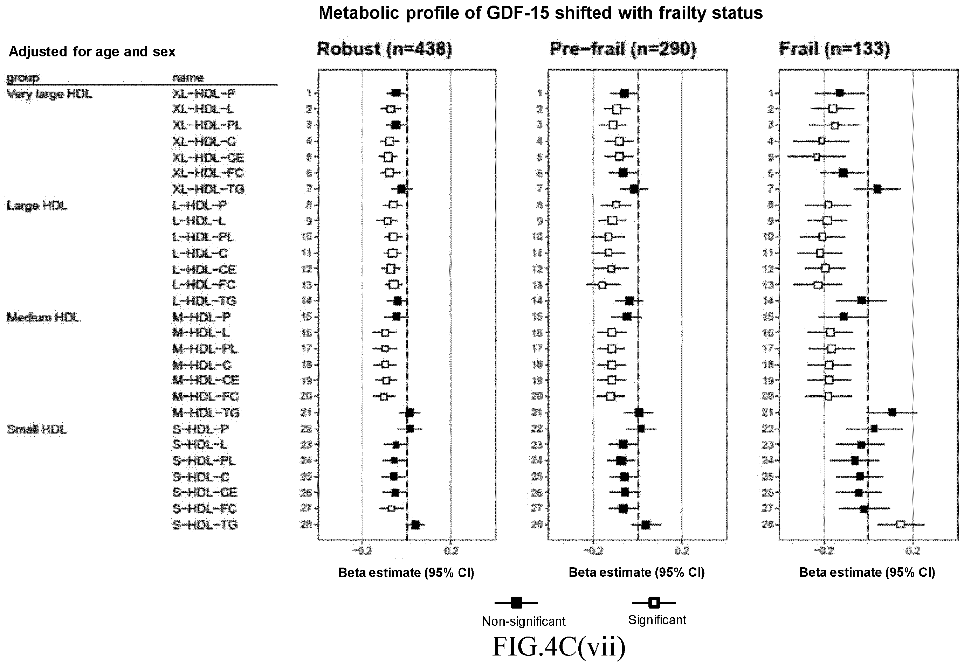

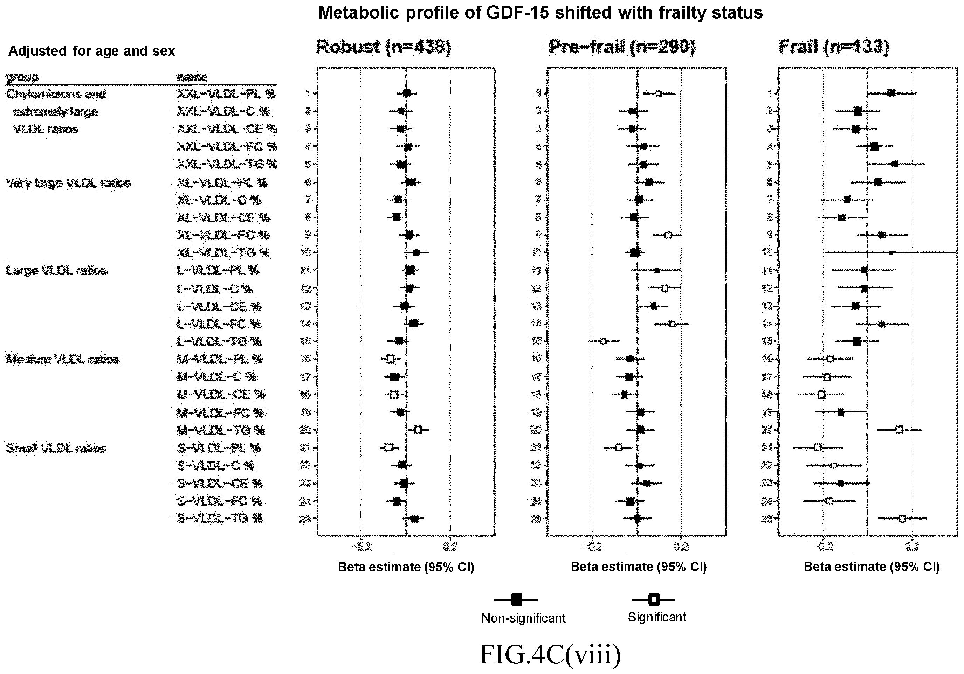

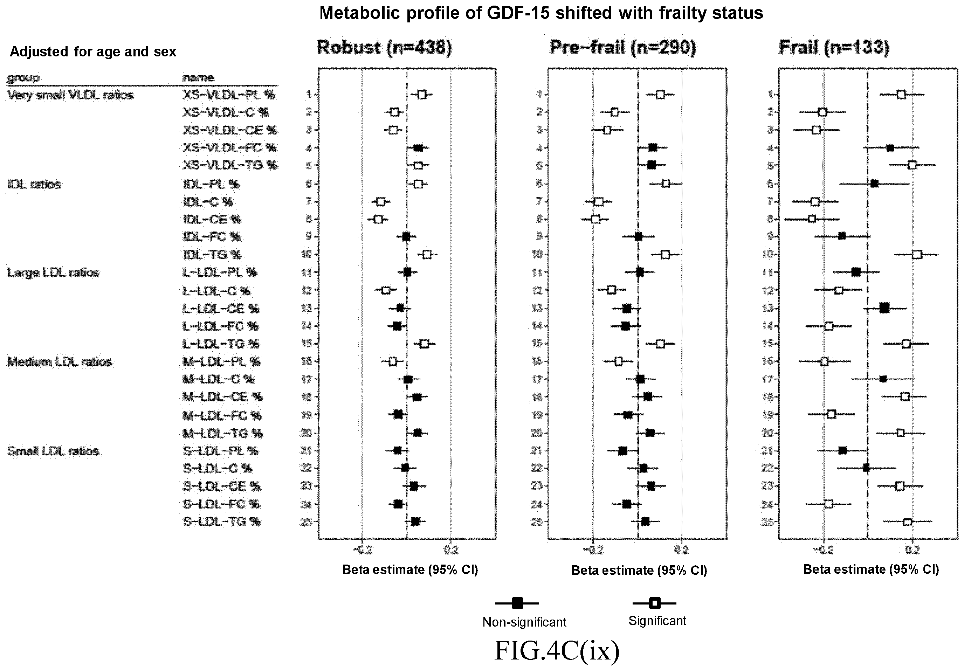

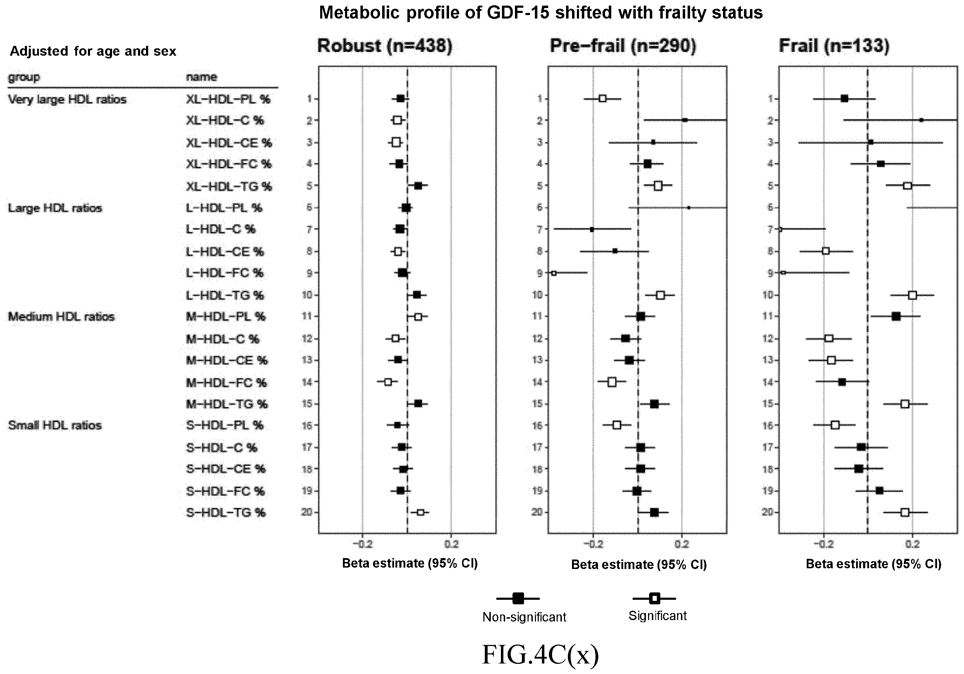

Fig. 4C(i) shows a combined set of discovery and replication/validation of metabolic profiles of GDF-15 shifted with frailty status.

Fig. 4C(ii) shows a continued combined set of discovery and replication/validation of metabolic profiles of GDF-15 of Fig. 4C(i) thereof.

Fig. 4C(iii) shows a continued combined set of discovery and replication/validation of metabolic profiles of GDF-15 of Fig. 4C(i) thereof.

Fig. 4C(iv) shows a continued combined set of discovery and replication/validation of metabolic profiles of GDF-15 of Fig. 4C(i) thereof.

Fig. 4C(v) shows a continued combined set of discovery and replication/validation of metabolic profiles of GDF-15 of Fig. 4C(i) thereof.

Fig. 4C(vi) shows a continued combined set of discovery and replication/validation of metabolic profiles of GDF-15 of Fig. 4C(i) thereof.

Fig. 4C(vii) shows a continued combined set of discovery and replication/validation of metabolic profiles of GDF-15 of Fig. 4C(i) thereof.

Fig. 4C(viii) shows a continued combined set of discovery and replication/validation of metabolic profiles of GDF-15 of Fig. 4C(i) thereof.

Fig. 4C(ix) shows a continued combined set of discovery and replication/validation of metabolic profiles of GDF-15 of Fig. 4C(i) thereof.

Fig. 4C(x) shows a continued combined set of discovery and replication/validation of metabolic profiles of GDF-15 of Fig. 4C(i) thereof.

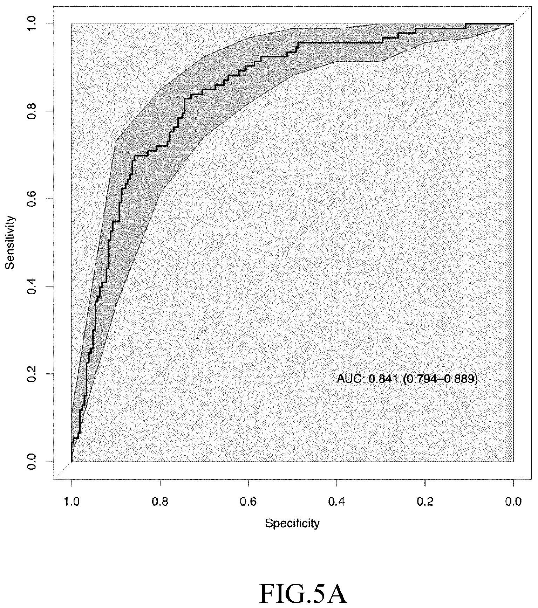

Fig. 5A shows receiver operating characteristic (ROC) curve for a discovery set of the combined classifiers GDF-15, albumin, glutamine, glycoprotein actetylation marker of inflammation (GlycA), and phosphoglycerides), age and sex in predicting frailty (area under the curve AUC).

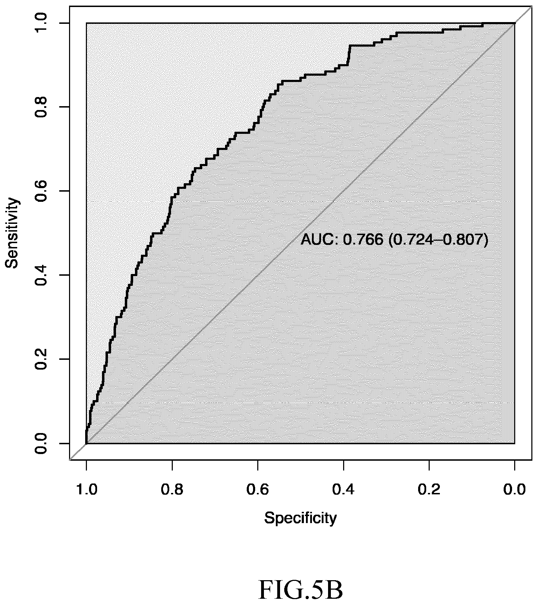

Fig. 5B shows receiver operating characteristic (ROC) curve for a set of discovery and replication/validation of the combined classifiers GDF-15, albumin, glutamine, glycoprotein actetylation marker of inflammation (GlycA), and phosphoglycerides), age and sex in predicting frailty (area under the curve AUC).

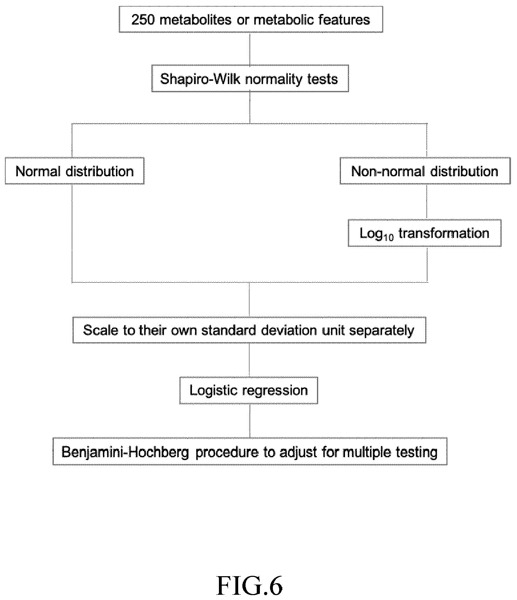

Fig. 6 shows a workflow of logistic regression analysis with adjustment for age and sex.

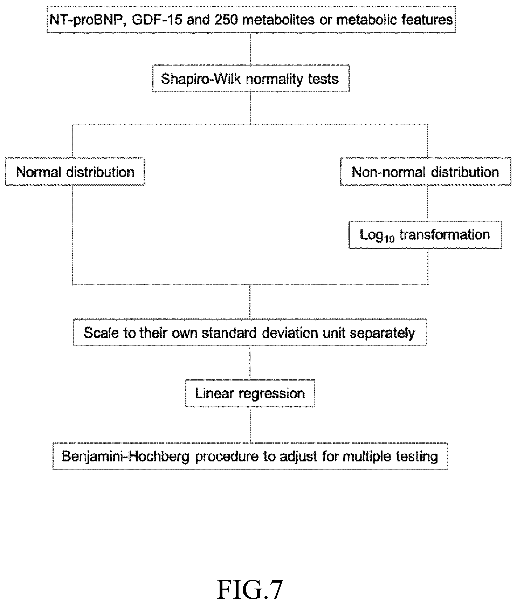

Fig. 7 shows a workflow of age- and sex-adjusted linear regression.

## DETAILED DESCRIPTION

Throughout this description for the purposes of explanation, numerous specific details are set forth in order to provide a thorough understanding of the many aspects and embodiments disclosed herein. It will be apparent, however, to one skilled in the art that the many aspects and embodiments may be practiced without some of these specific details. In other instances, known biological and biochemical entities, mechanisms and analyses are shown herein to avoid obscuring the underlying principles of the described aspects and embodiments. The present invention is in the technical field of diagnostics, classification of health status, and human health and disease management.

### Definitions and Abbreviations

As used herein and in the claims, the terms "comprise" (or any related form such as "comprises" and "comprising"), "include" (or any related forms such as "includes" or "including"), "contain" (or any related forms such as "contains" or "containing"), means including the following elements but not excluding others. As used herein, the singular forms "a", "an" and "the" are intended to include the plural forms as well, unless the context clearly indicates otherwise. Where a range is referred in the specification, the range is understood to include each discrete point within the range. For example, 1-7 means 1, 2, 3, 4, 5, 6, and 7.

As used herein and in the claims, an "effective amount", is an amount that is effective to achieve at least a measurable amount of a desired effect. For example, the amount may be effective to elicit an immune response, and/or it may be effective to elicit a protective response, against a pathogen bearing the polypeptide of interest. In some embodiments, the amount may be effective to maintain stable health, increase mobility, improved ability to retain nutrients, or improve FRAIL test results.

As used herein and in the claims, a "subject" refers to animals such as mammals and vertebrates, including, but not limited to, primates (e.g. humans), cows, sheep, goats, horses, pigs, dogs, cats, rabbits, rats, mice, frogs, zebrafish and the like.

As used herein, the term "treat," "treating" or "treatment" refers to methods of alleviating, abating or ameliorating a disease or condition symptoms, preventing additional symptoms, ameliorating or preventing the underlying metabolic causes of symptoms, inhibiting the disease or condition, arresting the development of the disease or condition, relieving the disease or condition, causing regression of the disease or condition, relieving a condition caused by the disease or condition, or stopping the symptoms of the disease or condition either prophylactically and/or therapeutically.

"GDF-15" means growth differentiation factor 15.

"NT-proBNP" means N-terminal prohormone of B-type (brain) natriuretic peptide, a biomarker of cardiac dysfunction.

"CD" means cardiac dysfunction.

"CI" means confidence interval.

"NS" means not significant.

"OR" means odds ratio.

"GlycA" means glycoprotein acetylation marker of inflammation measured clinically in blood by the presence of certain characteristic N-acetyl methyl group protons which are detectable by $^1$H-NMR

"Albumin" is a globular protein detectable in blood.

"Phosphoglycerides" is glycerol-based phospholipids.

Amino acids herein may be referred to by their full names or their abbreviated names, including, but not limited to, the below list:

| | | |
|---|---|---|
| Alanine: Ala | Glycine: Gly | Proline: Pro |
| Arginine: Arg | Histidine: His | Serine: Ser |
| Asparagine: Asn | Isoleucine: Ile | Threonine: Thr |
| Aspartic acid: Asp | Leucine: Leu | Tryptophan: Trp |
| Cysteine: Cys | Lysine: Lys | Tyrosine: Tyr |
| Glutamic acid: Glu | Methionine: Met | Valine: Val |
| Glutamine: Gln | Phenylalanine: Phe | |

Although the description referred to particular aspects and embodiments, the disclosure should not be construed as limited to the embodiments set forth herein.

One aspect provides an in vitro method of determining frailty severity in a subject comprising the steps of

a) measuring the levels of GDF-15 in a biological sample from the subject;

b) determining the subject as being frail if the level of the biomarker in the biological sample is higher than about 2,000 pg/ml to 6,000 pg/ml; pre-frail if the level of the biomarker in the biological sample is higher than 500 pg/ml to 2000 pg/ml; and robust if the level of the biomarker in the biological sample is less than 500 pg/ml.

Another aspect provides an in vitro method of determining frailty severity in a subject comprising the steps of

a) measuring the levels of GDF-15 in a biological sample from the subject;

b) measuring the levels of one or more biomarkers selected from the group consisting of albumin, glutamine, GlycA, phosphoglycerides, glycine, and alanine;

c) determining the overall biosignature score $p$ by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = \exp(-9.31 \pm 0.817 \times \text{sex} + (0.111 \times \text{age}) + (0.000121 \times \text{GDF-15}) + (0.355 \times \text{Gln})$$
$$+ (0.571 \times \text{albumin}) + (0.526 \times \text{GlycA}) + (-0.256 \times \text{phosphoglycerides})$$
$$+ (0.266 \times \text{Gly}) + (-0.0577 \times \text{Ala});$$

d) determining the subject as being frail if the score $p$ is a value defined in Table 1 wherein the corresponding sensitivity and specificity values add up to between 1.4 and 1.5.

Another aspect provides a method of determining frailty severity in a subject comprising the steps of

a) measuring the levels of GDF-15 in a biological sample from the subject;
b) measuring the levels of one or more biomarkers selected from the group consisting of albumin, glutamine, GlycA, phosphoglycerides, glycine, and alanine;
c) determining the overall biosignature score $p$; and
d) determining the subject as being frail if $p$ is Z; wherein Z is a value defined in Table 1 wherein the corresponding sensitivity and specificity values add up to between 1.4 and 1.5;

wherein the score $p$ is determined by

i) inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l)of the subject into the following equation:

$$p = \frac{\exp(H)}{1 + \exp(H)}$$

wherein

$$H = (-9.31 \pm 0.817 \times sex + (0.111 \times age) + (0.000121 \times GDF\text{-}15)$$
$$+ (0.355 \times Gln) + (0.571 \times albumin) + (0.526 \times GlycA)$$
$$+ (-0.256 \times phosphoglycerides) + (0.266 \times Gly)$$
$$+ (-0.0577 \times Ala);$$

or
ii) inputting the subject's age in years; sex as value of 0 if female, 1 if male; and $\log_{10}$-transformed serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = -25.54 + (-0.95 \times sex) + (0.10 \times age) + (1.20 \times GDF\text{-}15)$$
$$+ (7.26 \times Gln) + (10.0 \times albumin) + (6.30 \times GlycA)$$
$$+ (-3.11 \times phosphoglycerides) + (3.22 \times Gly) + (-0.97 \times Ala).$$

Another aspect provides a method of determining frailty severity in a subject comprising the steps of

a) measuring the levels of GDF-15 in a biological sample from the subject;
b) measuring the levels of one or more biomarkers selected from the group consisting of albumin, glutamine, GlycA, phosphoglycerides, glycine, and alanine;
c) determining the overall biosignature score $p$; and
d) determining the subject as being frail if $p$ is Z;

wherein Z is a value defined in Table 1 wherein the corresponding sensitivity and specificity values add up to between 1.4 and 1.5;
wherein the score $p$ is determined by inputting the subject's age in years; sex as value of 0 if female, 1 if male; and $\log_{10}$-transformed serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = -25.54 + (-0.95 \times \text{sex}) + (0.10 \times \text{age}) + (1.20 \times \text{GDF-15})$$
$$+ (7.26 \times \text{Gln}) + (10.0 \times \text{albumin}) + (6.30 \times \text{GlycA})$$
$$+ (-3.11 \times \text{phosphoglycerides}) + (3.22 \times \text{Gly})$$
$$+ (-0.97 \times \text{Ala}).$$

Another aspect provides a method of determining frailty severity in a subject comprising the steps of

a) measuring the levels of GDF-15 in a biological sample from the subject;
b) measuring the levels of one or more biomarkers selected from the group consisting of albumin, glutamine, GlycA, phosphoglycerides, glycine, and alanine;
c) determining the overall biosignature score *p*; and
d) determining the subject as being frail if *p* is Z; wherein Z is a value defined in Table 1 wherein the corresponding sensitivity and specificity values add up to between 1.4 and 1.5;

wherein the score *p* is determined by inputting the subject's age in years; sex as value of 0 if female, 1 if male; and $\log_{10}$-transformed serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = -25.54 + (-0.95 \times \text{sex}) + (0.10 \times \text{age}) + (1.20 \times \text{GDF-15})$$
$$+ (7.26 \times \text{Gln}) + (10.0 \times \text{albumin}) + (6.30 \times \text{GlycA})$$
$$+ (-3.11 \times \text{phosphoglycerides}) + (3.22 \times \text{Gly})$$
$$+ (-0.97 \times \text{Ala}).$$

In some embodiments, the method is an in vitro method.
In some embodiments, the Z is 0.15 to 0.56. In some embodiments, Z is 0.259 or Youden's J statistic. In some embodiments, the value defined by Table 1 wherein the corresponding sensitivity and specificity values add up to between 1.4 and 1.5 is 0.15 to 0.56; in other embodiments, 0.259 or Youden's J statistic. In some embodiments, the score *p* is 0.15 to 0.56. In some embodiments, the score *p* is 0.259 or Youden's J statistic.
Some embodiments further comprise the step of wherein if the subject is determined to be frail, treating the subject with exercise therapy, physical therapy, physiotherapy, nutritional supplementation (amino acid(s)/leucine (in non-cardiac failure)/protein), or administering a therapeutic drug for treating impaired cardiovascular or cardiopulmonary function. In some embodiments, the therapeutic drug is a combined angiotensin receptor blocker and neprilysin inhibitor (e.g. sacubitril/valsartan), a sodium-glucose transport protein 2 (SGLT2) inhibitor or gliflozins (e.g. dapagliflozin, empagliflozin), a beta blocker (e.g. metoprolol, carvedilol, bisoprolol), renin-angiotensin system inhibitor (e.g. enalapril, lisinopril), mineralocorticoid receptor antagonist (e.g. eplerenone, spironolactone), ivabradine, digoxin, inotropes (e.g. dobutamine, milrinone) or inodilator (e.g. levosimendan).
Some embodiments further comprise the step of measuring the levels of NT-proBNP, wherein if NT-proBNP levels are elevated but GDF-15 levels are not, determining the subject has cardiac dysfunction without frailty; if GDF-15 levels are elevated but NT-proBNP are not, determining the subject has systemic physiological injury or inflammation, hypoperfusion, or non-cardiac frailty; and if both GDF-15 and NT-proBNP levels are elevated, determining the subject has frailty and is predicted to have heart failure. In some embodiments, the subject has cardiac dysfunction, systemic tissue injury or hypoperfusion which can lead to heart failure.

**Table** 1 below shows thresholds and associated sensitivity, specificity values and Youden's index values (*J*) for the biosignature score *p*.

Table 1

| Thresholds | sensitivity | specificity | J |
|---|---|---|---|

| | Thresholds | sensitivity | specificity | J | | Thresholds | sensitivity | specificity | J |
|---|---|---|---|---|---|---|---|---|---|
| 1 | -Inf | 1 | 0 | 0 | 33 | 0.0448 | 0.989 | 0.153 | 0.142 |
| 2 | 0.0149 | 1 | 0.00493 | 0.00493 | 34 | 0.0455 | 0.989 | 0.158 | 0.147 |
| 3 | 0.0172 | 1 | 0.00985 | 0.00985 | 35 | 0.0488 | 0.989 | 0.163 | 0.152 |
| 4 | 0.0194 | 1 | 0.0148 | 0.0148 | 36 | 0.0533 | 0.989 | 0.167 | 0.156 |
| 5 | 0.0199 | 1 | 0.0197 | 0.0197 | 37 | 0.0553 | 0.989 | 0.172 | 0.161 |
| 6 | 0.0218 | 1 | 0.0246 | 0.0246 | 38 | 0.0556 | 0.989 | 0.177 | 0.166 |
| 7 | 0.0242 | 1 | 0.0296 | 0.0296 | 39 | 0.0568 | 0.978 | 0.177 | 0.155 |
| 8 | 0.0247 | 1 | 0.0345 | 0.0345 | 40 | 0.0581 | 0.978 | 0.182 | 0.16 |
| 9 | 0.026 | 1 | 0.0394 | 0.0394 | 41 | 0.0591 | 0.978 | 0.187 | 0.165 |
| 10 | 0.0285 | 1 | 0.0443 | 0.0443 | 42 | 0.0602 | 0.978 | 0.192 | 0.17 |
| 11 | 0.0304 | 1 | 0.0493 | 0.0493 | 43 | 0.0609 | 0.978 | 0.197 | 0.175 |
| 12 | 0.031 | 1 | 0.0542 | 0.0542 | 44 | 0.0621 | 0.968 | 0.197 | 0.165 |
| 13 | 0.0315 | 1 | 0.0591 | 0.0591 | 45 | 0.0631 | 0.968 | 0.202 | 0.17 |
| 14 | 0.033 | 1 | 0.064 | 0.064 | 46 | 0.0635 | 0.968 | 0.207 | 0.175 |
| 15 | 0.0339 | 1 | 0.069 | 0.069 | 47 | 0.0638 | 0.968 | 0.212 | 0.18 |
| 16 | 0.0344 | 1 | 0.0739 | 0.0739 | 48 | 0.0642 | 0.968 | 0.217 | 0.185 |
| 17 | 0.0353 | 0.989 | 0.0739 | 0.0629 | 49 | 0.0643 | 0.968 | 0.222 | 0.19 |
| 18 | 0.0369 | 0.989 | 0.0788 | 0.0678 | 50 | 0.0653 | 0.968 | 0.227 | 0.195 |
| 19 | 0.0383 | 0.989 | 0.0837 | 0.0727 | 51 | 0.0673 | 0.968 | 0.232 | 0.2 |
| 20 | 0.0384 | 0.989 | 0.0887 | 0.0777 | 52 | 0.0697 | 0.968 | 0.236 | 0.204 |
| 21 | 0.0387 | 0.989 | 0.0936 | 0.0826 | 53 | 0.0719 | 0.968 | 0.241 | 0.209 |
| 22 | 0.0401 | 0.989 | 0.0985 | 0.0875 | 54 | 0.0728 | 0.968 | 0.246 | 0.214 |
| 23 | 0.0414 | 0.989 | 0.103 | 0.092 | 55 | 0.0731 | 0.968 | 0.251 | 0.219 |
| 24 | 0.0415 | 0.989 | 0.108 | 0.097 | 56 | 0.0736 | 0.968 | 0.256 | 0.224 |
| 25 | 0.0417 | 0.989 | 0.113 | 0.102 | 57 | 0.0742 | 0.968 | 0.261 | 0.229 |
| 26 | 0.0419 | 0.989 | 0.118 | 0.107 | 58 | 0.0744 | 0.968 | 0.266 | 0.234 |
| 27 | 0.0422 | 0.989 | 0.123 | 0.112 | 59 | 0.0752 | 0.968 | 0.271 | 0.239 |
| 28 | 0.0427 | 0.989 | 0.128 | 0.117 | 60 | 0.0767 | 0.968 | 0.276 | 0.244 |
| 29 | 0.043 | 0.989 | 0.133 | 0.122 | 61 | 0.0781 | 0.968 | 0.281 | 0.249 |
| 30 | 0.0434 | 0.989 | 0.138 | 0.127 | 62 | 0.0789 | 0.968 | 0.286 | 0.254 |
| 31 | 0.0439 | 0.989 | 0.143 | 0.132 | 63 | 0.0795 | 0.968 | 0.291 | 0.259 |
| 32 | 0.0444 | 0.989 | 0.148 | 0.137 | 64 | 0.0807 | 0.968 | 0.296 | 0.264 |

| | Thresholds | sensitivity | specificity | J | | Thresholds | sensitivity | specificity | J |
|---|---|---|---|---|---|---|---|---|---|
| 65 | 0.0823 | 0.968 | 0.3 | 0.268 | 97 | 0.132 | 0.957 | 0.453 | 0.41 |
| 66 | 0.0842 | 0.968 | 0.305 | 0.273 | 98 | 0.132 | 0.957 | 0.458 | 0.415 |
| 67 | 0.0854 | 0.968 | 0.31 | 0.278 | 99 | 0.133 | 0.957 | 0.463 | 0.42 |
| 68 | 0.0869 | 0.968 | 0.315 | 0.283 | 100 | 0.135 | 0.957 | 0.468 | 0.425 |
| 69 | 0.0882 | 0.968 | 0.32 | 0.288 | 101 | 0.136 | 0.957 | 0.473 | 0.43 |
| 70 | 0.0884 | 0.968 | 0.325 | 0.293 | 102 | 0.138 | 0.957 | 0.478 | 0.435 |
| 71 | 0.0889 | 0.968 | 0.33 | 0.298 | 103 | 0.141 | 0.957 | 0.483 | 0.44 |
| 72 | 0.0893 | 0.968 | 0.335 | 0.303 | 104 | 0.143 | 0.957 | 0.488 | 0.445 |
| 73 | 0.0901 | 0.968 | 0.34 | 0.308 | 105 | 0.144 | 0.957 | 0.493 | 0.45 |
| 74 | 0.091 | 0.968 | 0.345 | 0.313 | 106 | 0.144 | 0.957 | 0.498 | 0.455 |
| 75 | 0.0918 | 0.968 | 0.35 | 0.318 | 107 | 0.146 | 0.957 | 0.502 | 0.459 |
| 76 | 0.0953 | 0.968 | 0.355 | 0.323 | 108 | 0.146 | 0.957 | 0.507 | 0.464 |
| 77 | 0.0984 | 0.968 | 0.36 | 0.328 | 109 | 0.148 | 0.946 | 0.507 | 0.453 |
| 78 | 0.0992 | 0.968 | 0.365 | 0.333 | 110 | 0.151 | 0.946 | 0.512 | 0.458 |
| 79 | 0.0996 | 0.968 | 0.369 | 0.337 | 111 | 0.152 | 0.946 | 0.517 | 0.463 |
| 80 | 0.0998 | 0.968 | 0.374 | 0.342 | 112 | 0.153 | 0.946 | 0.522 | 0.468 |
| 81 | 0.1 | 0.968 | 0.379 | 0.347 | 113 | 0.154 | 0.946 | 0.527 | 0.473 |
| 82 | 0.102 | 0.968 | 0.384 | 0.352 | 114 | 0.155 | 0.946 | 0.532 | 0.478 |
| 83 | 0.104 | 0.968 | 0.389 | 0.357 | 115 | 0.155 | 0.946 | 0.537 | 0.483 |
| 84 | 0.106 | 0.968 | 0.394 | 0.362 | 116 | 0.157 | 0.946 | 0.542 | 0.488 |
| 85 | 0.107 | 0.968 | 0.399 | 0.367 | 117 | 0.161 | 0.946 | 0.547 | 0.493 |
| 86 | 0.108 | 0.968 | 0.404 | 0.372 | 118 | 0.164 | 0.946 | 0.552 | 0.498 |
| 87 | 0.109 | 0.968 | 0.409 | 0.377 | 119 | 0.165 | 0.946 | 0.557 | 0.503 |
| 88 | 0.11 | 0.968 | 0.414 | 0.382 | 120 | 0.167 | 0.935 | 0.557 | 0.492 |
| 89 | 0.111 | 0.968 | 0.419 | 0.387 | 121 | 0.168 | 0.935 | 0.562 | 0.497 |
| 90 | 0.112 | 0.968 | 0.424 | 0.392 | 122 | 0.17 | 0.925 | 0.562 | 0.487 |
| 91 | 0.112 | 0.968 | 0.429 | 0.397 | 123 | 0.172 | 0.925 | 0.567 | 0.492 |
| 92 | 0.115 | 0.968 | 0.433 | 0.401 | 124 | 0.172 | 0.925 | 0.571 | 0.496 |
| 93 | 0.119 | 0.957 | 0.433 | 0.39 | 125 | 0.174 | 0.925 | 0.576 | 0.501 |
| 94 | 0.125 | 0.957 | 0.438 | 0.395 | 126 | 0.175 | 0.925 | 0.581 | 0.506 |
| 95 | 0.131 | 0.957 | 0.443 | 0.4 | 127 | 0.176 | 0.914 | 0.581 | 0.495 |
| 96 | 0.131 | 0.957 | 0.448 | 0.405 | 128 | 0.178 | 0.914 | 0.586 | 0.5 |

| | Thresholds | sensitivity | specificity | J | | Thresholds | sensitivity | specificity | J |
|---|---|---|---|---|---|---|---|---|---|
| 129 | 0.18 | 0.914 | 0.591 | 0.505 | 161 | 0.259 | 0.839 | 0.714 | 0.553 |
| 130 | 0.183 | 0.914 | 0.596 | 0.51 | 162 | 0.26 | 0.828 | 0.714 | 0.542 |
| 131 | 0.185 | 0.914 | 0.601 | 0.515 | 163 | 0.262 | 0.817 | 0.714 | 0.531 |
| 132 | 0.186 | 0.903 | 0.601 | 0.504 | 164 | 0.264 | 0.817 | 0.719 | 0.536 |
| 133 | 0.188 | 0.903 | 0.606 | 0.509 | 165 | 0.267 | 0.817 | 0.724 | 0.541 |
| 134 | 0.192 | 0.903 | 0.611 | 0.514 | 166 | 0.269 | 0.817 | 0.729 | 0.546 |
| 135 | 0.197 | 0.903 | 0.616 | 0.519 | 167 | 0.274 | 0.806 | 0.729 | 0.535 |
| 136 | 0.202 | 0.903 | 0.621 | 0.524 | 168 | 0.28 | 0.796 | 0.729 | 0.525 |
| 137 | 0.206 | 0.892 | 0.621 | 0.513 | 169 | 0.283 | 0.785 | 0.729 | 0.514 |
| 138 | 0.21 | 0.892 | 0.626 | 0.518 | 170 | 0.286 | 0.785 | 0.734 | 0.519 |
| 139 | 0.212 | 0.892 | 0.631 | 0.523 | 171 | 0.288 | 0.774 | 0.734 | 0.508 |
| 140 | 0.212 | 0.892 | 0.635 | 0.527 | 172 | 0.289 | 0.774 | 0.739 | 0.513 |
| 141 | 0.214 | 0.882 | 0.635 | 0.517 | 173 | 0.292 | 0.774 | 0.744 | 0.518 |
| 142 | 0.215 | 0.871 | 0.635 | 0.506 | 174 | 0.296 | 0.774 | 0.749 | 0.523 |
| 143 | 0.217 | 0.871 | 0.64 | 0.511 | 175 | 0.305 | 0.774 | 0.754 | 0.528 |
| 144 | 0.22 | 0.871 | 0.645 | 0.516 | 176 | 0.315 | 0.763 | 0.754 | 0.517 |
| 145 | 0.222 | 0.871 | 0.65 | 0.521 | 177 | 0.318 | 0.763 | 0.759 | 0.522 |
| 146 | 0.223 | 0.871 | 0.655 | 0.526 | 178 | 0.318 | 0.763 | 0.764 | 0.527 |
| 147 | 0.223 | 0.871 | 0.66 | 0.531 | 179 | 0.319 | 0.763 | 0.768 | 0.531 |
| 148 | 0.227 | 0.871 | 0.665 | 0.536 | 180 | 0.32 | 0.753 | 0.768 | 0.521 |
| 149 | 0.232 | 0.871 | 0.67 | 0.541 | 181 | 0.321 | 0.742 | 0.768 | 0.51 |
| 150 | 0.234 | 0.871 | 0.675 | 0.546 | 182 | 0.321 | 0.742 | 0.773 | 0.515 |
| 151 | 0.235 | 0.86 | 0.675 | 0.535 | 183 | 0.322 | 0.731 | 0.773 | 0.504 |
| 152 | 0.237 | 0.86 | 0.68 | 0.54 | 184 | 0.323 | 0.72 | 0.773 | 0.493 |
| 153 | 0.238 | 0.86 | 0.685 | 0.545 | 185 | 0.324 | 0.72 | 0.778 | 0.498 |
| 154 | 0.24 | 0.849 | 0.685 | 0.534 | 186 | 0.325 | 0.72 | 0.783 | 0.503 |
| 155 | 0.241 | 0.849 | 0.69 | 0.539 | 187 | 0.325 | 0.72 | 0.788 | 0.508 |
| 156 | 0.242 | 0.849 | 0.695 | 0.544 | 188 | 0.326 | 0.72 | 0.793 | 0.513 |
| 157 | 0.247 | 0.839 | 0.695 | 0.534 | 189 | 0.328 | 0.71 | 0.793 | 0.503 |
| 158 | 0.254 | 0.839 | 0.7 | 0.539 | 190 | 0.33 | 0.71 | 0.798 | 0.508 |
| 159 | 0.258 | 0.839 | 0.704 | 0.543 | 191 | 0.333 | 0.71 | 0.803 | 0.513 |
| 160 | 0.259 | 0.839 | 0.709 | 0.548 | 192 | 0.337 | 0.71 | 0.808 | 0.518 |

| | Thresholds | sensitivity | specificity | J | | Thresholds | sensitivity | specificity | J |
|---|---|---|---|---|---|---|---|---|---|
| 193 | 0.341 | 0.699 | 0.808 | 0.507 | 225 | 0.501 | 0.559 | 0.901 | 0.46 |
| 194 | 0.347 | 0.699 | 0.813 | 0.512 | 226 | 0.51 | 0.548 | 0.901 | 0.449 |
| 195 | 0.349 | 0.699 | 0.818 | 0.517 | 227 | 0.519 | 0.548 | 0.906 | 0.454 |
| 196 | 0.356 | 0.699 | 0.823 | 0.522 | 228 | 0.522 | 0.538 | 0.906 | 0.444 |
| 197 | 0.366 | 0.688 | 0.823 | 0.511 | 229 | 0.527 | 0.527 | 0.906 | 0.433 |
| 198 | 0.38 | 0.688 | 0.828 | 0.516 | 230 | 0.534 | 0.527 | 0.911 | 0.438 |
| 199 | 0.39 | 0.688 | 0.833 | 0.521 | 231 | 0.539 | 0.516 | 0.911 | 0.427 |
| 200 | 0.396 | 0.688 | 0.837 | 0.525 | 232 | 0.541 | 0.516 | 0.916 | 0.432 |
| 201 | 0.402 | 0.688 | 0.842 | 0.53 | 233 | 0.548 | 0.516 | 0.921 | 0.437 |
| 202 | 0.406 | 0.688 | 0.847 | 0.535 | 234 | 0.558 | 0.516 | 0.926 | 0.442 |
| 203 | 0.408 | 0.677 | 0.847 | 0.524 | 235 | 0.563 | 0.505 | 0.926 | 0.431 |
| 204 | 0.413 | 0.667 | 0.847 | 0.514 | 236 | 0.568 | 0.495 | 0.926 | 0.421 |
| 205 | 0.422 | 0.667 | 0.852 | 0.519 | 237 | 0.574 | 0.495 | 0.931 | 0.426 |
| 206 | 0.428 | 0.667 | 0.857 | 0.524 | 238 | 0.578 | 0.484 | 0.931 | 0.415 |
| 207 | 0.432 | 0.667 | 0.862 | 0.529 | 239 | 0.584 | 0.473 | 0.931 | 0.404 |
| 208 | 0.435 | 0.667 | 0.867 | 0.534 | 240 | 0.588 | 0.462 | 0.931 | 0.393 |
| 209 | 0.44 | 0.656 | 0.867 | 0.523 | 241 | 0.59 | 0.452 | 0.931 | 0.383 |
| 210 | 0.443 | 0.656 | 0.872 | 0.528 | 242 | 0.594 | 0.441 | 0.931 | 0.372 |
| 211 | 0.444 | 0.645 | 0.872 | 0.517 | 243 | 0.597 | 0.43 | 0.931 | 0.361 |
| 212 | 0.449 | 0.645 | 0.877 | 0.522 | 244 | 0.6 | 0.43 | 0.936 | 0.366 |
| 213 | 0.455 | 0.645 | 0.882 | 0.527 | 245 | 0.607 | 0.419 | 0.936 | 0.355 |
| 214 | 0.46 | 0.634 | 0.882 | 0.516 | 246 | 0.612 | 0.409 | 0.936 | 0.345 |
| 215 | 0.465 | 0.624 | 0.882 | 0.506 | 247 | 0.615 | 0.398 | 0.936 | 0.334 |
| 216 | 0.466 | 0.613 | 0.882 | 0.495 | 248 | 0.621 | 0.387 | 0.936 | 0.323 |
| 217 | 0.47 | 0.602 | 0.882 | 0.484 | 249 | 0.635 | 0.376 | 0.936 | 0.312 |
| 218 | 0.474 | 0.602 | 0.887 | 0.489 | 250 | 0.646 | 0.376 | 0.941 | 0.317 |
| 219 | 0.476 | 0.591 | 0.887 | 0.478 | 251 | 0.646 | 0.366 | 0.941 | 0.307 |
| 220 | 0.476 | 0.581 | 0.887 | 0.468 | 252 | 0.648 | 0.366 | 0.946 | 0.312 |
| 221 | 0.477 | 0.57 | 0.887 | 0.457 | 253 | 0.65 | 0.355 | 0.946 | 0.301 |
| 222 | 0.481 | 0.57 | 0.892 | 0.462 | 254 | 0.651 | 0.344 | 0.946 | 0.29 |
| 223 | 0.491 | 0.559 | 0.892 | 0.451 | 255 | 0.655 | 0.344 | 0.951 | 0.295 |
| 224 | 0.498 | 0.559 | 0.897 | 0.456 | 256 | 0.657 | 0.344 | 0.956 | 0.3 |

| | Thresholds | sensitivity | specificity | J | | Thresholds | sensitivity | specificity | J |
|---|---|---|---|---|---|---|---|---|---|
| 257 | 0.659 | 0.333 | 0.956 | 0.289 | 278 | 0.809 | 0.14 | 0.97 | 0.11 |
| 258 | 0.661 | 0.333 | 0.961 | 0.294 | 279 | 0.816 | 0.129 | 0.97 | 0.099 |
| 259 | 0.663 | 0.323 | 0.961 | 0.284 | 280 | 0.823 | 0.129 | 0.975 | 0.104 |
| 260 | 0.666 | 0.312 | 0.961 | 0.273 | 281 | 0.837 | 0.129 | 0.98 | 0.109 |
| 261 | 0.679 | 0.301 | 0.961 | 0.262 | 282 | 0.856 | 0.118 | 0.98 | 0.098 |
| 262 | 0.691 | 0.29 | 0.961 | 0.251 | 283 | 0.864 | 0.108 | 0.98 | 0.088 |
| 263 | 0.692 | 0.28 | 0.961 | 0.241 | 284 | 0.865 | 0.0968 | 0.98 | 0.0768 |
| 264 | 0.695 | 0.269 | 0.961 | 0.23 | 285 | 0.867 | 0.086 | 0.98 | 0.066 |
| 265 | 0.702 | 0.258 | 0.961 | 0.219 | 286 | 0.874 | 0.086 | 0.985 | 0.071 |
| 266 | 0.709 | 0.247 | 0.961 | 0.208 | 287 | 0.879 | 0.0753 | 0.985 | 0.0603 |
| 267 | 0.717 | 0.237 | 0.961 | 0.198 | 288 | 0.884 | 0.0645 | 0.985 | 0.0495 |
| 268 | 0.727 | 0.226 | 0.961 | 0.187 | 289 | 0.89 | 0.0538 | 0.985 | 0.0388 |
| 269 | 0.731 | 0.226 | 0.966 | 0.192 | 290 | 0.894 | 0.0538 | 0.99 | 0.0438 |
| 270 | 0.733 | 0.215 | 0.966 | 0.181 | 291 | 0.908 | 0.0538 | 0.995 | 0.0488 |
| 271 | 0.735 | 0.204 | 0.966 | 0.17 | 292 | 0.93 | 0.043 | 0.995 | 0.038 |
| 272 | 0.742 | 0.194 | 0.966 | 0.16 | 293 | 0.95 | 0.043 | 1 | 0.043 |
| 273 | 0.759 | 0.183 | 0.966 | 0.149 | 294 | 0.963 | 0.0323 | 1 | 0.0323 |
| 274 | 0.769 | 0.172 | 0.966 | 0.138 | 295 | 0.973 | 0.0215 | 1 | 0.0215 |
| 275 | 0.771 | 0.161 | 0.966 | 0.127 | 296 | 0.987 | 0.0108 | 1 | 0.0108 |
| 276 | 0.78 | 0.161 | 0.97 | 0.131 | 297 | Inf | 0 | 1 | 0 |
| 277 | 0.796 | 0.151 | 0.97 | 0.121 | | | | | |

Some embodiments further comprise the step of treating the subject for heart failure if NT-proBNP levels are elevated but GDF-15 levels are not; treating the subject for systemic physiological injury or inflammation, hypoperfusion, or non-cardiac frailty with a drug if GDF-15 levels are elevated but NT-proBNP are not; treating the subject with a heart failure and frailty drug if both GDF-15 and NT-proBNP levels are elevated.

Some embodiments further comprise the step of treating the subject in accordance with guideline-directed medical therapy (GDMT); wherein if the subject has elevated levels of both NT-proBNP and GDF-15, treating the subject as advanced stage D in accordance with GDMT; if the subject has elevated levels of NT-proBNP but not elevated levels of GDF-15, conducting cardiac imaging to determine the causes of cardiac dysfunction and treating the cardiac dysfunction in accordance with stage B or C in accordance with GDMT; if the subject has elevated levels of GDF-15 but not elevated levels of NT-proBNP, conducting a clinical assessment of medical comorbidities and treating the subject in accordance with stage B or C in accordance with GDMT; and if the subject does not have elevated levels of either NT-proBNP or GDF-15, treating the patient with as stage A in accordance with GDMT, particularly when the subject experiences no symptoms and/or when there are no cardiac structural abnormalities identified by imaging. In some embodiments, if the subject does not have elevated levels of either NT-proBNP or GDF-15 with/without the performance of other tests to exclude functional and/or structural abnormalities in the heart as deemed appropriate by the treating physician and according to standard practice guidelines, advising the subject on lifestyle modifications and managing particular risk factors without medical or therapeutic intervention.

In some embodiments, the stages A through D of the GDMT are based on the American College of Cardiology/American Heart Association (ACC/AHA) staging framework. In some embodiments, the treatments may be selected from one or more of pharmacological, device and other interventional therapies.

Another aspect provides a method of identifying and treating frailty, altered physiological and physical reserve, aging, or aging-related inflammation in a subject comprising the steps of

a) measuring the levels of GDF-15 in a biological sample from the subject;
b) measuring the levels of one or more biomarkers selected from the group consisting of albumin, glutamine, GlycA, phosphoglycerides, glycine, and alanine;
c) determining the overall biosignature score *p* by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = (-9.31 \pm 0.817 \times \text{sex} + (0.111 \times \text{age}) + (0.000121 \times \text{GDF-15}) + (0.355 \times \text{Gln})$$
$$+ (0.571 \times \text{albumin}) + (0.526 \times \text{GlycA})$$
$$+ (-0.256 \times \text{phosphoglycerides}) + (0.266 \times \text{Gly}) + (-0.0577 \times \text{Ala});$$

d) determining the subject as being frail if the score *p* is a value defined by a threshold value in Table 1 wherein the corresponding sensitivity and
specificity values of the threshold value add up to between 1.4 and 1.6; and wherein at least one of the sensitivity or specificity values is 0.5 or above.

In some embodiments, the score *p* is 0.15 to 0.56. In some embodiments, the score *p* is 0.259. In some embodiments, the score *p is* a threshold value having a maximum value of Youden's J statistic. In a further embodiment, the maximum value of Youden's J statistic is 0.553.
Another aspect provides a method of generating a biosignature for frailty comprising the steps of
Identifying a subpopulation with elevated levels of GDF-15;
Conducting a biomarker screen/metabolic/metabolomic profiling on the subpopulation and using mathematical modeling tools to identifying biomarkers that correlate with the subpopulation.
In some embodiments, the biomarker screen includes a disease-specific biomarker selected from one or more of a heart failure biomarker (NT-proBNP or BNP), a renal failure biomarker (serum creatinine alone or in combination with cystatin C (CysC), interleukin-18 (IL-18), kidney injury molecule-1 (KIM-1) and/or neutrophil-gelatinase-associated lipocalin (NGAL)), a panel of inflammatory biomarkers (proinflammatory cytokines, e.g. interleukins, chemokines), and a tissue-specific biomarker.
Another aspect provides a system for detecting frailty in a subject comprising:

a) a GDF-15 analyzer configured to analyze biological samples from the subject to provide a concentration of GDF-15 in the biological sample;
b) a computer programmed to execute the following steps:

i) determining the overall biosignature score *p* by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject the following equation:

$$p = \frac{\exp(H)}{1 + \exp(H)}$$

wherein

$$H = \exp(-9.31 \pm 0.817 \times \text{Sex} + (0.111 \times \text{age}) + (0.000121 \times \text{GDF-15})$$
$$+ (0.355 \times \text{Gln}) + (0.571 \times \text{albumin}) + (0.526 \times \text{GlycA})$$
$$+ (-0.256 \times \text{Phosphoglycerides}) + (0.266 \times \text{Gly})$$
$$+ (-0.0577 \times \text{Ala});$$

ii) determining the subject as being frail if the score $p$ is 0.15 to 0.56.

Another aspect provides a system for detecting frailty in a subject comprising:

a) a GDF-15 analyzer configured to analyze biological samples from the subject to provide a concentration of GDF-15 in the biological sample;
b) a computer programmed to execute at least the following:

i) inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = (-9.31 \pm 0.817 \times \text{sex} + (0.111 \times \text{age}) + (0.000121 \times \text{GDF-15})$$
$$+ (0.355 \times \text{Gln}) + (0.571 \times \text{albumin}) + (0.526 \times \text{GlycA})$$
$$+ (-0.256 \times \text{phosphoglycerides}) + (0.266 \times \text{Gly})$$
$$+ (-0.0577 \times \text{Ala});$$

or
ii) inputting the subject's age in years; sex as value of 0 if female, 1 if male; and $\log_{10}$-transformed serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = -25.54 + (-0.95 \times \text{sex}) + (0.10 \times \text{age}) + (1.20 \times \text{GDF-15}) +$$
$$(7.26 \times \text{Gln}) + (10.0 \times \text{albumin}) + (6.30 \times \text{GlycA}) + (-3.11 \times$$
$$\text{phosphoglycerides}) + (3.22 \times \text{Gly}) + (-0.97 \times \text{Ala});$$

and
iii) determining the subject as being frail if the score $p$ is 0.15 to 0.56.

Another aspect provides a method of improving the accuracy of frailty and non-frailty classification comprising using GDF-15 as a guiding biomarker with a metabolomic panel of metabolites selected from one or more of albumin, glutamine, GlycA, phosphoglycerides, glycine, and alanine; comprising the following steps:

a) measuring GDF-15 concentration in a blood serum or plasma sample from a subject using the Roche Elecsys Assay kit on a Roche Cobas e immunoassay analyzer;

b) measuring the levels of one or more biomarkers selected from the group consisting of albumin, glutamine, GlycA, phosphoglycerides, glycine, and alanine using $^1$H-NMR Nightingale metabolomic profiling;

c) determining the overall biosignature score *p* by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp{(H)}}{1 + \exp{(H)}}$$

wherein

$$H = \exp(-9.31 \pm 0.817 \times \text{sex} + (0.111 \times \text{age}) + (0.000121 \times \text{GDF-15}) +$$
$$(0.355 \times \text{Gln}) + (0.571 \times \text{albumin}) + (0.526 \times \text{GlycA}) + (-0.256 \times$$
$$\text{phosphoglycerides}) + (0.266 \times \text{Gly}) + (-0.0577 \times \text{Ala});$$

and
d) determining the subject as being frail if the score *p* is 0.15 to 0.56.

Another aspect provides a method of identifying subjects who will have improved survival outcomes when treated with exercise therapy, physical therapy, physiotherapy, nutritional supplementation (amino acid(s)/leucine (in non-cardiac failure)/protein), or administering a therapeutic drug for treating impaired cardiovascular or cardiopulmonary function; comprising the steps of

a) determining the overall biosignature score *p* by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp{(H)}}{1 + \exp{(H)}}$$

wherein

$$H = (-9.31 \pm 0.817 \times \text{sex} + (0.111 \times \text{age}) + (0.000121 \times \text{GDF-15}) + (0.355 \times \text{Gln})$$
$$+ (0.571 \times \text{albumin}) + (0.526 \times \text{GlycA})$$
$$+ (-0.256 \times \text{phosphoglycerides}) + (0.266 \times \text{Gly}) + (-0.0577 \times \text{Ala});$$

b) determining the subject as being frail if the score *p* is 0.15 to 0.56; and

c) treating the subjects determined as being frail with exercise therapy, physical therapy, physiotherapy, nutritional supplementation (amino acid(s)/leucine (in non-cardiac failure)/protein), or administering a therapeutic drug for treating impaired cardiovascular or cardiopulmonary function.

Another aspect provides a method of identifying subjects who will have improved survival outcomes when treated with exercise therapy, physical therapy, physiotherapy, nutritional supplementation (amino acid(s)/leucine (in non-cardiac failure)/protein), or administering a therapeutic drug for treating impaired cardiovascular or cardiopulmonary function; comprising the steps of

a) determining the overall biosignature score *p*; and
b) determining the subject as being frail if the score *p* is 0.15 to 0.56;

wherein the score *p* is determined by

i) inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp{(H)}}{1 + \exp{(H)}}$$

wherein

$$H = (-9.31 \pm 0.817 \times \text{sex} + (0.111 \times \text{age}) + (0.000121 \times \text{GDF-15})$$
$$+ (0.355 \times \text{Gln}) + (0.571 \times \text{albumin}) + (0.526 \times \text{GlycA})$$
$$+ (-0.256 \times \text{phosphoglycerides}) + (0.266 \times \text{Gly})$$
$$+ (-0.0577 \times \text{Ala}) \, ;$$

or

ii) inputting the subject's age in years; sex as value of 0 if female, 1 if male; and $\log_{10}$-transformed serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp{(H)}}{1 + \exp{(H)}}$$

wherein

$$H = -25.54 + (-0.95 \times \text{sex}) + (0.10 \times \text{age}) + (1.20 \times \text{GDF-15})$$
$$+ (7.26 \times \text{Gln}) + (10.0 \times \text{albumin}) + (6.30 \times \text{GlycA})$$
$$+ (-3.11 \times \text{phosphoglycerides}) + (3.22 \times \text{Gly})$$
$$+ (-0.97 \times \text{Ala}).$$

and

c) treating the subjects determined as being frail with exercise therapy, physical therapy, physiotherapy, nutritional supplementation (amino acid(s)/leucine (in non-cardiac failure)/protein), or administering a therapeutic drug for treating impaired cardiovascular or cardiopulmonary function.

Another aspect provides a method of identifying and treating subjects who will have improved survival outcomes when treated with exercise therapy, physical therapy, physiotherapy, nutritional supplementation (amino acid(s)/leucine (in non-cardiac failure)/protein), or administering a therapeutic drug for treating impaired cardiovascular or cardiopulmonary function; comprising the steps of

a) determining the overall biosignature score *p*; and
b) determining the subject as being frail if the score *p* is 0.15 to 0.56;
wherein the score *p* is determined by

i) inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp\,(H)}{1 + \exp\,(H)}$$

wherein

$$H = (-9.31 \pm 0.817 \times \text{sex} + (0.111 \times \text{age}) + (0.000121 \times \text{GDF-15})$$
$$+ (0.355 \times \text{Gln}) + (0.571 \times \text{albumin}) + (0.526 \times \text{GlycA})$$
$$+ (-0.256 \times \text{phosphoglycerides}) + (0.266 \times \text{Gly})$$
$$+ (-0.0577 \times \text{Ala})\,;$$

or
ii) inputting the subject's age in years; sex as value of 0 if female, 1 if male; and $\log_{10}$-transformed serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp\,(H)}{1 + \exp\,(H)}$$

wherein

$$H = -25.54 + (-0.95 \times \text{sex}) + (0.10 \times \text{age}) + (1.20 \times \text{GDF-15})$$
$$+ (7.26 \times \text{Gln}) + (10.0 \times \text{albumin}) + (6.30 \times \text{GlycA})$$
$$+ (-3.11 \times \text{phosphoglycerides}) + (3.22 \times \text{Gly}) + (-0.97 \times \text{Ala}).$$

and

c) treating the subjects determined as being frail with exercise therapy, physical therapy, physiotherapy, nutritional supplementation (amino acid(s)/leucine (in non-cardiac failure)/protein), or administering a therapeutic drug for treating impaired cardiovascular or cardiopulmonary function.

Another aspect provides a kit for evaluating frailty comprising

a) a test for measuring blood levels of GDF-15; and
b) optionally one or more tests for measuring blood levels of albumin, glutamine, GlycA, phosphoglycerides, glycine, and alanine.

In some embodiments, the test for measuring albumin, glutamine, GlycA, and phosphoglyceride is the $^1$H-NMR Nightingale system.
Another embodiment provides the use of tissue-specific blood biomarkers (e.g. NT-proBNP) to identify impaired organ or organ system (e,g. cardiac failure) as a subclassification (or subphenotyping) of frailty. In one embodiment, elevation of both circulating NT-proBNP and GDF-15 levels indicate CD and systemic tissue injury or hypoperfusion, and increase the probability of a diagnosis of heart failure and frailty (cardiac frailty). In another embodiment, sole elevation of NT-proBNP but not GDF-15 indicates cardiac dysfunction that is not so extensive as to cause systemic physiological compromise (cardiac dysfunction without frailty). In yet another embodiment, elevation of GDF-15 alone (with normal NT-proBNP levels) indicates systemic physiological injury, hypoperfusion or abnormalities that are less likely to be attributable cardiac dysfunction (noncardiac frailty). In some embodiments, GDF-15 elevation broadly indicates systemic tissue injury, inflammation, compromised systemic physiology and impaired physical fitness that characterize frailty (e.g. reduced skeletal muscle growth, weight loss, reduced appetite, easy fatigability).
In conjunction with physical measures (e.g. 6-minute walk distance (6MWD), gait speed, handgrip strength), GDF-15 is a useful biomarker for the classification and stratification of frailty classes.
In some embodiments, GDF-15 elevation is defined as having GDF-15 blood levels greater than 1000 pg/ml.

In some embodiments, the GDF-15 blood levels are in a range between 1,000 to 6,000 pg/ml, 1000 to 4000 pg/ml, 2000 to 4000 pg/ml, 2500 to 3500 pg/ml, or 3000 $\pm$ 1000 pg/ml. In some embodiments, the GDF-15 blood level is 3,206.6 $\pm$ 2,565.4 pg/ml.

## EXAMPLES

Provided herein are examples that describe in more detail certain embodiments of the present disclosure. The examples provided herein are merely for illustrative purposes and are not meant to limit the scope of the invention in any way. All references given below and elsewhere in the present application are hereby included by reference.

## EXAMPLE 1

Blood levels of a number of biomarkers and metabolites were measured in 306 subjects (derivation set). Blood serum levels of GDF-15 and NT-proBNP were measured using the Roche ELECSYS® GDF-15 Assay kit and a Roche COBAS® e immunoassay analyzer, or a compatible instrument, as per manufacturer.

Subjects were also evaluated based on the FRAIL Scale [Abellan van Kan 2008; Morley 2012; Woo 2012]. The 5-point FRAIL scale is a multi-domain instrument that assesses the key deficits and risks associated with frailty. A subject is frail if the score is 3 to 5; pre-frail if the score is 1 to 2; and robust if the score is 0.

It shall be understood that frailty severity can be defined by many different types of scores or methods, and there are other known frailty scoring methods that could stand in the place of the FRAIL scale, such as the Edmonton frail scale [Rolfson 2006]), or a cumulative deficit approach whereby an index is calculated from the proportion of health and medical problems relative to a predefined inventory (e.g. Rockwood frailty index)[Mitnitski 2002; Rockwood 2011].

Logistic regression analysis was done with adjustments for age and sex. Biomarker levels were expressed as mean $\pm$ standard deviation (SD). Kruskal-Wallis H test with Dunn post hoc test, Chi-square or Fisher's exact test were used to compare differences between groups. [a]$P$ <0.05, pre-frail vs. robust; [b]$P$ <0.05, frail vs. robust; [c]$P$ <0.05, frail vs. pre-frail (Table 2). The workflow of age- and sex-adjusted linear regression is shown in Fig. 7. The continuous variable of $\log_{10}$-transformed NT-proBNP or GDF-15 level, was modeled on each (transformed) metabolite or metabolic feature as the dependent variable.

## GDF-15 as an Indicator of Frailty

Table 2 shows that circulating blood levels of the biomarkers, NT-proBNP and GDF-15, can indicate non-frailty and frailty irrespective of the etiology. Table 2 also shows that subjects with GDF-15 blood levels in the range of 3,206.6 $\pm$ 2,565.4 pg/ml were confirmed frail according to the FRAIL scale.

**Table 2**

| Variables | Robust | Pre-frail | Frail | Overall |
|---|---|---|---|---|
| Sample size, $n$ | 104 | 107 | 95 | 306 |
| NT-proBNP, *pg/ml* | 180.8 $\pm$ 506.1 | 239.4 $\pm$ 466.4[a] | 361.0 $\pm$ 745.5[b,c] | 257.2 $\pm$ 582.1 |
| *GDF-15,pg/ml* | 1,667.4 $\pm$ 1,114.9 | 2,220.0 $\pm$ 1,781.3[a] | 3,206.6 $\pm$ 2,565.4[b,c] | 2,338.5 $\pm$ 1,986.0 |

## GDF-15 Surprisingly Effective as a Differentiator of Frailty vs. Non-Frailty

Table 3 shows that $\log_{10}$-transformed GDF-15 does predict and differentiate frailty from non-frailty (P = 1.29 $\times$ 10$^{-3}$) whereas NT-proBNP does not (P = not significant (NS)). NT-proBNP is a strong independent predictor of CD, whereas GDF-15 can independently differentiate between individuals with and without frailty.

**Table 3**

| | Log$_{10}$ NT-proBNP | | Log$_{10}$ GDF-15 | |
|---|---|---|---|---|
| Comparisons | OR (95% CI) | $P$ | OR (95% CI) | $P$ |
| **Frailty vs. non-frailty** | 1.07 (0.95-1.20) | NS | 1.38 (1.13-1.67) | **1.29 $\times$ 10$^{-3}$** |
| CD vs. non-CD | 1.47 (1.33-1.62) | **5.49 $\times$ 10$^{-13}$** | 1.02 (0.85-1.23) | NS |

**Comparison Data: GDF-15 vs. NT-proBNP in Prediction of Frailty & CD**

Table 4 shows multiple linear regression analysis of phenotypic variables modeling on $\log_{10}$ NT-proBNP and $\log_{10}$ GDF-15 levels as dependent variables identifying CD and frailty as their respective explanatory factors. Table 4 further confirms that elevated GDF-15 levels are predictive of subjects with CD and frailty.

## Table 4

| Independent variables | Log₁₀ NT-proBNP | | | | Log₁₀ GDF-15 | | | |
|---|---|---|---|---|---|---|---|---|
| | $\beta$ | SE | t | *P*-values | $\beta$ | SE | t | *P*-values |
| Age, *y* | 0.02 | 0.00 | 7.27 | $3.23 \times 10^{-12}$ | 0.01 | 0.00 | 7.33 | $2.18 \times 10^{-12}$ |
| Sex, % female | -0.03 | 0.05 | -0.65 | NS | 0.02 | 0.03 | 0.49 | NS |
| BMI | 0.00 | 0.01 | 0.05 | NS | 0.01 | 0.00 | 1.94 | NS |
| Frailty status | 0.05 | 0.03 | 1.47 | NS | 0.07 | 0.02 | 3.38 | **$8.36 \times 10^{-4}$** |
| CD | 0.41 | 0.06 | 7.37 | **$1.71 \times 10^{-12}$** | -0.01 | 0.03 | -0.22 | NS |
| $R^2$ | | | 0.39 | | | | 0.27 | |

BMI, body mass index; CD, cardiac dysfunction; GDF-15, growth differentiation factor 15; NS, not significant; NT-proBNP, N-terminal prohormone of B-type natriuretic peptide; SE, standard error

**NT-proBNP**

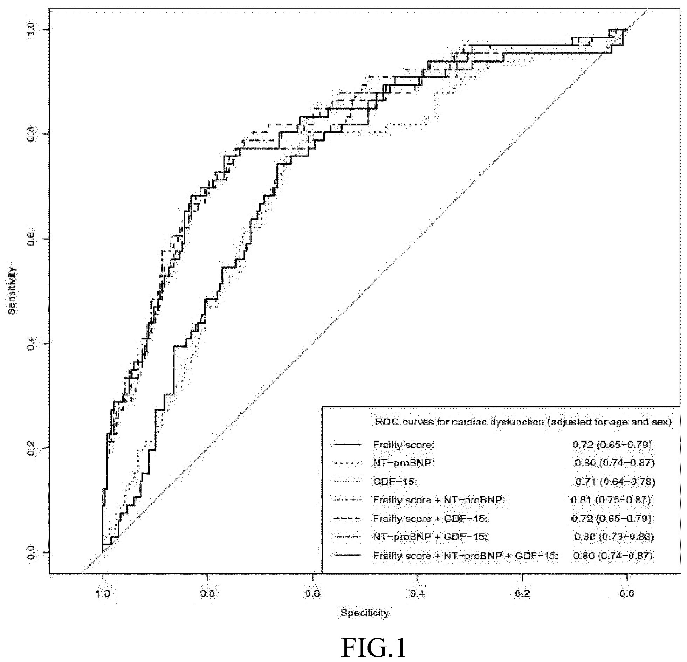

Fig. 1 shows how NT-proBNP independently distinguishes older adults with and without CD (n = 306 subjects). Adding additional variables including frailty (FRAIL score) and/or GDF-15 did not further improve the predictive performance of NT-proBNP for CD. DeLong test was used to test for statistical difference between classifiers.

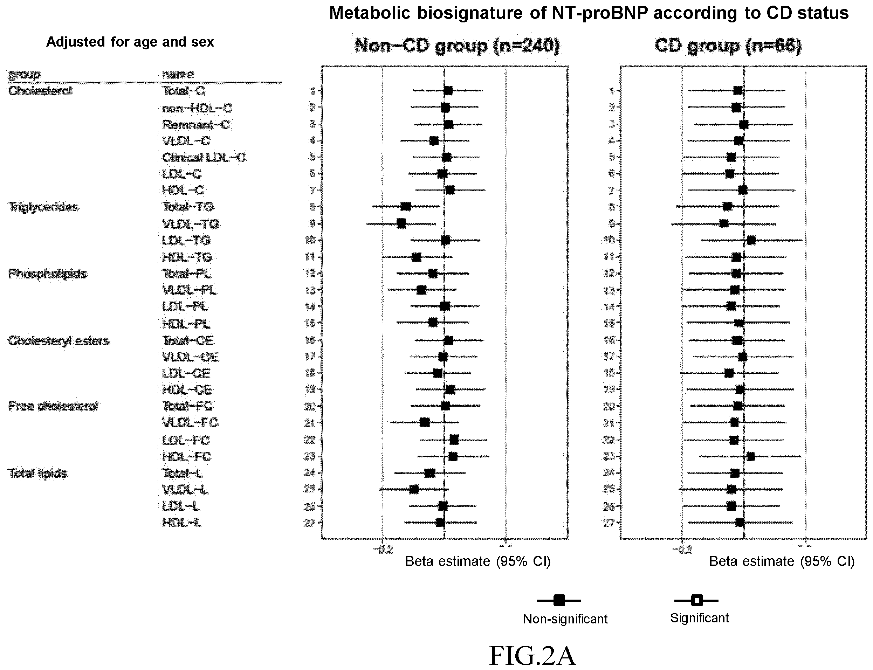

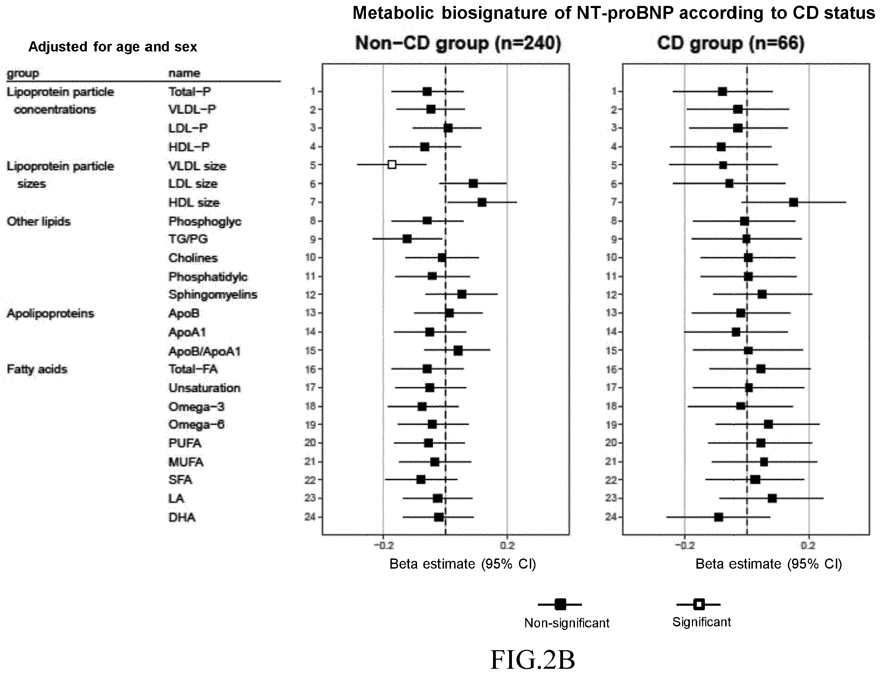

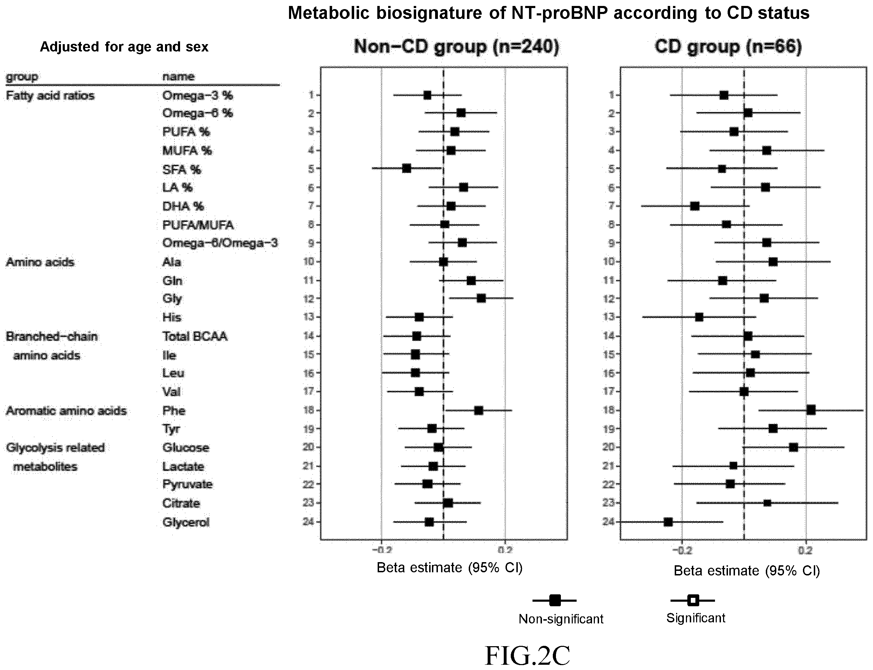

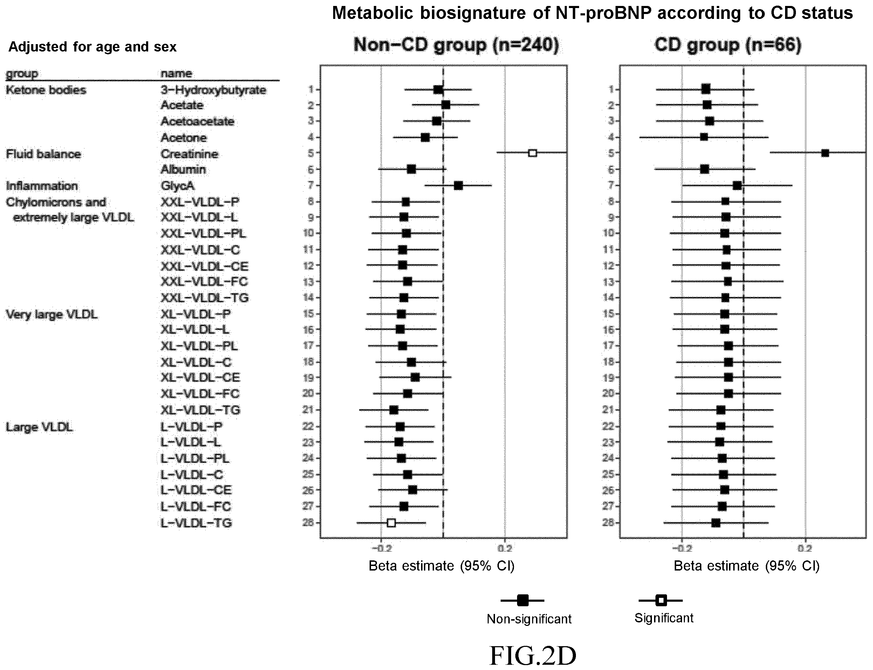

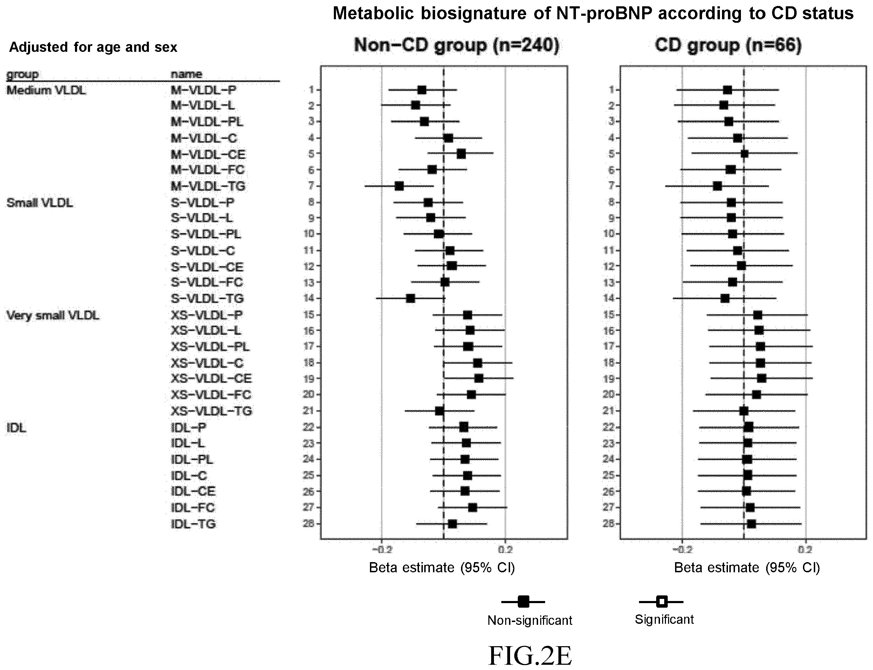

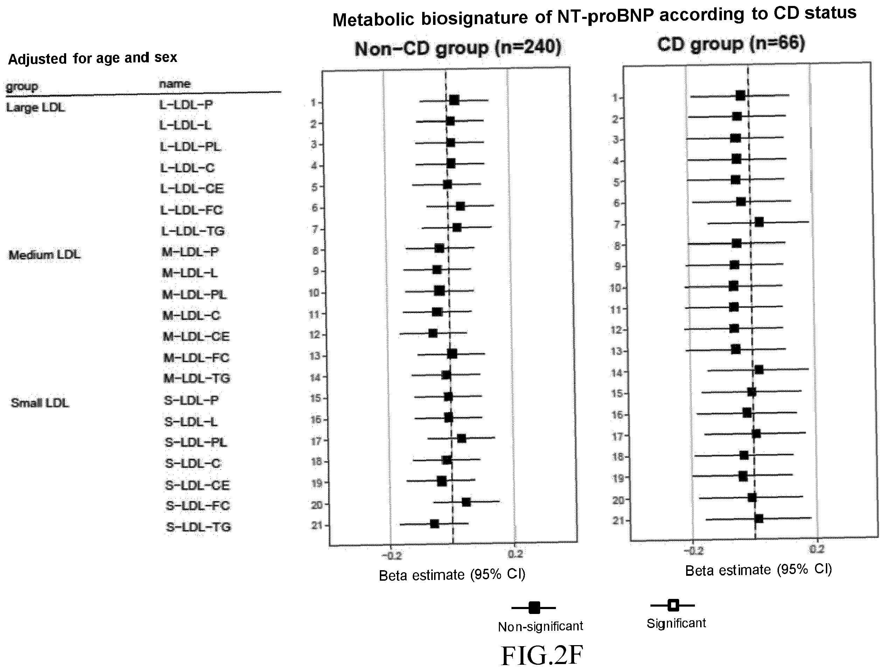

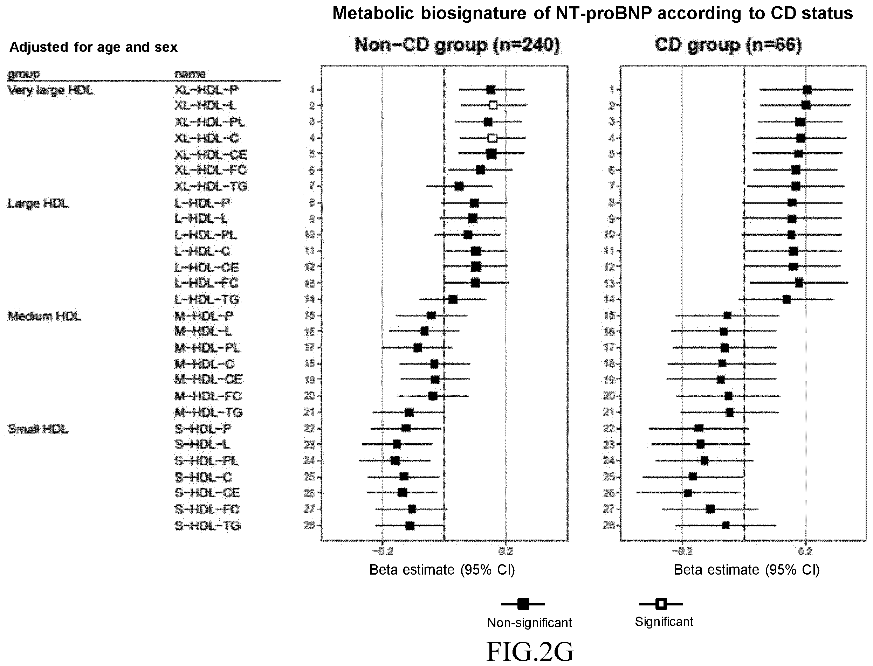

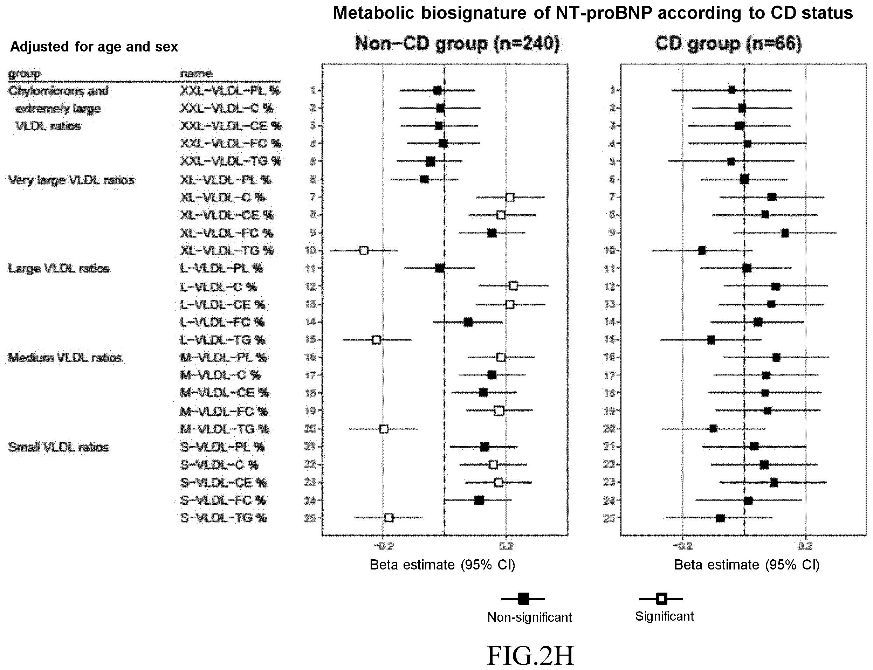

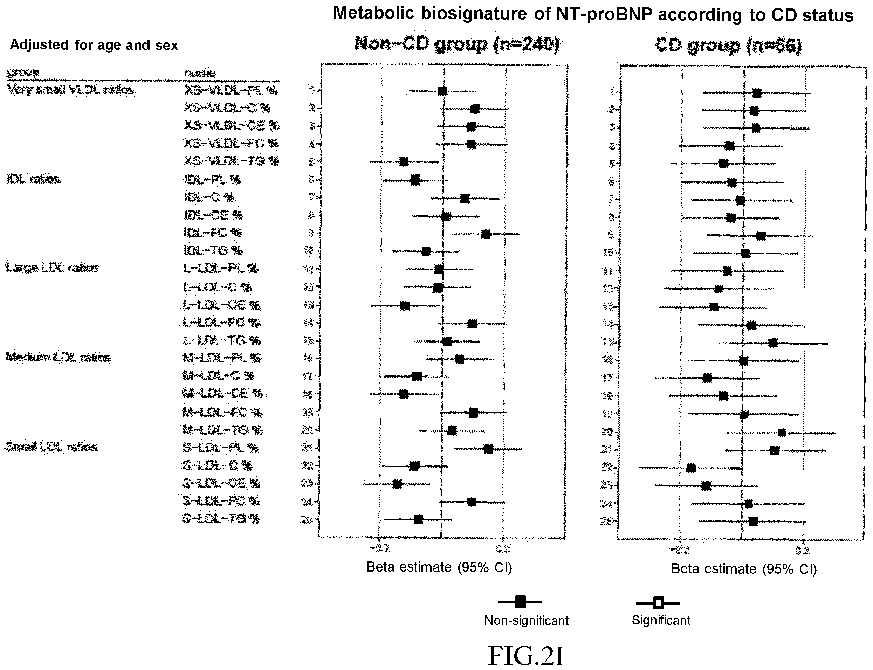

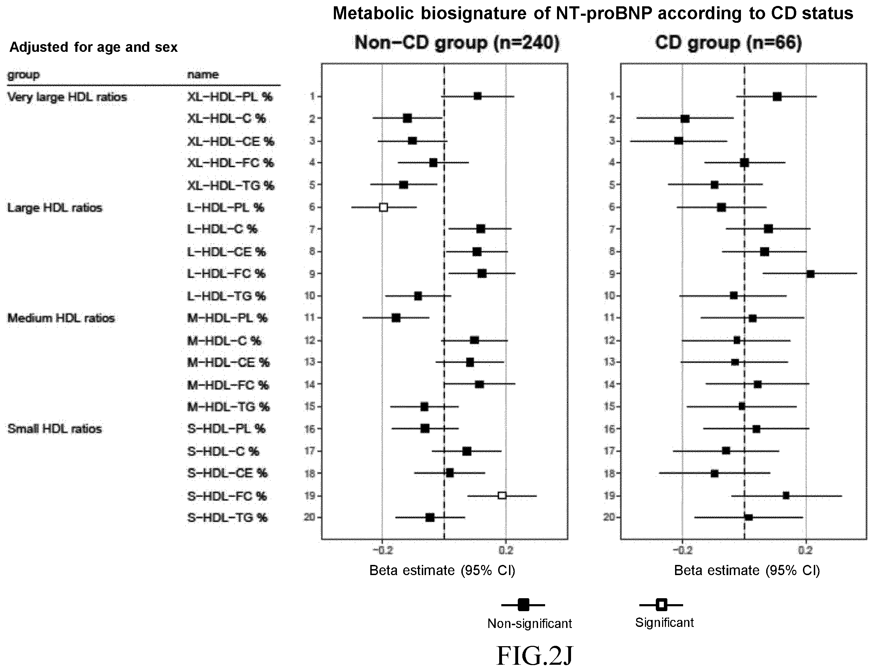

Figs. 2A-J show metabolomic biosignatures of NT-proBNP generated using linear regression for all 250 metabolites/metabolic features and the strength of the association between NT-proBNP and the metabolites measured using the $\beta$ coefficient values. Individuals with CD and without CD (non-CD) were classified according to whether or not echocardiographic CD was present. Metabolomic biosignature of NT-proBNP classified according to whether or not echocardiographic CD is present. Linear regression with adjustment for age and sex was used to estimate the strength of association ($\beta$ coefficient) between each metabolite/metabolic feature and NT-proBNP in non-CD and CD groups. Refer to **Table 8** for identity of metabolite number.

**Pairwise Comparisons without GDF-15 Guidance Leads to Weaker Predictive Abilities**

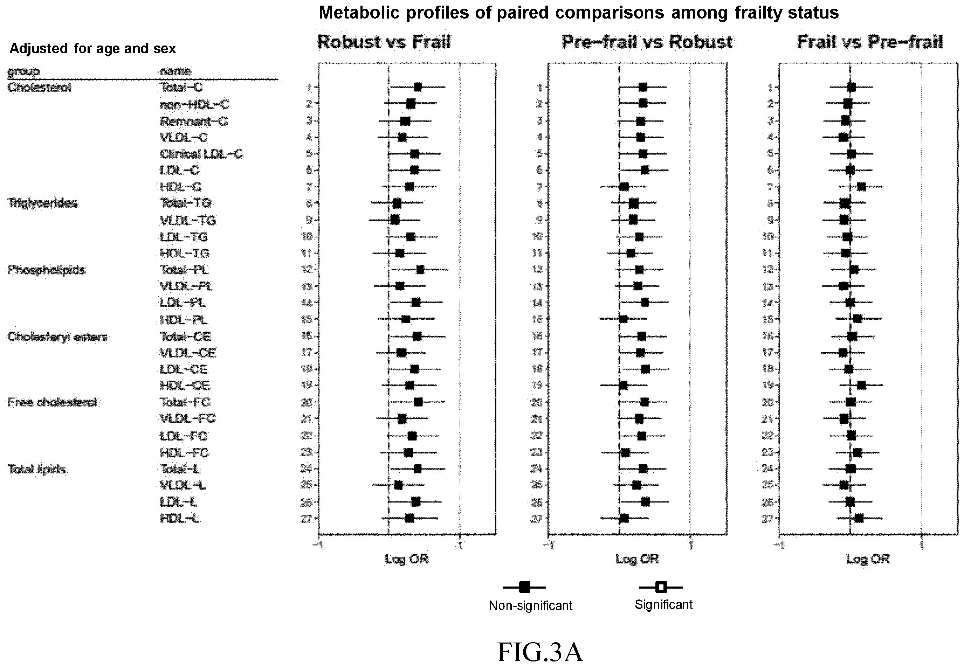

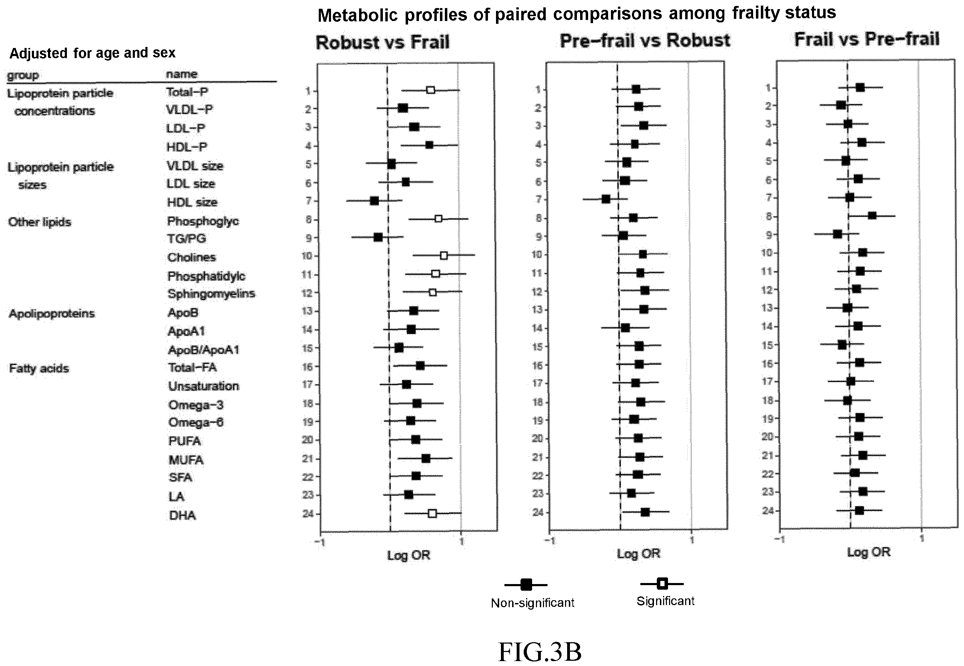

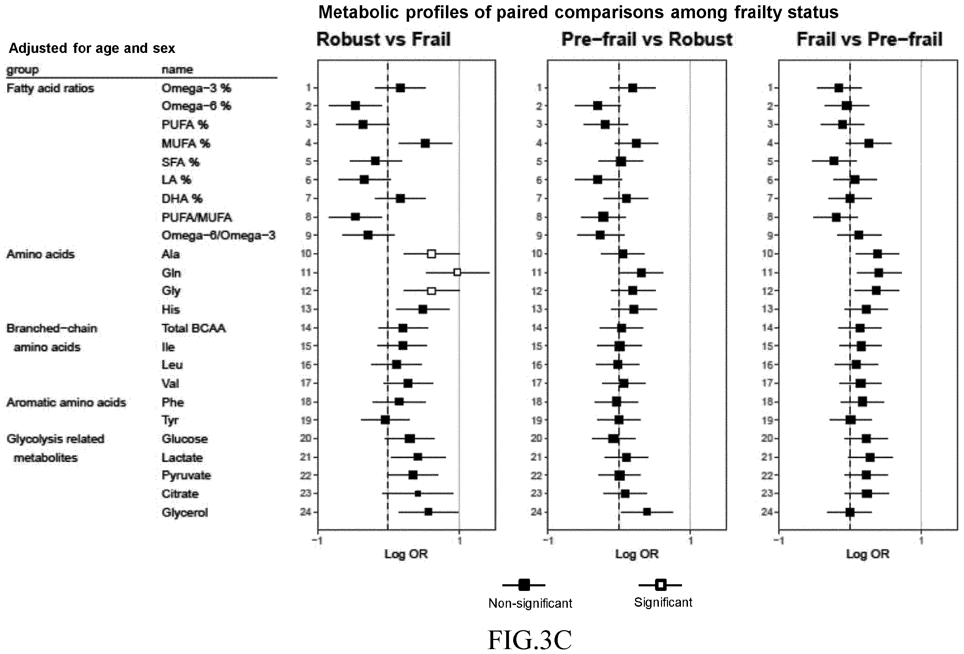

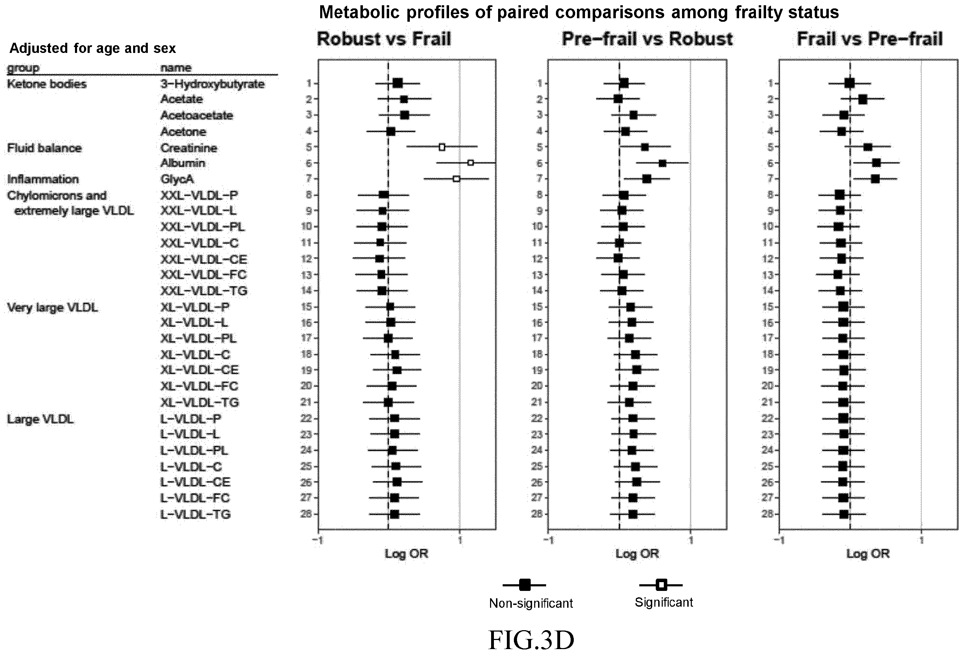

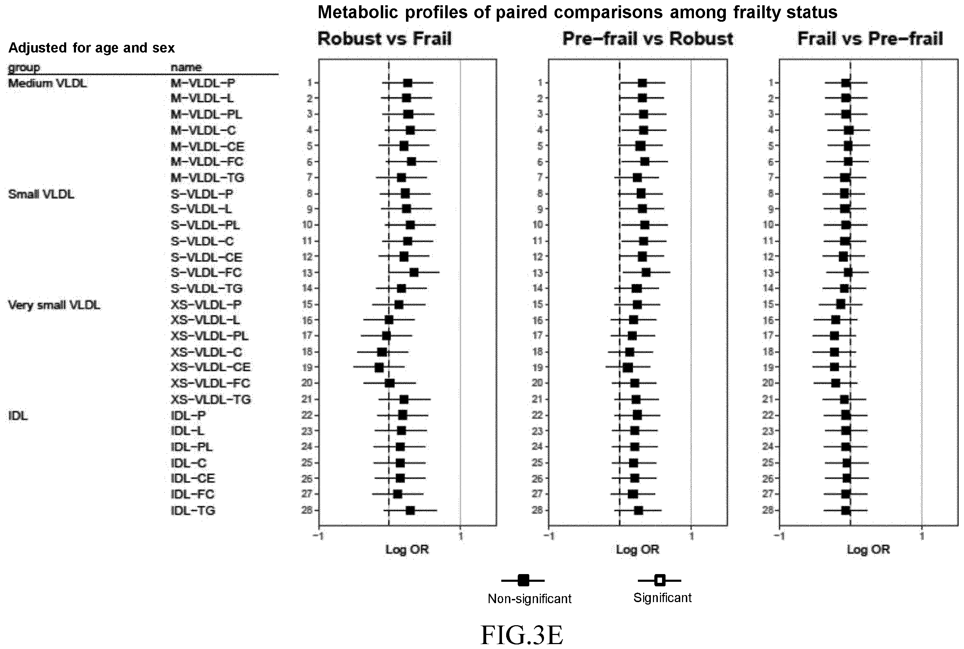

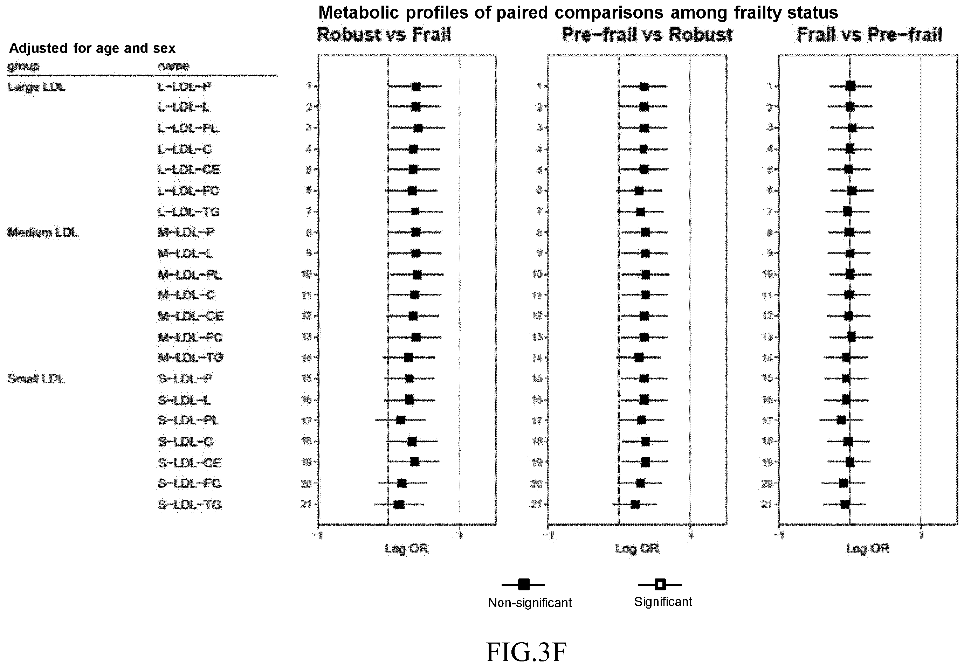

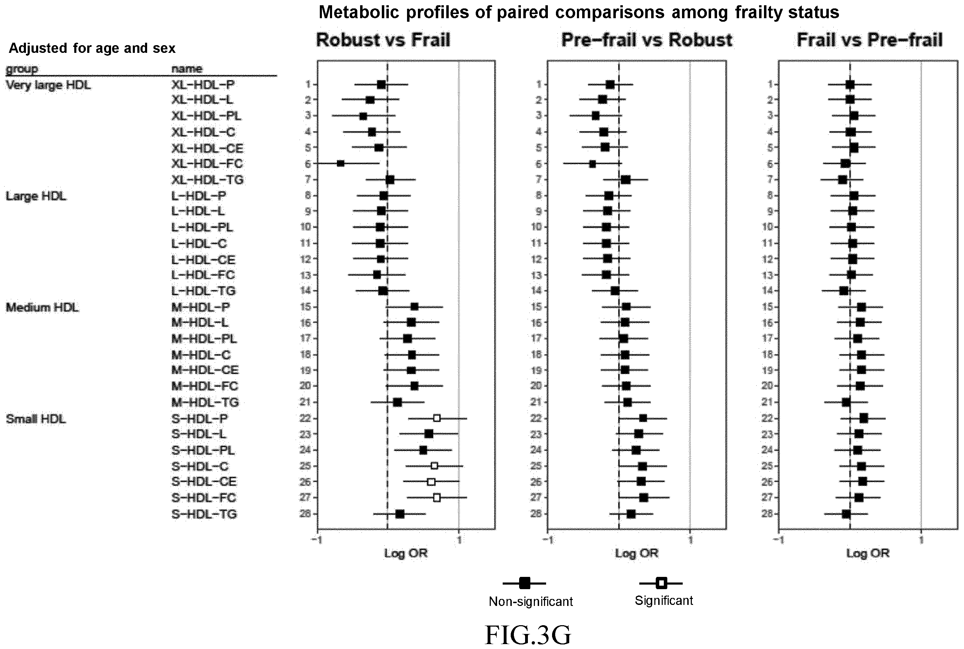

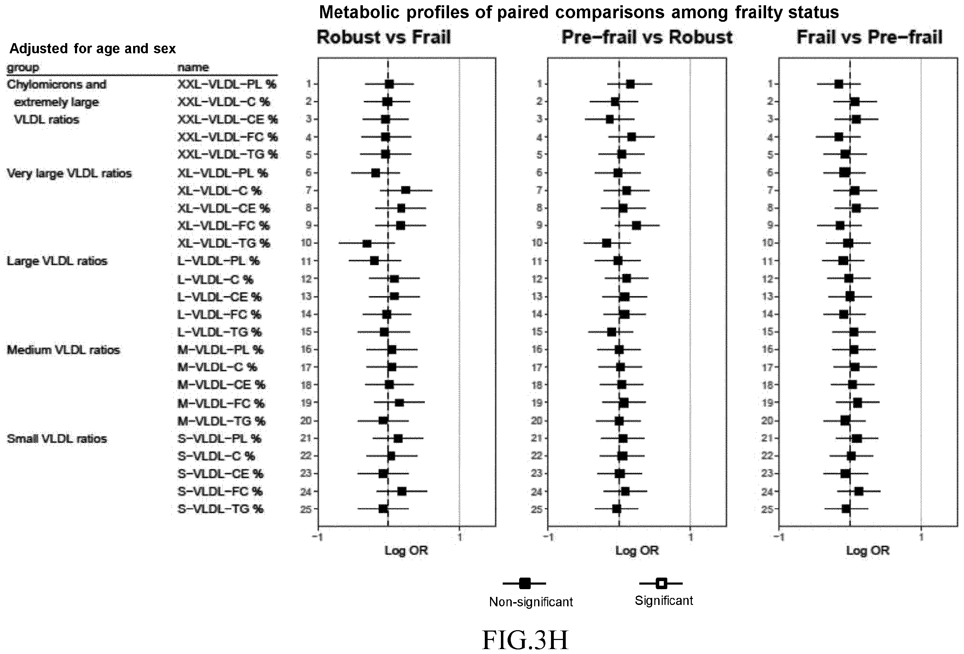

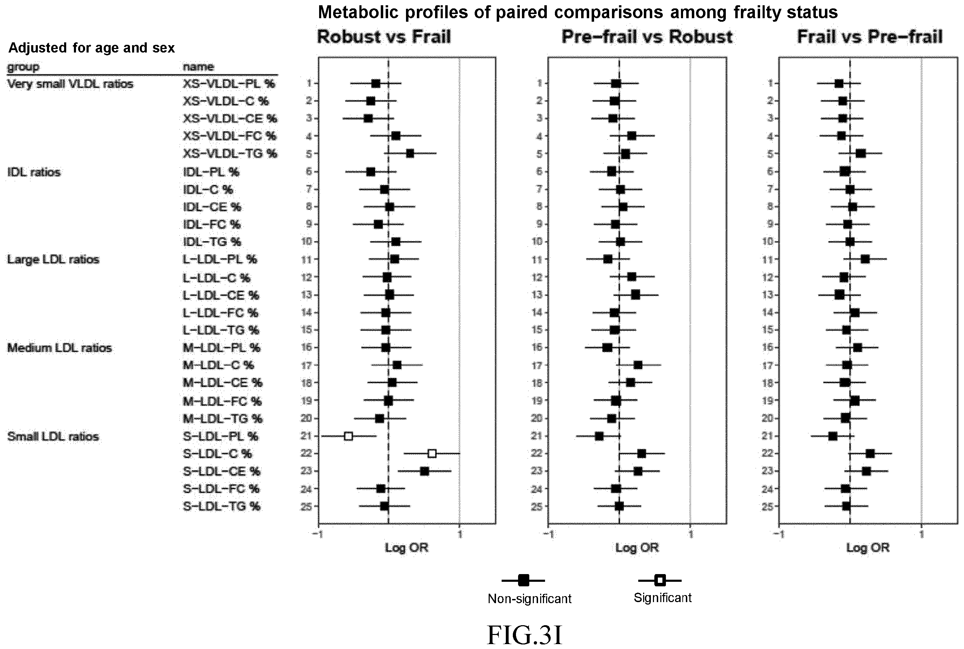

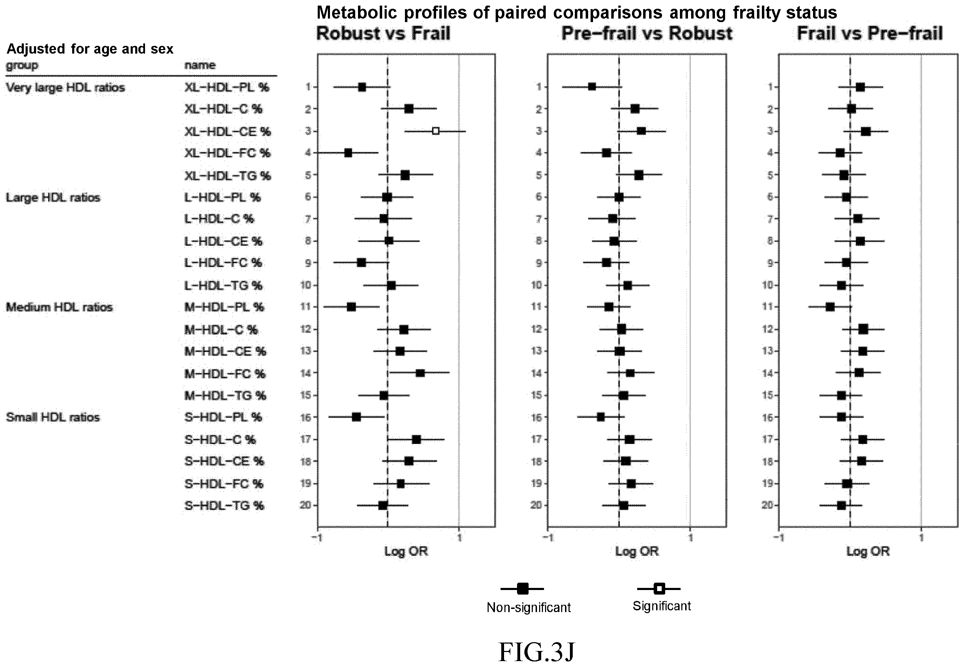

Figs. 3A-J show that without biomarker guidance, pairwise comparisons between frail and non-frail groups (frail vs. robust; pre-frail vs. robust; frail vs. pre-frail) are possible. In Figs. 3A-J, logistic regression with adjustment for age and sex is used to model each metabolite/metabolic feature on frailty status. Odds ratios are used to estimate the direction, size, and strength of the association between the metabolite/metabolic feature and the frailty or non-frailty phenotype. No biomarker is used in this analysis. Statistically significant variables are highlighted in blue. Refer to **Table 8** for identity of metabolite number.

Figs. 4A(i)-4A(x), Figs. 4B(i)-4B(x) and Figs. 4C(i)-4C(x) show the metabolomic biosignature of GDF-15 classified according to frailty status (robust, pre-frail or frail). The metabolomic biosignature of GDF-15 was generated using linear regression for all 250 metabolites/metabolic features (see Table 8 for metabolites). Linear regression with adjustment for age and sex was used to estimate the strength of association ($\beta$ coefficient) between each metabolite/metabolic feature and GDF-15 in robust, pre-frail or frail groups. $\beta$ coefficient values were calculated from correlating each metabolite/ metabolic feature against its respective GDF-15 level. Refer to **Table 8** for identity of metabolite number. Of note, the GDF-15-guided metabolomic biosignature for the robust and pre-frail (collectively, non-frail) groups are not markedly similar, whereas a plethora of statistically different metabolites/metabolic features highlighted as shown is evident. At the individual metabolite/metabolic feature level, the identities are shown in **Table 6**

**Table 6.** Frailty-related metabolic biosignatures in different studies.[Fung 2018; Pujos-Guillot 2018; Marron 2019].

| | NU-AGE study | | | | Health ABC study | | | UFO study | | |
|---|---|---|---|---|---|---|---|---|---|---|
| Study population | Free-living elderly in Europe | | | | Community-dwelling black men in America | | | Community-dwelling elderly in HK (China) | | |
| Frailty assessment | Fried et al. (2001) | | | | SAVE score | | | FRAIL score | | |
| Subgroups | Robust male | Pre-frail male | Robust female | Pre-frail female | Vigorous (range: 0-3) | Average (range: 4-5) | Frail (range: 6-10) | Robust | Pre-frail | Frail |
| Sample size | 60 | 31 | 67 | 54 | 73 | 105 | 109 | 104 | 107 | 95 |
| Age, y | 71 ±4 | 73 ± 4 | 71 ± 4 | 72 ± 4 | 74 ± 3 | 75 ± 3 | 75 ± 3 | 71 ± 6 | 74 ± 8 | 79 ± 8 |
| Sex, % female | 0 | 0 | 100 | 100 | 0 | 0 | 0 | 42 | 81 | 84 |
| Metabolomics platform | UPLC coupled to QTOF-MS | | | | LC-MS | | | $^1$H-NMR | | |
| Blood sample types | Serum | | | | Overnight-fasting plasma | | | Non-fasting serum | | |
| Significant metabolites or metabolic features | Significant metabolites for pre-frailty at baseline in males (stable):<br>- Pipecolic acid ↑<br>- 2,3-dihydromethylpyrrole ↑<br>- Proline ↑<br>- Butyrylcarnitine ↑<br>Significant metabolites for pre-frailty at baseline in females (stable):<br>- Amino-octanoic acid ↓<br>Significant metabolites for pre-frailty at baseline in males (improved):<br>- **Glutamine** ↑<br>- Mannose ↓<br>- Gly-Phe ↓<br>Significant metabolites for pre-frailty at baseline in females (improved):<br>- Threonine ↑<br>- Fructose ↓<br>- Phenylalanine ↓ | | | | 8 metabolites positively correlated with SAVE scores:<br>- Glucoronate<br>- N-carbamoyl-beta-alanine<br>- Isocitrate<br>- **Creatinine**<br>- C4-OH carnitine<br>- Cystathionine<br>- Hydroxyphenylacetate<br>- Putrescine<br>6 metabolites negatively correlated with SAVE scores:<br>- Tryptophan<br>- Methionine<br>- Tyrosine<br>- C14:0 sphingomyelin<br>- 1-methylnicotinamide<br>- asparagine | | | Compared with robust, the frail group:<br>- Total concentration of lipoprotein particles↑<br>- Phosphoglycerides ↑<br>- Cholines ↑<br>- Phosphatidylcholines ↑<br>- Sphingomyelins ↑<br>- Docosahexaenoic acid ↑<br>- Alanine ↑<br>- **Glutamine** ↑<br>- Glycerol ↑<br>- **Creatinine** ↑<br>- Albumin ↑<br>- GlycA ↑<br>- Concentrations of particles in the S_HDL ↑<br>- Concentrations of free cholesterol in the S_HDL ↑<br>- Ratio of cholesterol to total lipids in S_LDL ↑<br>- Ratio of cholesterol esters to total lipids in XL_HDL ↑<br>- Ratio of free cholesterol to total lipids in XL_HDL ↓<br>- Ratio of phospholipids to total lipids in M_HDL ↓ | | |

$^1$H-NMR, proton nuclear magnetic resonance; LC-MS, liquid chromatography-mass spectrometry; QTOF-MS, quadrupole time-of-flight mass spectrometry; UPLC, ultra-performance liquid chromatography

**GDF-15 Predictive of Reduced Physical Activity**

**Table 5** below shows how NT-proBNP or GDF-15 levels impact other physical fitness measures (recognized surrogate markers of frailty and physical fitness) using Spearman's test with adjustment for age and sex. Both NT-proBNP or GDF-15 are markers of functional and physical domains of frailty. The data show that GDF-15 is significantly and inversely correlated with physical fitness and strength.

**GDF-15 Guided Metabolomic Signature Most Predictive of Frailty**

Figs. 5A-B show the validation of the combined classifier of metabolites/metabolic features (albumin, glutamine, and glycoprotein actetylation marker of inflammation (GlycA) [Bell 1987; Otyos 2015; Ritchie 2015] that met the FDR 5% (from 4A(i)-4A(x), 4B(i)-4B(x) and 4C(i)-4C(x)) with addition of phosphoglycerides (Table 7), age, sex and GDF-15 to demonstrate an excellent predictive capacity of AUC 0.841 for prediction of frailty against non-frailty.

Fig. 6 shows a workflow of logistic regression analysis with adjustment for age and sex. A binary variable is modelled on each (transformed) metabolite or metabolic feature as the dependent variable.

**Table 5**

| Physical fitness measures | Model with adjustment for age and sex | | | |
|---|---|---|---|---|
| | NT-proBNP | | GDF-15 | |
| | Rho | *P* | Rho | *P* |
| 6-minute walk distance | -0.13 | **0.02** | -0.29 | **4.71 × 10$^{-7}$** |
| Gait speed | -0.10 | NS | -0.26 | **3,14 × 10$^{-6}$** |
| HGS/BMI | -0.06 | NS | -0.20 | **3.80 × 10$^{-4}$** |
| HGS/BMI, handgrip strength indexed to body mass index; NS, not significant. | | | | |

**EXAMPLE 2: Metabolic Profiling**

Metabolomic profiling of blood samples from 306 subjects was done using $^1$H-NMR (Nightingale Health Ltd (Helsinki, Finland) [Soininen P, et al. Circ Cardiovasc Genet 2015;8:192-206]). Table 8 shows the biomarkers profiled. Fresh blood serum or newly thawed specimens retrieved from -80°C storage (or in transit on dry ice) were processed on the Nightingale proprietary platform and a proprietary Nightingale algorithm was used to identify and quantify levels of GlycA, phosphoglycerides, albumin and glutamine based on $^1$H-NMR spectral data.

Univariate regression, adjusted logistic (Fig. 6) and linear regression analyses (Fig. 7) was done to identify associations represented by β values between the biomarker (e.g. NT-proBNP, GDF-15) and each metabolite/metabolic feature and the dependent variable for the different clinical phenotypes or subphenotypes under study (Figs. 2A-J, 3A-J, 4A(i)-4A(x), 4B(i)-4B(x) and 4C(i)-4C(x)).

A series of biosignatures were generated for each group or subgroup using a biomarker-guided metabolomic profiling strategy (Figs. 6-7 and Table 7), to show the significant correlations (β values) between the biomarker and the metabolites/metabolic feature (Figs. 2A-J, 3A-J, 4A(i)-4A(x), 4B(i)-4B(x) and 4C(i)-4C(x)). The differences in the patterns of each group's metabolome can be visualized in a forest plot and the statistically significant findings are highlighted in the respective figures for the particular metabolites/metabolic features that reach the stringent false discovery rate (FDR) cut-off of 0.05 (5%).

Table 7 below shows how subjects who had GDF-15 levels that highly correlated with the following three or six biomarkers also showed the phenotype of frailty according to the FRAIL scale. Significant metabolites/metabolic features and area under the receiver operating curve (AUC) values at the respective false-discovery rates (FDR) are shown. Incremental lowering of the FDR threshold from 0.05 (standard) to 0.135 and beyond yields a greater number of metabolites/metabolic features.

**Table 7**

| FDR | Significant metabolic features (along with age, sex, GDF-15) | AUC |
|---|---|---|
| 0.05 | Albumin, Gln, GlycA | 0.8181 |
| 0.075 | Albumin, Gln, GlycA | 0.8181 |
| 0.1 | Albumin, Gln, GlycA | 0.8181 |

(continued)

| FDR | Significant metabolic features (along with age, sex, GDF-15) | AUC |
|---|---|---|
| 0.125 | Albumin, Gln, GlycA | 0.8181 |
| 0.135 | Albumin, Gln, GlycA, Phosphoglycerides, Gly, Ala | 0.8438 |
| 0.15 | Albumin, Gln, GlycA, Phosphoglycerides, Gly, Ala | 0.8438 |
| 0.175 | Albumin, Gln, GlycA, Phosphoglycerides, Gly, Ala | 0.8438 |
| 0.2 | Albumin, Gln, GlycA, Phosphoglycerides, Gly, Ala | 0.8438 |

**Table 8** shows a list of 250 metabolites/metabolic features analyzed by Nightingale's $^1$H-NMR platform.

Table 8

| No. | CHOLESTEROL | Units |
|---|---|---|
| 001 | Total cholesterol | mmol/l |
| 002 | Total cholesterol minus HDL-C | mmol/l |
| 003 | Remnant cholesterol (non-HDL, non-LDL-cholesterol) | mmol/l |
| 004 | VLDL cholesterol | mmol/l |
| 005 | Clinical LDL cholesterol | mmol/l |
| 006 | LDL cholesterol | mmol/l |
| 007 | HDL cholesterol | mmol/l |
| | | |
| | TRIGLYCERIDES | |
| 008 | Total triglycerides | mmol/l |
| 009 | Triglycerides in VLDL | mmol/l |
| 010 | Triglycerides in LDL | mmol/l |
| 011 | Triglycerides in HDL | mmol/l |
| | | |
| | PHOSPHOLIPIDS | |
| 012 | Total phospholipids in lipoprotein particles | mmol/l |
| 013 | Phospholipids in VLDL | mmol/l |
| 014 | Phospholipids in LDL | mmol/l |
| 015 | Phospholipids in HDL | mmol/l |
| | | |
| | CHOLSTERYL ESTERS | |
| 016 | Total esterified cholesterol | mmol/l |
| 017 | Cholesteryl esters in VLDL | mmol/l |
| 018 | Cholesteryl esters in LDL | mmol/l |
| 019 | Cholesteryl esters in HDL | mmol/l |
| | | |
| | FREE CHOLESTEROL | |
| 020 | Total free cholesterol | mmol/l |
| 021 | Free cholesterol in VLDL | mmol/l |

(continued)

| | | FREE CHOLESTEROL | |
|---|---|---|---|
| | 022 | Free cholesterol in LDL | mmol/l |
| | 023 | Free cholesterol in HDL | mmol/l |
| | | | |
| | | **TOTAL LIPIDS** | |
| | 024 | Total lipids in lipoprotein particles | mmol/l |
| | 025 | Total lipids in VLDL | mmol/l |
| | 026 | Total lipids in LDL | mmol/l |
| | 027 | Total lipids in HDL | mmol/l |
| | | | |
| | | **LIPOPROTEIN PARTICLE CONCENTRATIONS** | |
| | 028 | Total concentration of lipoprotein particles | mmol/l |
| | 029 | Concentration of VLDL particles | mmol/l |
| | 030 | Concentration of LDL particles | mmol/l |
| | 031 | Concentration of HDL particles | mmol/l |
| | | | |
| | | **LIPOPROTEIN PARTICLE SIZES** | |
| | 032 | Average diameter for VLDL particles | nm |
| | 033 | Average diameter for LDL particles | nm |
| | 034 | Average diameter for HDL particles | nm |
| | 035 | Phosphoglycerides | mmol/l |
| | 036 | Ratio of triglycerides to phosphoglycerides | ratio (%) |
| | 037 | Total cholines | mmol/l |
| | 038 | Phosphatidylcholines | mmol/l |
| | 039 | Sphingomyelins | mmol/l |
| | | | |
| | | **APOLIPOPROTEINS** | |
| | 040 | Apolipoprotein B | g/l |
| | 041 | Apoliproprotein A1 | g/l |
| | 042 | Ratio of apolipoprotein B to apolipoprotein A1 | ratio (%) |
| | | | |
| | | **FATTY ACIDS** | |
| | 043 | Total fatty acids | mmol/l |
| | 044 | Degree of unsaturation | degree |
| | 045 | Omega-3 fatty acids | mmol/l |
| | 046 | Omega-6 fatty acids | mmol/l |
| | 047 | Polyunsaturated fatty acids | mmol/l |
| | 048 | Monosaturated fatty acids | mmol/l |
| | 049 | Saturated fatty acids | mmol/l |

(continued)

| | | FATTY ACIDS | |
|---|---|---|---|
| | 050 | Linoleic acid | mmol/l |
| | 051 | Docosahexaenoic acid | mmol/l |
| | | | |
| | | FATTY ACID RATIOS | |
| | 052 | Ratio of omega-3 fatty acids to total fatty acids | ratio (%) |
| | 053 | Ratio of omega-6 fatty acids to total fatty acids | ratio (%) |
| | 054 | Ratio of polyunsaturated fatty acids to total fatty acids | ratio (%) |
| | 055 | Ratio of monounsaturated fatty acids to total fatty acids | ratio (%) |
| | 056 | Ratio of saturated fatty acids to total fatty acids | ratio (%) |
| | 057 | Ratio of linoleic acid to total fatty acids | ratio (%) |
| | 058 | Ratio of docosahexaenoic acid to total fatty acids | ratio (%) |
| | 059 | Ratio of polyunsaturated fatty acids to monosaturated fatty acids | ratio (%) |
| | 060 | Ratio of omega-6 fatty acids to omega-3 fatty acids | ratio (%) |
| | | | |
| | | AMINO ACIDS | |
| | 061 | Alanine | mmol/l |
| | 062 | Glutamine | mmol/l |
| | 063 | Glycine | mmol/l |
| | 064 | Histidine | mmol/l |
| | | | |
| | | BRANCHED-CHAIN AMINO ACIDS | |
| | 065 | Total concentration of branched-chain amino acids | mmol/l |
| | 066 | Isoleucine | mmol/l |
| | 067 | Leucine | mmol/l |
| | 068 | Valine | mmol/l |
| | | | |
| | | AROMATIC AMINO ACIDS | |
| | 069 | Phenylalanine | mmol/l |
| | 070 | Tyrosine | mmol/l |
| | | | |
| | | GLYCOLYSIS-RELATED METABOLITES | |
| | 071 | Glucose | mmol/l |
| | 072 | Lactate | mmol/l |
| | 073 | Pyruvate | mmol/l |
| | 074 | Citrate | mmol/l |
| | 075 | Glycerol | mmol/l |
| | | | |

(continued)

| | | | |
|---|---|---|---|
| | | **KETONE BODIES** | |
| | 076 | 3-Hydroxybutyrate | mmol/l |
| | 077 | Acetate | mmol/l |
| | 078 | Acetoacetate | mmol/l |
| | 079 | Acetone | mmol/l |
| | | | |
| | | **FLUID BALANCE** | |
| | 080 | Creatinine | mmol/l |
| | 081 | Albumin | g/l |
| | | | |
| | | INFLAMMATION | |
| | 082 | Glycoprotein acetyls | mmol/l |
| | | | |
| | | **LIPOPROTEIN SUBCLASSES** | |
| | | **Chylomicrons and extremely large VLDL (diameter 75 nm upwards)** | |
| | 083 | Concentrations of chylomicron and extremely large VLDL particles | mmol/l |
| | 084 | Total lipids in chylomicron and extremely large VLDL | mmol/l |
| | 085 | Phospholipids in chylomicrons and extremely large VLDL | mmol/l |
| | 086 | Cholesterol in chylomicrons and extremely large VLDL | mmol/l |
| | 087 | Cholesteryl esters in chylomicrons and extremely large VLDL | mmol/l |
| | 088 | Free cholesterol in chylomicrons and extremely large VLDL | mmol/l |
| | 089 | Triglycerides in chylomicrons and extremely large VLDL | mmol/l |
| | | | |
| | | **Very large VLDL (average diameter 64 nm)** | |
| | 090 | Concentration of very large VLDL particles | mmol/l |
| | 091 | Total lipids in very large VLDL | mmol/l |
| | 092 | Phospholipids in very large VLDL | mmol/l |
| | 093 | Cholesterol in very large VLDL | mmol/l |
| | 094 | Cholesteryl esters in very large VLDL | mmol/l |
| | 095 | Free cholesterol in very large VLDL | mmol/l |
| | 096 | Triglycerides in very large VLDL | mmol/l |
| | | **Large VLDL (average diameter 53.6 nm)** | |
| | 097 | Concentration of large VLDL particles | mmol/l |
| | 098 | Total lipids in large VLDL | mmol/l |
| | 099 | Phospholipids in large VLDL | mmol/l |
| | 100 | Cholesterol in large VLDL | mmol/l |
| | 101 | Cholesteryl esters in large VLDL | mmol/l |
| | 102 | Free cholesterol in large VLDL | mmol/l |
| | 103 | Triglycerides in large VLDL | mmol/l |

(continued)

|  | Large VLDL (average diameter 53.6 nm) | |
|---|---|---|
|  |  | |
|  | Medium VLDL (average diameter 44.5 nm) | |
| 104 | Concentration of medium VLDL particles | mmol/l |
| 105 | Total lipids in medium VLDL | mmol/l |
| 106 | Phospholipids in medium VLDL | mmol/l |
| 107 | Cholesterol in medium VLDL | mmol/l |
| 108 | Cholesteryl esters in medium VLDL | mmol/l |
| 109 | Free cholesterol in medium VLDL | mmol/l |
| 110 | Triglycerides in medium VLDL | mmol/l |
|  |  | |
|  | Small VLDL (average diameter 36.8 nm) | |
| 111 | Concentration of small VLDL particles | mmol/l |
| 112 | Total lipids in small VLDL | mmol/l |
| 113 | Phospholipids in small VLDL | mmol/l |
| 114 | Cholesterol in small VLDL | mmol/l |
| 115 | Cholesteryl esters in small VLDL | mmol/l |
| 116 | Free cholesterol in small VLDL | mmol/l |
| 117 | Triglycerides in small VLDL | mmol/l |
|  |  | |
|  | Very small VLDL (average diameter 31.3 nm) | |
| 118 | Concentration of small VLDL particles | mmol/l |
| 119 | Total lipids in small VLDL | mmol/l |
| 120 | Phospholipids in small VLDL | mmol/l |
| 121 | Cholesterol in small VLDL | mmol/l |
| 122 | Cholesteryl esters in small VLDL | mmol/l |
| 123 | Free cholesterol in small VLDL | mmol/l |
| 124 | Triglycerides in small VLDL | mmol/l |
|  |  | |
|  | IDL (average diameter 28.6 nm) | |
| 125 | Concentration of IDL particles | mmol/l |
| 126 | Total lipids in IDL | mmol/l |
| 127 | Phospholipids in IDL | mmol/l |
| 128 | Cholesterol in IDL | mmol/l |
| 129 | Cholesteryl esters in IDL | mmol/l |
| 130 | Free cholesterol in IDL | mmol/l |
| 131 | Triglycerides in IDL | mmol/l |
|  |  | |

(continued)

| | Large LDL (average diameter 25.5 nm) | |
|---|---|---|
| 132 | Concentration of large LDL particles | mmol/l |
| 133 | Total lipids in large LDL | mmol/l |
| 134 | Phospholipids in large LDL | mmol/l |
| 135 | Cholesterol in large LDL | mmol/l |
| 136 | Cholesteryl esters in large LDL | mmol/l |
| 137 | Free cholesterol in large LDL | mmol/l |
| 138 | Triglycerides in large LDL | mmol/l |
| | | |
| | Medium LDL (average diameter 23 nm) | |
| 139 | Concentration of medium LDL particles | mmol/l |
| 140 | Total lipids in medium LDL | mmol/l |
| 141 | Phospholipids in medium LDL | mmol/l |
| 142 | Cholesterol in medium LDL | mmol/l |
| 143 | Cholesteryl esters in medium LDL | mmol/l |
| 144 | Free cholesterol in medium LDL | mmol/l |
| 145 | Triglycerides in medium LDL | mmol/l |
| | | |
| | Small LDL (average diameter 18.7 nm) | |
| 146 | Concentration of small LDL particles | mmol/l |
| 147 | Total lipids in small LDL | mmol/l |
| 148 | Phospholipids in small LDL | mmol/l |
| 149 | Cholesterol in small LDL | mmol/l |
| 150 | Cholesteryl esters in small LDL | mmol/l |
| 151 | Free cholesterol in small LDL | mmol/l |
| 152 | Triglycerides in small LDL | mmol/l |
| | | |
| | Very large HDL (average diameter 14.3 nm) | |
| 153 | Concentration of very large HDL particles | mmol/l |
| 154 | Total lipids in very large HDL | mmol/l |
| 155 | Phospholipids in very large HDL | mmol/l |
| 156 | Cholesterol in very large HDL | mmol/l |
| 157 | Cholesteryl esters in very large HDL | mmol/l |
| 158 | Free cholesterol in very large HDL | mmol/l |
| 159 | Triglycerides in very large HDL | mmol/l |
| | | |
| | Large HDL (average diameter 12.1 nm) | |
| 160 | Concentration of large HDL particles | mmol/l |
| 161 | Total lipids in large HDL | mmol/l |

(continued)

| | | Large HDL (average diameter 12.1 nm) | |
|---|---|---|---|
| | 162 | Phospholipids in large HDL | mmol/l |
| | 163 | Cholesterol in large HDL | mmol/l |
| | 164 | Cholesteryl esters in large HDL | mmol/l |
| | 165 | Free cholesterol in large HDL | mmol/l |
| | 166 | Triglycerides in large HDL | mmol/l |
| | | | |
| | | Medium HDL (average diameter 10.9 nm) | |
| | 167 | Concentration of large HDL particles | mmol/l |
| | 168 | Total lipids in large HDL | mmol/l |
| | 169 | Phospholipids in large HDL | mmol/l |
| | 170 | Cholesterol in large HDL | mmol/l |
| | 171 | Cholesteryl esters in large HDL | mmol/l |
| | 172 | Free cholesterol in large HDL | mmol/l |
| | 173 | Triglycerides in large HDL | mmol/l |
| | | | |
| | | Small HDL (average diameter 8.7 nm) | |
| | 174 | Concentration of small HDL particles | mmol/l |
| | 175 | Total lipids in small HDL | mmol/l |
| | 176 | Phospholipids in small HDL | mmol/l |
| | 177 | Cholesterol in small HDL | mmol/l |
| | 178 | Cholesteryl esters in small HDL | mmol/l |
| | 179 | Free cholesterol in small HDL | mmol/l |
| | 180 | Triglycerides in small HDL | mmol/l |
| | | | |
| | | RELATIVE LIPOPROTEIN LIPID CONCENTRATIONS | |
| | | Chvlomicrons and extremely large VLDL ratios | |
| | 181 | Phospholipids to total lipids ratio in chylomicrons and extremely large VLDL | ratio (%) |
| | 182 | Cholesterol to total lipids ratio in chylomicrons and extremely large VLDL | ratio (%) |
| | 183 | Cholesteryl esters to total lipids ratio in chylomicrons and extremely large VLDL | ratio (%) |
| | 184 | Free cholesterol to total lipids ratio in chylomicrons and extremely large VLDL | ratio (%) |
| | 185 | Triglycerides to total lipids ratio in chylomicrons and extremely large VLDL | ratio (%) |
| | | | |
| | | Very large VLDL ratios | |
| | 186 | Phospholipids to total lipids ratio in very large VLDL | ratio (%) |
| | 187 | Cholesterol to total lipids ratio in very large VLDL | ratio (%) |
| | 188 | Cholesteryl esters to total lipids ratio in very large VLDL | ratio (%) |
| | 189 | Free cholesterol to total lipids ratio in very large VLDL | ratio (%) |
| | 190 | Triglycerides to total lipids ratio in very large VLDL | ratio (%) |

(continued)

| | | Very large VLDL ratios | |
|---|---|---|---|
| | | | |
| | | **Large VLDL ratios** | |
| | 191 | Phospholipids to total lipids ratio in large VLDL | ratio (%) |
| | 192 | Cholesterol to total lipids ratio in large VLDL | ratio (%) |
| | 193 | Cholesteryl esters to total lipids ratio in large VLDL | ratio (%) |
| | 194 | Free cholesterol to total lipids ratio in large VLDL | ratio (%) |
| | 195 | Triglycerides to total lipids ratio in large VLDL | ratio (%) |
| | | | |
| | | **Medium VLDL ratios** | |
| | 196 | Phospholipids to total lipids ratio in medium VLDL | ratio (%) |
| | 197 | Cholesterol to total lipids ratio in medium VLDL | ratio (%) |
| | 198 | Cholesteryl esters to total lipids ratio in medium VLDL | ratio (%) |
| | 199 | Free cholesterol to total lipids ratio in medium VLDL | ratio (%) |
| | 200 | Triglycerides to total lipids ratio in medium VLDL | ratio (%) |
| | | | |
| | | **Small VLDL ratios** | |
| | 201 | Phospholipids to total lipids ratio in small VLDL | ratio (%) |
| | 202 | Cholesterol to total lipids ratio in small VLDL | ratio (%) |
| | 203 | Cholesteryl esters to total lipids ratio in small VLDL | ratio (%) |
| | 204 | Free cholesterol to total lipids ratio in small VLDL | ratio (%) |
| | 205 | Triglycerides to total lipids ratio in small VLDL | ratio (%) |
| | | | |
| | | **Very small VLDL ratios** | |
| | 206 | Phospholipids to total lipids ratio in very small VLDL | ratio (%) |
| | 207 | Cholesterol to total lipids ratio in very small VLDL | ratio (%) |
| | 208 | Cholesteryl esters to total lipids ratio in very small VLDL | ratio (%) |
| | 209 | Free cholesterol to total lipids ratio in very small VLDL | ratio (%) |
| | 210 | Triglycerides to total lipids ratio in very small VLDL | ratio (%) |
| | | | |
| | | **IDL ratios** | |
| | 211 | Phospholipids to total lipids ratio in IDL | ratio (%) |
| | 212 | Cholesterol to total lipids ratio in IDL | ratio (%) |
| | 213 | Cholesteryl esters to total lipids ratio in IDL | ratio (%) |
| | 214 | Free cholesterol to total lipids ratio in IDL | ratio (%) |
| | 215 | Triglycerides to total lipids ratio in IDL | ratio (%) |
| | | | |
| | | **Large LDL ratios** | |
| | 216 | Phospholipids to total lipids ratio in large LDL | ratio (%) |

(continued)

| | | Large LDL ratios | |
|---|---|---|---|
| | 217 | Cholesterol to total lipids ratio in large LDL | ratio (%) |
| | 218 | Cholesteryl esters to total lipids ratio in large LDL | ratio (%) |
| | 219 | Free cholesterol to total lipids ratio in large LDL | ratio (%) |
| | 220 | Triglycerides to total lipids ratio in large LDL | ratio (%) |
| | | | |
| | | Medium LDL ratios | |
| | 221 | Phospholipids to total lipids ratio in medium VLDL | ratio (%) |
| | 222 | Cholesterol to total lipids ratio in medium VLDL | ratio (%) |
| | 223 | Cholesteryl esters to total lipids ratio in medium VLDL | ratio (%) |
| | 224 | Free cholesterol to total lipids ratio in medium VLDL | ratio (%) |
| | 225 | Triglycerides to total lipids ratio in medium VLDL | ratio (%) |
| | | | |
| | | Small LDL ratios | |
| | 226 | Phospholipids to total lipids ratio in small LDL | ratio (%) |
| | 227 | Cholesterol to total lipids ratio in small LDL | ratio (%) |
| | 228 | Cholesteryl esters to total lipids ratio in small LDL | ratio (%) |
| | 229 | Free cholesterol to total lipids ratio in small LDL | ratio (%) |
| | 230 | Triglycerides to total lipids ratio in small LDL | ratio (%) |
| | | | |
| | | Very large HDL ratios | |
| | 231 | Phospholipids to total lipids ratio in very large HDL | ratio (%) |
| | 232 | Cholesterol to total lipids ratio in very large HDL | ratio (%) |
| | 233 | Cholesteryl esters to total lipids ratio in very large HDL | ratio (%) |
| | 234 | Free cholesterol to total lipids ratio in very large HDL | ratio (%) |
| | 235 | Triglycerides to total lipids ratio in very large HDL | ratio (%) |
| | | | |
| | | Large HDL ratios | |
| | 286 | Phospholipids to total lipids ratio in large HDL | ratio (%) |
| | 237 | Cholesterol to total lipids ratio in large HDL | ratio (%) |
| | 238 | Cholesteryl esters to total lipids ratio in large HDL | ratio (%) |
| | 239 | Free cholesterol to total lipids ratio in large HDL | ratio (%) |
| | 240 | Triglycerides to total lipids ratio in large HDL | ratio (%) |
| | | | |
| | | Medium HDL ratios | |
| | 241 | Phospholipids to total lipids ratio in medium HDL | ratio (%) |
| | 242 | Cholesterol to total lipids ratio in medium HDL | ratio (%) |
| | 243 | Cholesteryl esters to total lipids ratio in medium HDL | ratio (%) |
| | 244 | Free cholesterol to total lipids ratio in medium HDL | ratio (%) |

(continued)

| | | Medium HDL ratios | |
|---|---|---|---|
| | 245 | Triglycerides to total lipids ratio in medium HDL | ratio (%) |
| | | | |
| | | Small HDL ratios | |
| | 246 | Phospholipids to total lipids ratio in small HDL | ratio (%) |
| | 247 | Cholesterol to total lipids ratio in small HDL | ratio (%) |
| | 248 | Cholesteryl esters to total lipids ratio in small HDL | ratio (%) |
| | 249 | Free cholesterol to total lipids ratio in small HDL | ratio (%) |
| | 250 | Triglycerides to total lipids ratio in small HDL | ratio (%) |

NUMBERED EMBODIMENTS

1. A method of determining frailty severity in a subject comprising the steps of

a) measuring the levels of GDF-15 in a biological sample from the subject;

b) determining the subject as being frail if the level of the biomarker in the biological sample is higher than about 2,000 pg/ml to 6,000 pg/ml; pre-FRAIL if the level of the biomarker in the biological sample is higher than 500 pg/ml to 2000 pg/ml; and robust if the level of the biomarker in the biological sample is less than 500 pg/ml.

2. A method of determining frailty severity in a subject comprising the steps of

a) measuring the levels of GDF-15 in a biological sample from the subject;
b) measuring the levels of one or more biomarkers selected from the group consisting of albumin, glutamine, GlycA, phosphoglycerides, glycine, and alanine;
c) determining the overall biosignature score $p$ by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = \exp(-9.31 \pm 0.817 \times \text{sex} + (0.111 \times \text{age}) + (0.000121 \times \text{GDF-15}) + (0.355 \times \text{Gln}) + (0.571 \times \text{albumin}) + (0.526 \times \text{GlycA}) + (-0.256 \times \text{phosphoglycerides}) + (0.266 \times \text{Gly}) + (-0.0577 \times \text{Ala});$$

and
d) determining the subject as being frail if the score $p$ is a value defined by a threshold value in Table 1 wherein the corresponding sensitivity and specificity values of the threshold value add up to between 1.4 and 1.6; and wherein at least one of the sensitivity or specificity values is 0.5 or above.

3. The method of embodiment 2, wherein the score $p$ is 0.15 to 0.56.

4. The method of embodiment 3, wherein the score $p$ is 0.259 or a threshold value with the maximum value of Youden's J statistic according to Table 1.

5. The method of embodiment 4, wherein the maximum value of Youden's J statistic is 0.553.

6. The method of any one of embodiments 1-4, wherein if the subject is determined to be frail, treating the subject with exercise therapy, physical therapy, physiotherapy, nutritional supplementation (amino acid(s)/leucine (in non-cardiac failure)/protein), or administering a therapeutic drug for treating impaired cardiovascular or cardiopulmonary function.

7. The method of embodiment 5, wherein the therapeutic drug is sacubitril/valsartan, dapagliflozin, empagliflozin, beta blocker (e.g. metoprolol, carvedilol, bisoprolol), renin-angiotensin system inhibitor (e.g. enalapril, lisinopril), mineralocorticoid receptor antagonist (e.g. eplerenone, spironolactone), ivabradine, digoxin, inotropes (e.g. dobutamine, milrinone) or inodilator (e.g. levosimendan).

8. The method of any one of embodiments 1-7, wherein the method is an in vitro method.

9. A method of determining frailty severity in a subject comprising the steps of

a) measuring the levels of GDF-15 in a biological sample from the subject; and
b) measuring the levels of NT-proBNP,
wherein if NT-proBNP levels are elevated but GDF-15 levels are not, determining the subject has cardiac dysfunction without frailty; if GDF-15 levels are elevated but NT-proBNP are not, determining the subject has systemic physiological injury, hypoperfusion, diabetes mellitus, inflammatory disorders, or non-cardiac frailty; if both GDF-15 and NT-proBNP levels are elevated, determining the subject has frailty and is predicted to have heart failure and if neither NT-proBNP levels nor GDF-15 levels are elevated, determining the subject is at low risk for frailty and low risk for heart failure.

10. The method of embodiment 9, further comprising the step of treating the subject with guideline-directed medical therapy (GDMT); wherein if the subject has elevated levels of both NT-proBNP and GDF-15, treating the subject as advanced stage D in accordance with GDMT; if the subject has elevated levels of NT-proBNP but not elevated levels of GDF-15, conducting cardiac imaging to determine the causes of cardiac dysfunction and treating the cardiac dysfunction in accordance with stage B or C in accordance with GDMT; if the subject has elevated levels of GDF-15 but not elevated levels of NT-proBNP, conducting a clinical assessment of medical comorbidities and treating the subject in accordance with stage B or C in accordance with GDMT; and if the subject does not have elevated levels of either NT-proBNP or GDF-15, treating the patient with as stage A in accordance with GDMT.

11. A method of identifying and treating frailty, altered physiological and physical reserve, aging, or aging-related inflammation in a subject comprising the steps of

a) measuring the levels of GDF-15 in a biological sample from the subject;
b) measuring the levels of one or more biomarkers selected from the group consisting of albumin, glutamine, GlycA, phosphoglycerides, glycine, and alanine;
c) determining the overall biosignature score $p$ by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp{(H)}}{1 + \exp{(H)}}$$

wherein

$$H = \exp(-9.31 \pm 0.817 \times \text{sex} + (0.111 \times \text{age}) + (0.000121 \times \text{GDF-15}) + (0.355 \times \text{Gln}) +$$
$$(0.571 \times \text{albumin}) + (0.526 \times \text{GlycA}) + (-0.256 \times \text{phosphoglycerides}) + (0.266 \times \text{Gly}) +$$
$$(-0.0577 \times \text{Ala});$$

and
d) determining the subject as being frail if the score *p* is 0.15 to 0.56.

12. A system for detecting frailty in a subject comprising:

a) a GDF-15 analyzer configured to analyze biological samples from the subject to provide a concentration of GDF-15 in the biological sample;
b) a computer programmed to execute the following steps:

i) determining the overall biosignature score *p* by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = \exp(-9.31 \pm 0.817 \times \text{sex} + (0.111 \times \text{age}) + (0.000121 \times \text{GDF-15}) +$$
$$(0.355 \times \text{Gln}) + (0.571 \times \text{albumin}) + (0.526 \times \text{GlycA}) + (-0.256 \times$$
$$\text{phosphoglycerides}) + (0.266 \times \text{Gly}) + (-0.0577 \times \text{Ala});$$

and
ii) determining the subject as being frail if the score *p* is 0.15 to 0.56.

13. A method of improving the accuracy of frailty and non-frailty classification comprising using GDF-15 as a guiding biomarker with a metabolomic panel of metabolites selected from one or more of albumin, glutamine, GlycA, phosphoglycerides, glycine, and alanine; comprising the following steps:

a) measuring GDF-15 concentration in a blood serum or plasma sample from a subject using the Roche Elecsys Assay kit on a Roche Cobas e immunoassay analyzer;
b) measuring the levels of one or more biomarkers selected from the group consisting of albumin, glutamine, GlycA, phosphoglycerides, glycine, and alanine using $^1$H-NMR Nightingale metabolomic profiling;
c) determining the overall biosignature score *p* by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = \exp(-9.31 \pm 0.817 \times \text{sex} + (0.111 \times \text{age}) + (0.000121 \times \text{GDF-15}) + (0.355 \times \text{Gln}) +$$
$$(0.571 \times \text{albumin}) + (0.526 \times \text{GlycA}) + (-0.256 \times \text{phosphoglycerides}) + (0.266 \times \text{Gly}) +$$
$$(-0.0577 \times \text{Ala});$$

and
d) determining the subject as being frail if the score *p* is 0.15 to 0.56.

14. A method of identifying subjects who will have improved survival outcomes when treated with exercise therapy, physical therapy, physiotherapy, nutritional supplementation (amino acid(s)/leucine (in non-cardiac failure)/protein), or administering a therapeutic drug for treating impaired cardiovascular or cardiopulmonary function; comprising the steps of

a) determining the overall biosignature score *p* by inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp(H)}{1 + \exp(H)}$$

wherein

$$H = \exp(-9.31 \pm 0.817 \times \text{sex} + (0.111 \times \text{age}) + (0.000121 \times \text{GDF-15}) + (0.355 \times \text{Gln})$$
$$+ (0.571 \times \text{albumin}) + (0.526 \times \text{GlycA}) + (-0.256 \times \text{phosphoglycerides})$$
$$+ (0.266 \times \text{Gly}) + (-0.0577 \times \text{Ala});$$

b) determining the subject as being frail if the score *p* is 0.15 to 0.56; and
c) treating the subjects determined as being frail with exercise therapy, physical therapy, physiotherapy, nutritional supplementation (amino acid(s)/leucine (in non-cardiac failure)/protein), or administering a therapeutic drug for treating impaired cardiovascular or cardiopulmonary function.

15. A kit for evaluating frailty comprising

a) a test for measuring blood levels of GDF-15; and
b) optionally one or more tests for measuring blood levels of albumin, glutamine, GlycA, phosphoglycerides, glycine, and alanine.

16. The kit of embodiment 9 wherein the test for measuring albumin, glutamine, GlycA, and phosphoglyceride is the $^1$H-NMR Nightingale system.

The exemplary embodiments of the present invention are thus fully described. Although the description referred to particular embodiments, it will be clear to one skilled in the art that the present invention may be practiced with variation of these specific details. Hence this invention should not be construed as limited to the embodiments set forth herein.

**Claims**

1. An in vitro method of determining frailty severity in a subject comprising the steps of

a) measuring the levels of GDF-15 in a biological sample from the subject; and
b) determining the subject as being frail if the level of the biomarker in the biological sample is higher than about 2,000 pg/ml to 6,000 pg/ml; pre-frail if the level of the biomarker in the biological sample is higher than 500 pg/ml to 2000 pg/ml; and robust if the level of the biomarker in the biological sample is less than 500 pg/ml.

2. An in vitro method of determining frailty severity in a subject comprising the steps of:

  a) measuring the levels of GDF-15 in a biological sample from the subject;
  b) measuring the levels of one or more biomarkers selected from the group consisting of albumin, glutamine, GlycA, phosphoglycerides, glycine, and alanine;
  c) determining the overall biosignature score $p$; and
  d) determining the subject as being frail if $p$ is Z;

    wherein Z is a value defined in Table 1 wherein the corresponding sensitivity and specificity values add up to between 1.4 and 1.5;
    wherein the score $p$ is determined by

      i) inputting the subject's age in years; sex as value of 0 if female, 1 if male; and serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp{(H)}}{1 + \exp{(H)}}$$

      wherein

$$H = (-9.31 \pm 0.817 \times \text{sex} + (0.111 \times \text{age}) + (0.000121 \times \text{GDF-15})$$
$$+ (0.355 \times \text{Gln}) + (0.571 \times \text{albumin}) + (0.526 \times \text{GlycA})$$
$$+ (-0.256 \times \text{phosphoglycerides}) + (0.266 \times \text{Gly}) + (-0.0577 \times \text{Ala});$$

      or
      ii) inputting the subject's age in years; sex as value of 0 if female, 1 if male; and $\log_{10}$-transformed serum concentrations of biomarkers GDF-15 (pg/ml), albumin (g/1), glutamine (mmol/l), GlycA (mmol/l), phosphoglycerides (mmol/l), glycine (mmol/l), and alanine (mmol/l) of the subject into the following equation:

$$p = \frac{\exp{(H)}}{1 + \exp{(H)}}$$

      wherein

$$H = -25.54 + (-0.95 \times \text{sex}) + (0.10 \times \text{age}) + (1.20 \times \text{GDF-15}) +$$
$$(7.26 \times \text{Gln}) + (10.0 \times \text{albumin}) + (6.30 \times \text{GlycA}) + (-3.11 \times$$
$$\text{phosphoglycerides}) + (3.22 \times \text{Gly}) + (-0.97 \times \text{Ala}).$$

3. The method of claim 2, wherein Z is 0.15 to 0.56.

4. The method of claim 3, wherein Z is 0.259 or a threshold value with the maximum value of Youden's J statistic according to Table 1.

5. The method of claim 4, wherein the maximum value of Youden's *J* statistic is 0.553.

6. The method of claim 2, wherein the biological sample is a blood serum or plasma sample from a subject; and optionally is measured using the ROCHE Elecsys Assay kit on a ROCHE Cobas e immunoassay analyzer; and optionally the levels of one or more biomarkers selected from the group consisting of albumin, glutamine, GlycA, phosphoglycerides, glycine, and alanine are measured using $^1$H-NMR Nightingale metabolomic profiling.

7. An in vitro method of determining frailty severity in a subject comprising the steps of

a) measuring the levels of GDF-15 in a biological sample from the subject;
b) measuring the levels of NT-proBNP,
wherein if NT-proBNP levels are elevated but GDF-15 levels are not, determining the subject has cardiac dysfunction without frailty; if GDF-15 levels are elevated but NT-proBNP are not, determining the subject has systemic physiological injury, hypoperfusion, diabetes mellitus, inflammatory disorders, or non-cardiac frailty; if both GDF-15 and NT-proBNP levels are elevated, determining the subject has frailty and is predicted to have heart failure and if neither NT-proBNP levels nor GDF-15 levels are elevated, determining the subject is at low risk for frailty and low risk for heart failure.

8. A system for detecting frailty in a subject comprising:

a) a GDF-15 analyzer configured to analyze biological samples from the subject to provide a concentration of GDF-15 in the biological sample;
b) a computer programmed to execute at least the steps i) or step ii) of claim 2.

9. A kit for evaluating frailty comprising

a) a test for measuring blood levels of GDF-15; and
b) optionally one or more tests for measuring blood levels of albumin, glutamine, GlycA, phosphoglycerides, glycine, and alanine; and
optionally wherein the test for measuring albumin, glutamine, GlycA, and phosphoglyceride is the $^1$H-NMR Nightingale system.

FIG.1

**Metabolic biosignature of NT-proBNP according to CD status**

Adjusted for age and sex

Non-CD group (n=240)

CD group (n=66)

| group | name |
|---|---|
| Cholesterol | Total-C |
| | non-HDL-C |
| | Remnant-C |
| | VLDL-C |
| | Clinical LDL-C |
| | LDL-C |
| | HDL-C |
| Triglycerides | Total-TG |
| | VLDL-TG |
| | LDL-TG |
| | HDL-TG |
| Phospholipids | Total-PL |
| | VLDL-PL |
| | LDL-PL |
| | HDL-PL |
| Cholesteryl esters | Total-CE |
| | VLDL-CE |
| | LDL-CE |
| | HDL-CE |
| Free cholesterol | Total-FC |
| | VLDL-FC |
| | LDL-FC |
| | HDL-FC |
| Total lipids | Total-L |
| | VLDL-L |
| | LDL-L |
| | HDL-L |

Beta estimate (95% CI)

Non-significant         Significant

FIG.2A

Metabolic biosignature of NT-proBNP according to CD status

CD group (n=66)

Non-CD group (n=240)

Beta estimate (95% CI)

Beta estimate (95% CI)

Significant

Non-significant

FIG.2B

Adjusted for age and sex

| group | name |
|---|---|
| Lipoprotein particle concentrations | Total-P |
| | VLDL-P |
| | LDL-P |
| | HDL-P |
| Lipoprotein particle sizes | VLDL size |
| | LDL size |
| | HDL size |
| Other lipids | Phosphoglyc |
| | TG/PG |
| | Cholines |
| | Phosphatidylc |
| | Sphingomyelins |
| Apolipoproteins | ApoB |
| | ApoA1 |
| | ApoB/ApoA1 |
| Fatty acids | Total-FA |
| | Unsaturation |
| | Omega-3 |
| | Omega-6 |
| | PUFA |
| | MUFA |
| | SFA |
| | LA |
| | DHA |

FIG.2C

**Metabolic biosignature of NT-proBNP according to CD status**

FIG.2D

**Metabolic biosignature of NT-proBNP according to CD status**

FIG.2E

**FIG.2F**

FIG.2G

Metabolic biosignature of NT-proBNP according to CD status

CD group (n=66)

Non-CD group (n=240)

Beta estimate (95% CI)

Adjusted for age and sex

| group | name |
|---|---|
| Chylomicrons and extremely large VLDL ratios | XXL-VLDL-PL % |
| | XXL-VLDL-C % |
| | XXL-VLDL-CE % |
| | XXL-VLDL-FC % |
| | XXL-VLDL-TG % |
| Very large VLDL ratios | XL-VLDL-PL % |
| | XL-VLDL-C % |
| | XL-VLDL-CE % |
| | XL-VLDL-FC % |
| | XL-VLDL-TG % |
| Large VLDL ratios | L-VLDL-PL % |
| | L-VLDL-C % |
| | L-VLDL-CE % |
| | L-VLDL-FC % |
| | L-VLDL-TG % |
| Medium VLDL ratios | M-VLDL-PL % |
| | M-VLDL-C % |
| | M-VLDL-CE % |
| | M-VLDL-FC % |
| | M-VLDL-TG % |
| Small VLDL ratios | S-VLDL-PL % |
| | S-VLDL-C % |
| | S-VLDL-CE % |
| | S-VLDL-FC % |
| | S-VLDL-TG % |

Significant

Non-significant

FIG.2H

46

Metabolic biosignature of NT-proBNP according to CD status

FIG.2I

**Metabolic biosignature of NT-proBNP according to CD status**

Adjusted for age and sex

FIG.2J

Metabolic profiles of paired comparisons among frailty status

FIG.3A

EP 3 961 219 A2

**Metabolic profiles of paired comparisons among frailty status**

FIG.3B

**Metabolic profiles of paired comparisons among frailty status**

FIG.3C

**Metabolic profiles of paired comparisons among frailty status**

FIG.3D

EP 3 961 219 A2

Metabolic profiles of paired comparisons among frailty status

FIG.3E

Metabolic profiles of paired comparisons among frailty status

Robust vs Frail    Pre-frail vs Robust    Frail vs Pre-frail

Adjusted for age and sex

| group | name |
|---|---|
| Large LDL | L-LDL-P |
| | L-LDL-L |
| | L-LDL-PL |
| | L-LDL-C |
| | L-LDL-CE |
| | L-LDL-FC |
| | L-LDL-TG |
| Medium LDL | M-LDL-P |
| | M-LDL-L |
| | M-LDL-PL |
| | M-LDL-C |
| | M-LDL-CE |
| | M-LDL-FC |
| | M-LDL-TG |
| Small LDL | S-LDL-P |
| | S-LDL-L |
| | S-LDL-PL |
| | S-LDL-C |
| | S-LDL-CE |
| | S-LDL-FC |
| | S-LDL-TG |

Non-significant    Significant

FIG.3F

Metabolic profiles of paired comparisons among frailty status

FIG.3G

FIG.3H

Metabolic profiles of paired comparisons among frailty status

FIG.3I

Metabolic profiles of paired comparisons among frailty status

FIG.3J

**Metabolic profile of GDF-15 shifted with frailty status**

Adjusted for age and sex

| group | name |
|---|---|
| Cholesterol | Total-C |
| | non-HDL-C |
| | Remnant-C |
| | VLDL-C |
| | Clinical LDL-C |
| | LDL-C |
| | HDL-C |
| Triglycerides | Total-TG |
| | VLDL-TG |
| | LDL-TG |
| | HDL-TG |
| Phospholipids | Total-PL |
| | VLDL-PL |
| | LDL-PL |
| | HDL-PL |
| Cholesteryl esters | Total-CE |
| | VLDL-CE |
| | LDL-CE |
| | HDL-CE |
| Free cholesterol | Total-FC |
| | VLDL-FC |
| | LDL-FC |
| | HDL-FC |
| Total lipids | Total-L |
| | VLDL-L |
| | LDL-L |
| | HDL-L |

Robust (n=104) — Beta estimate (95% CI)

Pre-frail (n=107) — Beta estimate (95% CI)

Frail (n=95) — Beta estimate (95% CI)

Non-significant ■    Significant □

FIG.4A(i)

EP 3 961 219 A2

Metabolic profile of GDF-15 shifted with frailty status

FIG.4A(ii)

Metabolic profile of GDF-15 shifted with frailty status

FIG.4A(iii)

Metabolic profile of GDF-15 shifted with frailty status

FIG.4A(iv)

Metabolic profile of GDF-15 shifted with frailty status

FIG.4A(v)

Metabolic profile of GDF-15 shifted with frailty status

FIG.4A(vi)

**Metabolic profile of GDF-15 shifted with frailty status**

Adjusted for age and sex

| group | name |
|---|---|
| Very large HDL | XL-HDL-P |
| | XL-HDL-L |
| | XL-HDL-PL |
| | XL-HDL-C |
| | XL-HDL-CE |
| | XL-HDL-FC |
| | XL-HDL-TG |
| Large HDL | L-HDL-P |
| | L-HDL-L |
| | L-HDL-PL |
| | L-HDL-C |
| | L-HDL-CE |
| | L-HDL-FC |
| | L-HDL-TG |
| Medium HDL | M-HDL-P |
| | M-HDL-L |
| | M-HDL-PL |
| | M-HDL-C |
| | M-HDL-CE |
| | M-HDL-FC |
| | M-HDL-TG |
| Small HDL | S-HDL-P |
| | S-HDL-L |
| | S-HDL-PL |
| | S-HDL-C |
| | S-HDL-CE |
| | S-HDL-FC |
| | S-HDL-TG |

Robust (n=104) — Beta estimate (95% CI)

Pre-frail (n=107) — Beta estimate (95% CI)

Frail (n=95) — Beta estimate (95% CI)

Non-significant   Significant

FIG.4A(vii)

Metabolic profile of GDF-15 shifted with frailty status

Adjusted for age and sex

FIG.4A(viii)

Metabolic profile of GDF-15 shifted with frailty status

FIG.4A(ix)

FIG.4A(x)

**Metabolic profile of GDF-15 shifted with frailty status**

**Adjusted for age and sex**

| group | name |
|---|---|
| Cholesterol | Total-C |
| | non-HDL-C |
| | Remnant-C |
| | VLDL-C |
| | Clinical LDL-C |
| | LDL-C |
| | HDL-C |
| Triglycerides | Total-TG |
| | VLDL-TG |
| | LDL-TG |
| | HDL-TG |
| Phospholipids | Total-PL |
| | VLDL-PL |
| | LDL-PL |
| | HDL-PL |
| Cholesteryl esters | Total-CE |
| | VLDL-CE |
| | LDL-CE |
| | HDL-CE |
| Free cholesterol | Total-FC |
| | VLDL-FC |
| | LDL-FC |
| | HDL-FC |
| Total lipids | Total-L |
| | VLDL-L |
| | LDL-L |
| | HDL-L |

Robust (n=334)  Pre-frail (n=183)  Frail (n=38)

Beta estimate (95% CI)   Beta estimate (95% CI)   Beta estimate (95% CI)

Non-significant    Significant

FIG.4B(i)

EP 3 961 219 A2

Metabolic profile of GDF-15 shifted with frailty status

FIG.4B(ii)

FIG.4B(iii)

**Metabolic profile of GDF-15 shifted with frailty status**

FIG.4B(iv)

EP 3 961 219 A2

Metabolic profile of GDF-15 shifted with frailty status

FIG.4B(v)

FIG.4B(vi)

Metabolic profile of GDF-15 shifted with frailty status

FIG.4B(vii)

Metabolic profile of GDF-15 shifted with frailty status

FIG.4B(viii)

Metabolic profile of GDF-15 shifted with frailty status

FIG.4B(ix)

Metabolic profile of GDF-15 shifted with frailty status

FIG.4B(x)

FIG.4C(i)

Metabolic profile of GDF-15 shifted with frailty status

Adjusted for age and sex

Frail (n=133)

Pre-frail (n=290)

Robust (n=438)

Beta estimate (95% CI)

| group | name |
|---|---|
| Lipoprotein particle concentrations | Total-P |
| | VLDL-P |
| | LDL-P |
| | HDL-P |
| Lipoprotein particle sizes | VLDL size |
| | LDL size |
| | HDL size |
| Other lipids | Phosphoglyc |
| | TG/PG |
| | Cholines |
| | Phosphatidylc |
| | Sphingomyelins |
| Apolipoproteins | ApoB |
| | ApoA1 |
| | ApoB/ApoA1 |
| Fatty acids | Total-FA |
| | Unsaturation |
| | Omega-3 |
| | Omega-6 |
| | PUFA |
| | MUFA |
| | SFA |
| | LA |
| | DHA |

Non-significant   Significant

FIG.4C(ii)

**Metabolic profile of GDF-15 shifted with frailty status**

FIG.4C(iii)

FIG.4C(iv)

# Metabolic profile of GDF-15 shifted with frailty status

**Adjusted for age and sex**

| group | name | |
|---|---|---|
| Medium VLDL | M-VLDL-P | 1 |
| | M-VLDL-L | 2 |
| | M-VLDL-PL | 3 |
| | M-VLDL-C | 4 |
| | M-VLDL-CE | 5 |
| | M-VLDL-FC | 6 |
| | M-VLDL-TG | 7 |
| Small VLDL | S-VLDL-P | 8 |
| | S-VLDL-L | 9 |
| | S-VLDL-PL | 10 |
| | S-VLDL-C | 11 |
| | S-VLDL-CE | 12 |
| | S-VLDL-FC | 13 |
| | S-VLDL-TG | 14 |
| Very small VLDL | XS-VLDL-P | 15 |
| | XS-VLDL-L | 16 |
| | XS-VLDL-PL | 17 |
| | XS-VLDL-C | 18 |
| | XS-VLDL-CE | 19 |
| | XS-VLDL-FC | 20 |
| | XS-VLDL-TG | 21 |
| IDL | IDL-P | 22 |
| | IDL-L | 23 |
| | IDL-PL | 24 |
| | IDL-C | 25 |
| | IDL-CE | 26 |
| | IDL-FC | 27 |
| | IDL-TG | 28 |

**Robust (n=438)** — Beta estimate (95% CI)

**Pre-frail (n=290)** — Beta estimate (95% CI)

**Frail (n=133)** — Beta estimate (95% CI)

Non-significant   Significant

FIG.4C(v)

EP 3 961 219 A2

**Metabolic profile of GDF-15 shifted with frailty status**

Adjusted for age and sex

| group | name |
|---|---|
| Large LDL | L–LDL–P |
| | L–LDL–L |
| | L–LDL–PL |
| | L–LDL–C |
| | L–LDL–CE |
| | L–LDL–FC |
| | L–LDL–TG |
| Medium LDL | M–LDL–P |
| | M–LDL–L |
| | M–LDL–PL |
| | M–LDL–C |
| | M–LDL–CE |
| | M–LDL–FC |
| | M–LDL–TG |
| Small LDL | S–LDL–P |
| | S–LDL–L |
| | S–LDL–PL |
| | S–LDL–C |
| | S–LDL–CE |
| | S–LDL–FC |
| | S–LDL–TG |

Robust (n=438)   Pre–frail (n=290)   Frail (n=133)

Beta estimate (95% CI)

Non-significant    Significant

FIG.4C(vi)

EP 3 961 219 A2

84

Metabolic profile of GDF-15 shifted with frailty status

Adjusted for age and sex

FIG.4C(vii)

Metabolic profile of GDF-15 shifted with frailty status

FIG.4C(viii)

## Metabolic profile of GDF-15 shifted with frailty status

FIG.4C(ix)

Metabolic profile of GDF-15 shifted with frailty status

**Robust (n=438)**  **Pre-frail (n=290)**  **Frail (n=133)**

Adjusted for age and sex

| group | name | |
|---|---|---|
| Very large HDL ratios | XL-HDL-PL % | 1 |
| | XL-HDL-C % | 2 |
| | XL-HDL-CE % | 3 |
| | XL-HDL-FC % | 4 |
| | XL-HDL-TG % | 5 |
| Large HDL ratios | L-HDL-PL % | 6 |
| | L-HDL-C % | 7 |
| | L-HDL-CE % | 8 |
| | L-HDL-FC % | 9 |
| | L-HDL-TG % | 10 |
| Medium HDL ratios | M-HDL-PL % | 11 |
| | M-HDL-C % | 12 |
| | M-HDL-CE % | 13 |
| | M-HDL-FC % | 14 |
| | M-HDL-TG % | 15 |
| Small HDL ratios | S-HDL-PL % | 16 |
| | S-HDL-C % | 17 |
| | S-HDL-CE % | 18 |
| | S-HDL-FC % | 19 |
| | S-HDL-TG % | 20 |

Beta estimate (95% CI)    Beta estimate (95% CI)    Beta estimate (95% CI)

Non-significant    Significant

FIG.4C(x)

EP 3 961 219 A2

FIG.5A

AUC: 0.766 (0.724–0.807)

FIG.5B

```
                    ┌─────────────────────────────────────┐
                    │  250 metabolites or metabolic features │
                    └─────────────────────────────────────┘
                                      │
                    ┌─────────────────────────────────────┐
                    │     Shapiro-Wilk normality tests     │
                    └─────────────────────────────────────┘


    ┌─────────────────────┐                    ┌──────────────────────────┐
    │ Normal distribution  │                    │  Non-normal distribution  │
    └─────────────────────┘                    └──────────────────────────┘
                                                            │
                                                ┌──────────────────────────┐
                                                │    Log₁₀ transformation   │
                                                └──────────────────────────┘


          ┌───────────────────────────────────────────────────────┐
          │  Scale to their own standard deviation unit separately  │
          └───────────────────────────────────────────────────────┘
                                   │
                    ┌─────────────────────────────┐
                    │     Logistic regression      │
                    └─────────────────────────────┘
                                   │
    ┌───────────────────────────────────────────────────────────────────┐
    │  Benjamini-Hochberg procedure to adjust for multiple testing        │
    └───────────────────────────────────────────────────────────────────┘
```

FIG.6

FIG.7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SOININEN P et al.** *Circ Cardiovasc Genet,* 2015, vol. 8, 192-206